# EUROPEAN PATENT SPECIFICATION

(11) **EP 4 448 787 B1**
(45) Date of publication and mention of the grant of the patent: **20.05.2026**
(21) Application number: 22851139.0
(22) Date of filing: 13.12.2022
(51) Int. Cl.: C12Q 1/6806

(54) **GENOTYPING OF TARGETED LOCI WITH SINGLE-CELL CHROMATIN ACCESSIBILITY**
GENOTYPISIERUNG VON GEZIELTEN LOCI MIT EINZELZELLCHROMATINZUGÄNGLICHKEIT
GÉNOTYPAGE DE LOCI CIBLÉS AVEC ACCESSIBILITÉ À LA CHROMATINE MONO-CELLULE

(30) Priority: 13.12.2021 US 202163288874 P
(43) Date of publication of application: 23.10.2024
(73) Proprietor: Cornell University, Ithaca, NY 14850 (US); New York Genome Center, Inc., New York, NY 10013 (US)
(72) Inventor: LANDAU, Dan-Avi, New York, NY 10021 (US); MYERS, Robert, New York, NY 10021 (US); IZZO, Franco, New York, NY 10021 (US); CHALIGNE, Ronan, New York, NY 10021 (US); SMIBERT, Peter, New York, NY 10021 (US); MIMITOU, Eleni, New York, NY 10021 (US)
(74) Representative: HGF
(86) International application number: PCT/US2022/052701
(87) International publication number: WO 2023/114203

(56) References cited:
- WO-A1-2018/218226
- WO-A1-2019/079640
- WO-A1-2020/072380
- WO-A1-2020/150356
- WO-A1-2021/194699
- WO-A2-2012/083225

## Description

### RELATED APPLICATIONS

This patent application claims the benefit of U.S. Provisional Patent Application No. 63/288,874, filed December 13, 2021,

### BACKGROUND

Pervasive somatic mutations have recently been identified across healthy tissues. Advances in deep nucleotide sequencing and single-cell technologies have revealed that somatic mutations, even those found in cancer-associated genes, are common throughout all human tissues, including blood, skin, lung, and esophagus. Indeed, accumulation of somatic mutations is widely recognized as a critical feature driving the onset of cancer, responsible for aberrant phenotypes leading to malignant clonal expansions. In addition to driving carcinogenesis, somatic mutations have also been established as causative in a wide variety of human diseases, including ulcerative colitis, fatty liver disease, severe adult-onset inflammatory syndrome (VEXAS), and focal cortical dysplasia, amongst many others.

Despite the ubiquitous occurrence of mutations throughout healthy tissues and their wide implications in human disease, the presence of a pathologically associated mutation does not always result in progression to disease. This suggests that additional biological features, such as epigenetic regulation, shape the cellular context within which somatic mutations may drive disease. Moreover, clonal complexity in normal and malignant tissue has largely been studied exclusively at the genetic level, with limited ability to connect genotypes to cellular phenotypes in primary human samples. However, two main challenges arise when studying clonal expansions both in healthy and malignant tissues. Firstly, patient samples with clonal expansions are inherently comprised of an admixture of normal and mutant cells, and mutations often do not result in distinct cell surface markers that would allow for the physical isolation and further study of mutant cells. Secondly, complex tissues can display a high level of cellular heterogeneity across cell types and states, and therefore require high-throughput technologies allowing the capture of thousands of single cells to resolve such complexity. Thus, the ability to interrogate the impact of somatic mutations directly in human samples with high-throughput single-cell technologies is a major goal of the biomedical field.
WO 2012/083225 A2 describes systems and methods for massively parallel analysis of nucleic acids in single cells.
WO 2020/150356 A1 describes polymerase chain reaction normalization through primer titration.
WO 2020/072380 A1 describes determining 5' transcript sequences.
WO 2021/194699 A1 describes single cell genetic analysis.
WO 2019/079640 A1 describes method, systems and apparatus
for high-throughput single-cell DNA sequencing with droplet microfluidics.
WO 2018/218226 A1 describes single cell analysis of transposase accessible chromatin.

### SUMMARY

The invention is defined by claims 1 and 7. Further embodiments of the invention are defined by the dependent claims.

In one aspect the present invention provides a method of genotyping a locus, comprising:generating a plurality of biological particles, each biological particle comprising a polynucleotide;generating a plurality of partitions, each partition comprising one of the plurality of biological particles, a primer pair, and a barcoded bead; and wherein the primer pair comprises:a 5' primer-adapter nucleic acid molecule comprising a nucleotide sequence that is complementary to a sequence located at the 3' end of the locus within the polynucleotide, a 3' primer-adapter nucleic acid molecule comprising a nucleotide sequence that is complementary to a sequence located at the 5' end of the locus,wherein the biological particle comprises genomic DNA and chromatin, and wherein the genomic DNA is fragmented by a transposase to generate the plurality of genomic DNA fragments.

In some embodiments, the polynucleotide is a genomic DNA or fragment thereof. In some embodiments, the polynucleotide is a DNA molecule, RNA molecule, or DNA/RNA hybrid molecule. In some embodiments, the methods further comprises amplifying the locus using the primer pair to generate a locus nucleic acid fragment. In some embodiments, the locus nucleic acid fragment is generated inside the partition. In some embodiments, the 5' end of the 5' primer-adapter nucleic acid molecule further comprises an adapter sequence. In some embodiments, the adapter sequence is Read 1 Nextera sequence (R1N). In some embodiments, the 5' end of the 3' primer-adapter nucleic acid molecule further comprises a second adapter sequence. In some embodiments, the second adapter sequence is a Read 1 Nextera sequence (R1N). In some embodiments, the locus nucleic acid fragment further comprises a sequence complementary to the Read 1 Nextera sequence.

In some embodiments, the barcoded bead is attached to a nucleic acid. In some embodiments, the nucleic acid attached to the barcoded bead comprises a common barcode sequence. In some embodiments, the barcode is 10-20 nucleotides. In some embodiments, the nucleic acid attached to the barcoded bead further comprises a sequence complementary to at least a portion of the adapter sequence. In some embodiments, the adapter sequence is the Read 1 N. In some embodiments, the nucleic acid attached to the barcoded bead further comprises a functional sequence configured to attach to a flow cell of a sequencer. In some embodiments, the functional sequence for attachment to a sequencing flow cell is a P5 sequence or a P7 sequence. In some embodiments, the methods further comprise amplifying the locus nucleic acid fragment, or a derivative thereof, using a second primer pair comprising: a 3' primer-adapter nucleic acid molecule comprising a nucleotide sequence that is complementary to a sequence located at the 5' end of the locus, and a 5' primer-adapter nucleic acid molecule comprising a nucleotide sequence that is complementary to functional sequence, thereby generating a plurality of barcoded locus nucleic acid fragments, each fragment of the plurality of barcoded locus nucleic acid fragments comprising the locus.

In some embodiments, the methods further comprise amplifying the locus nucleic acid fragment, or a derivative thereof, using a third primer pair comprising: a 5' primer-adapter nucleic acid molecule comprising a nucleotide sequence that is complementary to a sequence located at the 3' end of the locus, and a 3' primer-adapter nucleic acid molecule comprising a nucleotide sequence that is complementary to functional sequence, thereby generating a plurality of barcoded locus nucleic acid fragments, each fragment of the plurality of barcoded locus nucleic acid fragments comprising the locus. In some embodiments, the plurality of barcoded locus nucleic acid fragments comprising the locus are generated inside the partition. In some embodiments, each fragment of the plurality of barcoded locus nucleic acid fragments further comprises the Read 1 N sequence. In some embodiments, each fragment of the plurality of barcoded locus nucleic acid fragments further comprises the common barcode sequence and/or the functional sequence configured to attach to a flow cell of a sequencer. In some embodiments, the plurality of barcoded locus nucleic acid fragments are amplified exponentially.

In some embodiments, each biological particle further comprises a plurality of genomic DNA fragments. In some embodiments, the polynucleotide comprises a plurality of genomic DNA fragments. In some embodiments, the biological particle comprises genomic DNA and chromatin. In some embodiments, the genomic DNA is fragmented by a transposase to generate the plurality of genomic DNA fragments. In some embodiments, the transposase is fused to a set of secondary nanobodies (nb). In some embodiments, a primary antibody is bound to a target of interest on the chromatin. In some embodiments, the transposase-nb fusion protein specifically binds to primary antibody. In some embodiments, the transposase is a Tn5 transposase. In some embodiments, the genomic DNA is fragmented by a transposase-nucleic acid complex to generate a plurality of tagged genomic DNA fragments. In some embodiments, the 5' end of each of the tagged genomic DNA fragments comprises an adapter sequence. In some embodiments, the 3' end of each of the tagged genomic DNA fragments comprises a second adapter sequence. In some embodiments, the adapter sequence is a Read 1 sequence (R1N).

In some embodiments, the second adapter sequence is Read 2 sequence (R2N). In some embodiments, the methods further comprise amplifying the plurality of genomic DNA fragments, or derivatives thereof, by nucleic acid amplification generating a plurality of barcoded genomic DNA fragments. In some embodiments, the plurality of barcoded genomic DNA fragments are generated inside the partition. In some embodiments, each fragment of the plurality of barcoded genomic DNA fragments comprises the tagged genomic DNA. In some embodiments, each fragment of the plurality of barcoded genomic DNA fragments further comprises the common barcode sequence and/or the functional sequence configured to attach to a flow cell of a sequencer. In some embodiments, the plurality of barcoded genomic DNA fragments are amplified linearly. In some embodiments, the plurality of tagged genomic DNA, or derivatives thereof are amplified linearly. In some embodiments, the methods further comprise amplifying the barcoded locus nucleic acid fragments, or derivatives thereof, by nucleic acid amplification. In some embodiments, the methods further comprise amplifying the plurality of barcoded genomic DNA fragments, or derivatives thereof, by nucleic acid amplification. In some embodiments, the methods further comprise sequencing said barcoded locus nucleic acid fragments or derivatives thereof. In some embodiments, the methods further comprise sequencing said barcoded genomic DNA fragments or derivatives thereof. In some embodiments, the methods further comprise sequencing said barcoded locus nucleic acid fragments and said barcoded genomic DNA fragments simultaneously.

In some embodiments, the method provides chromatin accessibility and/or genotyping information. In some embodiments, the method provides genotype-phenotype mapping in clonal mosaicism. In some embodiments, said barcoded bead is a gel bead. In some embodiments, said gel bead is a degradable gel bead. In some embodiments, said partition comprises a chemical agent configured to degrade said degradable gel bead. In some embodiments, said nucleic acid barcode molecule is releasably attached to said barcoded bead. In some embodiments, the methods comprise releasing said nucleic acid barcode molecule from said barcoded bead. In some embodiments, said plurality of partitions is a plurality of droplets. In some embodiments, said plurality of partitions is a plurality of wells. In some embodiments, said barcoded nucleic acid fragment, or derivative thereof, is indicative of an accessible region of said chromatin. In some embodiments, said barcoded nucleic acid fragment, or derivative thereof, is indicative of a genotype. In some embodiments, said biological particle is a cell, a cell nucleus, or a cell bead. In some embodiments, the cell is a fixed cell. In some embodiments, said barcoded nucleic acid fragment is generated by a nucleic acid extension reaction. In some embodiments, said barcoded nucleic acid fragment is generated by a ligation reaction. In some embodiments, the method provides single-cell genotyping and chromatin accessibility mapping. In some embodiments, the method provides single-cell genotyping and chromatin accessibility mapping in clonal mosaicism.

In another aspect, disclosed herein is a partition comprising: a biological particle comprising a polynucleotide; a primer pair, and a barcoded bead; and wherein the primer pair comprises: a 5' primer-adapter nucleic acid molecule comprising a nucleotide sequence that is complementary to a sequence located at the 3' end of the locus within the polynucleotide, and a 3' primer-adapter nucleic acid molecule comprising a nucleotide sequence that is complementary to a sequence located at the 5' end of the locus.
**wherein the biological particle comprises genomic DNA and chromatin, and wherein the genomic DNA has been fragmented by a transposase into a plurality of genomic DNA fragments**

In some embodiments, the 5' end of the 5' primer-adapter nucleic acid molecule further comprises an adapter sequence. In some embodiments, the adapter sequence is Read 1 Nextera sequence (R1N). In some embodiments, the 5' end of the 3' primer-adapter nucleic acid molecule further comprises a second adapter sequence. In some embodiments, the second adapter sequence is a Read 1 Nextera sequence (R1N).

In some embodiments, the barcoded bead is attached to a nucleic acid. In some embodiments, the nucleic acid attached to the barcoded bead comprises a common barcode sequence. In some embodiments, the barcode is 10-20 nucleotides. In some embodiments, the nucleic acid attached to the barcoded bead further comprises a sequence complementary to at least a portion of the adapter sequence. In some embodiments, the adapter sequence is the Read 1 N. In some embodiments, the nucleic acid attached to the barcoded bead further comprises a functional sequence configured to attach to a flow cell of a sequencer. In some embodiments, the functional sequence for attachment to a sequencing flow cell is a P5 sequence or a P7 sequence. In some embodiments, each biological particle further comprises a plurality of genomic DNA fragments. In some embodiments, the polynucleotide comprises a plurality of genomic DNA fragments. In some embodiments, the biological particle comprises genomic DNA and chromatin. In some embodiments, the genomic DNA is fragmented by a transposase to generate the plurality of genomic DNA fragments. In some embodiments, the transposase is fused to a set of secondary nanobodies (nb). In some embodiments, a primary antibody is bound to a target of interest on the chromatin. In some embodiments, the transposase-nb fusion protein specifically binds to primary antibody. In some embodiments, the transposase is a Tn5 transposase.

In some embodiments, the genomic DNA is fragmented by a transposase-nucleic acid complex to generate a plurality of tagged genomic DNA fragments. In some embodiments, the 5' end of each of the tagged genomic DNA fragments comprises an adapter sequence. In some embodiments, the 3' end of each of the tagged genomic DNA fragments comprises a second adapter sequence. In some embodiments, the adapter sequence is a Read 1 sequence (R1N). In some embodiments, the second adapter sequence is Read 2 sequence (R2N). In some embodiments, said barcoded bead is a gel bead. In some embodiments, said gel bead is a degradable gel bead. In some embodiments, said partition comprises a chemical agent configured to degrade said degradable gel bead. In some embodiments, said nucleic acid barcode molecule is releasably attached to said barcoded bead. In some embodiments, said plurality of partitions is a plurality of droplets. In some embodiments, said plurality of partitions is a plurality of wells. In some embodiments, said barcoded nucleic acid fragment, or derivative thereof, is indicative of an accessible region of said chromatin. In some embodiments, said barcoded nucleic acid fragment, or derivative thereof, is indicative of a genotype. In some embodiments, said biological particle is a cell, a cell nucleus, or a cell bead. In some embodiments, the cell is a fixed cell.

In some embodiments, said barcoded nucleic acid fragment is generated by a nucleic acid extension reaction. In some embodiments, 1 said barcoded nucleic acid fragment is generated by a ligation reaction.

**The description describes** a kit comprising partitioning fluid, a barcoded bead, and a primer pair comprising: a 5' primer-adapter nucleic acid molecule comprising a nucleotide sequence that is complementary to a sequence located at the 3' end of a specific locus within a polynucleotide, and a 3' primer-adapter nucleic acid molecule comprising a nucleotide sequence that is complementary to a sequence located at the 5' end of the specific locus. **The kit may** further comprises a reagent for disrupting a cell. **The kit may** further comprises a reagent for nucleic acid amplification. **The partitioning fluid may** comprise an aqueous buffer, a non-aqueous partitioning fluid, or oils. **The kit may** further comprises instructions for genotyping a locus or generating a barcoded nucleic acid fragment.

**The barcoded bead may be** attached to a nucleic acid comprising a common barcode sequence. The 5' end of the 5' primer-adapter nucleic acid molecule may further comprises an adapter sequence. **The adapter sequence may be** Read 1 sequence (R1rc). The 5' end of the 3' primer-adapter nucleic acid molecule may further comprises an adapter sequence.

**The adapter sequence may be** Read 1 sequence (R1N). **The nucleic acid may be** attached to the barcoded bead further comprises a sequence complementary to at least a portion of the adapter sequence. **The barcoded bead may be** a gel bead. **The gel bead may be** a degradable gel bead.

### DESCRIPTION OF THE FIGURES

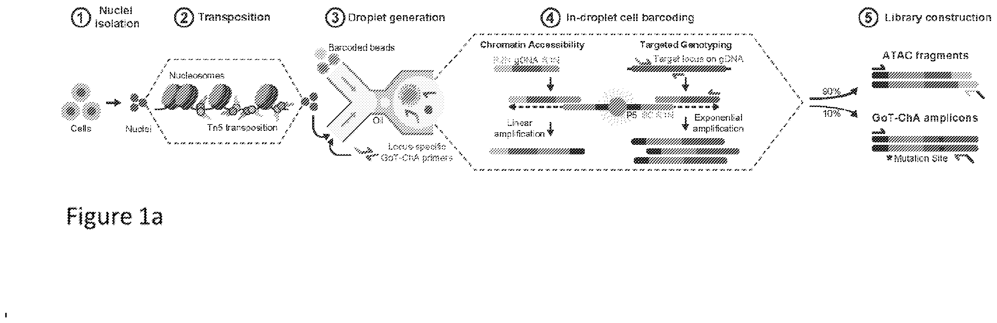

**Figures 1a-1n** show that GoT-ChA allows for accurate single-cell genotyping of somatic mutations together with chromatin accessibility information. a, Schematic representation of GoT-ChA workflow. P5, Illumina sequencing handle; BC, unique cell barcode; R1N, Read 1 Nextera adapter; gDNA, genomic DNA; R2N, Read 2 Nextera adapter. b, Schematic representation of the TP53R248 mixing study (upper panel) and accessibility-based uniform manifold approximation and projection (UMAP) for HEL (n = 2,540 cells; TP53WT/WT; purple) and CA46 (n = 2,117 cells; TP53MUT/MUT; green; bottom panel). c, Chromatin accessibility coverage of marker genes (FDR < 0.05 and log2FC > 1.25; Wilcoxon rank sum test followed by Benjamini-Hochberg correction), agnostic to genotyping information, used for cell line identity assignments. ATAC signal was normalized by the number of reads covering transcriptional start sites (TSS). d, Heatmap showing heteroplasmy for mutually exclusive mitochondrial variants detected in the scATAC-seq data for either HEL or CA46 cell lines. Minimum coverage of 10 reads mapping to the variant site was required. e, Detected coverage for the TP53R248 locus of interest for either HEL or CA46 cell lines illustrating improved genotyping capture with GoT-ChA versus standard 10x scATAC. Percent of cells reported for the 10x scATAC libraries was defined as the percent of cells with at least one read mapping to the assayed locus. Percent of cells for the GoT-ChA libraries was defined as successful genotyping post processing (see materials and methods). f, UMAP colored by GoT-ChA genotype classifications of CA46 (n = 2,117 cells) and HEL (n = 2,540 cells) assigned as wildtype (WT, blue), mutant (MUT, red), or not assignable (NA, grey) cells. The percentage of cells assigned to each genotype per cell line, as well as accuracy and overall percent of cells genotyped are shown. g, Inferred copy number variation (CNV) scores from the scATAC-seq data (see materials and methods) in either TP53R248 wildtype or mutant cells as defined by GoT-ChA mixing study relative to a healthy control (see materials and methods), verifying the concordance between known amplification of the region including the JAK2 gene on chromosome 9 in HEL cells, which are wildtype for TP53R248. h, Schematic representation of the JAK2^{V617} mixing study (top panel) and chromatin accessibility-based UMAP for HEL (n = 1,334 cells; JAK2MUT/MUT; purple), CCRF-CEM (n = 638 cells; JAK2WT/WT; light blue) and SET-2 (n = 1,268 cells; JAK2WT/MUT; pink) cell lines (bottom panel). i, Chromatin accessibility coverage of marker genes (FDR < 0.05, log2FC > 1.25), agnostic to genotyping information used for cell line identity assignments. Wilcoxon rank sum test followed by Benjamini-Hochberg correction. j, Heatmap showing heteroplasmy of mutually exclusive mitochondrial variants detected in the scATACseq data for HEL, CCRF-CEM and SET-2 cell lines. Minimum coverage of 10 reads mapping to the variant site was required. k, Detected coverage for the locus of interest per cell line illustrating improved genotyping capture with GoT-ChA versus standard 10x scATAC. Percent of cells reported for 10x scATAC-seq and GoT-ChA are as defined in panel e. l, Chromatin accessibility-based UMAP for HEL (n = 1,334 cells), SET-2 (n = 1,268 cells) and CCRF-CEM (n = 638 cells) colored by GoT-ChA genotype classifications of homozygous wildtype (WT, blue), homozygous mutant (MUT, red), heterozygous (HET, yellow), and not assignable (NA, grey) cells (left panel). Percentage of cells assigned to each genotype and overall accuracy and percent of genotyped cells is reported for the HEL and CCRF-CEM homozygous cell lines. Genotyping results for the SET-2 cell line indicating multi-allelic capture by GoT-ChA genotyping in a subset of cells, allowing for accurate heterozygous classification in 34.3% of genotyped cells (right panel). We note that mutated and wildtype annotation likely represents incomplete capture of all alleles, and the observed percentages correspond to the 3:1 allelic ratio of mutated:wildtype alleles present in SET-2 cells, as verified by Sanger sequencing. m, CNV scores inferred from the scATAC-seq data (see materials and methods) comparing homozygous wildtype and mutant cells in the JAK2^{V617} GoT-ChA mixing study relative to a healthy control (see materials and methods), illustrating concordance between known amplification of the region including the JAK2 gene on chromosome 9 in HEL cells, which are mutant for JAK2^{V617}. n, GoT-ChA quality metrics in relation to target locus copy number: fraction of cells genotyped (left panel) positively correlates with locus copy number, while genotyping accuracy (right panel) remains above 95% irrespective of locus copy number.

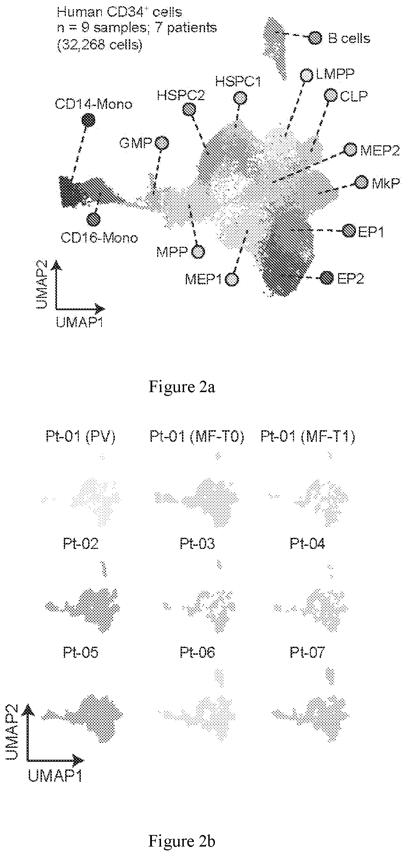

**Figures 2a-2i** show GoT-ChA on human JAK2^{V617F}-mutated myelofibrosis samples reveals myeloid differentiation bias that is abrogated with ruxolitinib treatment. a, Integrated chromatin accessibility uniform manifold approximation and projection (UMAP) after reciprocal latent semantic indexing (LSI) integration (see materials and methods) from CD34+ sorted patient samples (n = 9 samples, 7 patients, 32,268 cells) illustrating the expected progenitor populations in early hematopoiesis. HSPC = hematopoietic stem and progenitor cells; MPP = multi-potent progenitors; GMP = granulocyte monocyte progenitors; MEP = megakaryocyte-erythrocyte progenitors; EP = erythroid progenitors; MkP = megakaryocytic progenitors; LMPP = lymphoidmyeloid pluripotent progenitors; CLP = common lymphoid progenitors; CD16-Mono = CD16+ monocyte progenitors; CD14-Mono = CD14+ monocytes. b, Integrated UMAP plotted for each patient sample, illustrating consistent distribution of cells after reciprocal LSI integration (see materials and methods). c, UMAPs showing the gene accessibility scores of canonical marker genes (FDR < 0.05 and log2FC > 1; Wilcoxon rank sum test followed by Benjamini-Hochberg correction) used to identify the individual progenitor clusters within the integrated UMAP. d, Transcription factor footprinting of canonical transcription factors for progenitor clusters, further confirming cluster identity assignments. e, Comparison of JAK2^{V617} genotyping efficiency across recent publications applying single-cell droplet-based technologies for genotyping. Bars represent mean values, each point represents an individual patient sample, and error bars indicate standard deviation. f, Integrated UMAP colored by GoT-ChA assigned JAK2^{V617} genotypes as homozygous wildtype (WT; n = 4,195 cells; blue), homozygous mutant (MUT; n = 9,202 cells; red) or heterozygous (HET; n = 2,340 cells; yellow) and not assignable (NA; n = 16,531 cells; grey). g, Radar plot showing the mutant fraction for each cluster, relative to the fraction of mutant cells observed for the entire sample, of either untreated (green) or ruxolitinib-treated (yellow) patient samples across myeloid and HSPC/MPP clusters. h, UMAP depicting semisupervised pseudotime estimation for the erythroid differentiation trajectory, with the HSPC1 cluster as initial state and the EP2 cluster as final state. i, Fraction of mutant cells along erythroid pseudotime for untreated (n = 3,894 cells) or ruxolitinibtreated (n = 1,765 cells) samples. Erythroid pseudotime was divided in 10 quantiles; each point represents the mean fraction of mutant cells, error bars indicate standard error, lines indicate the fit and shadowed areas represent the 95% confidence interval of the generalized additive model (upper panel). The fraction of cells belonging to the cluster specified by color (HSPC = hematopoietic stem and progenitor cell; MPP = multipotent progenitor; MEP = megakaryocyte erythrocyte progenitor; EP = erythroid progenitor) within the indicated pseudotime quantile is shown (bottom panel).

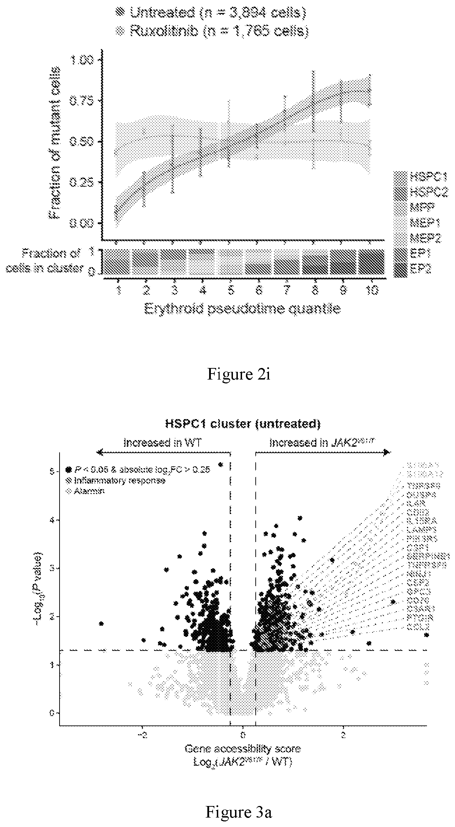

**Figures 3a-3k** show JAK2^{V617F} mutant HSPCs exhibit intrinsic pro-inflammatory and myeloid-biased epigenetic priming that is reduced with JAK inhibition. a, Volcano plot illustrating differential gene accessibility scores between wildtype (n = 128 cells) and mutant (n = 93 cells) cells within the HSPC1 cluster of untreated MF patients (n = 4; excluding PV sample). Horizontal dotted line represents P = 0.05; vertical dotted lines represent absolute log2FC > 0.25. Genes involved in the inflammatory response pathway (Hallmark M5932) are highlighted in green. Alarmin genes are highlighted in yellow. Linear mixture model (LMM) modeling patient sample as a random effect for accounting for inter-patient variability followed by likelihood ratio test (see materials and methods). b, Pre-ranked gene set enrichment of genes within the Inflammatory response pathway for wildtype vs JAK2^{V617F} HSPC1 gene accessibility scores (Bonferroni correction; FWER = family wise error rate; NES = normalized enrichment score; Hallmark pathway M5932). c, Chromatin accessibility coverage tracks of two differentially accessible (P <0.05 and absolute log2FC > 0.25) genes between wildtype (blue) and mutant cells (red). d, Volcano plot of the differentially accessible transcription factor motifs (FDR < 0.05 and absolute Dz-score > 0.25; LMM followed by likelihood ratio test and Benjamini-Hochberg correction) between wildtype (n = 128 cells) and JAK2^{V617F} mutant (n = 93 cells) HSPC1 cells from untreated MF patients (n = 4; excluding PV sample). Only motif accessibility of transcription factors expressed in scRNA-seq data from MPN patients29 were considered. Transcription factors involved in biological pathways such as myeloid/erythroid differentiation (pink), NF-kB signaling (teal) and AP-1 complex (JUN/FOS; blue) are highlighted. The horizontal dotted line represents FDR = 0.05, the vertical dotted lines represent absolute Dz-score > 0.25. e, Transcription factor footprinting for JUN or NFKB 1 motif sites in either wildtype or JAK2^{V617F} mutant cells within the HSPC1 cluster for untreated or ruxolitinib-treated patients. f, Motif accessibility for either JUN or NFKB 1 transcription factors for a longitudinal sample (Pt-01) from a patient that progressed from polycythemia vera (PV) to myelofibrosis (MF). Wilcoxon rank sum test. g, Volcano plot of the differentially accessible transcription factor motifs (FDR < 0.05 and absolute Dz-score > 0.25; LMM followed by likelihood ratio test and Benjamini-Hochberg correction) between wildtype (n = 312 cells) and mutant (n = 517 cells) HSPC2 cells from untreated MF patients (n = 4; excluding PV sample). Transcription factors involved in biological pathways such as myeloid/erythroid differentiation (pink) and TGF-b signaling (purple) are highlighted. The horizontal dotted line represents FDR = 0.05, the vertical dotted lines represent absolute Dz-score > 0.25. h, Correlation between motif accessibility of transcription factors belonging to the STAT family and transcription factors involved in NF-kB signaling within HSPCs (HSPC1 and HSPC2 clusters combined) in untreated MF patients (n = 4; excluding PV sample). Spearman correlation. i, Scatter plots illustrating transcription factor motif accessibility correlation in untreated MF HSPCs (HSPC1 and HSPC2) between NFKB1 and STAT1 (left panel) and STAT4 (right panel). Each point represents a single cell, and the lines represent the linear fit for either wildtype (blue) or JAK2^{V617F} mutant (red). Shadowed areas represent 95% confidence intervals. Linear modeling followed by ANOVA test for comparison between wildtype and mutant slopes, as well as the Rho values representing the goodness of fit for each genotype are shown. j, Schematic representation of a Jak2RL mouse experiment110 in which bulk RNA-seq was performed on sorted LSK cells from Jak2^{V617F} and Jak2^{V617F}-deleted mice. k, Pre-ranked gene set enrichment of differentially expressed genes within the erythroid (FDR = 2.5 x 10⁻⁴; NES = -1.87; heme metabolism Hallmark gene set) and TNF via NF-kB (FDR = 4.1 x 10⁻⁴; NES = -1.59) gene sets in Jak2^{V617F} compared to Jak2^{V617F}-deleted mouse LSK cells.

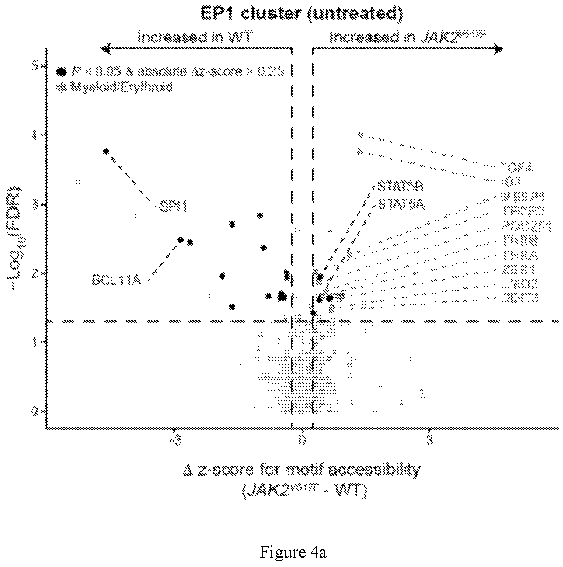

**Figures 4a-4i** show GoT-ChA reveals JAK2^{V617F}-driven epigenetic dysregulation of hemoglobin locus in EPs and increased JUN/FOS signaling in MkPs. a, Differential transcription factor motif accessibility between wildtype (n = 121) and mutant (n = 703) cells within the EP1 cluster of untreated MF patient samples (n = 4). Horizontal dotted line represents FDR = 0.05; vertical dotted lines represent absolute Dz-score > 0.25; transcription factors involved in erythroid differentiation are highlighted in pink. Linear mixture model (LMM) followed by likelihood ratio test and Benjamini-Hochberg correction. b, Examples of co-accessibility measurements (correlation > 0.1 and FDR < 0.05; Wilcoxon rank sum test followed by Benjamini-Hochberg correction) of regulatory elements surrounding the HBG1 locus for wildtype (n = 121 cells; blue) and mutant (n = 703 cells; red) cells in the EP1 cluster. Peaks (light blue), the hemoglobin locus control region (LCR, purple), and transcription factor motifs for BCL11A (red) and LYL1 (yellow) are indicated. Co-accessibility measurements centered in two peaks of interest are indicated via shaded dotted lines for an enhancer controlling HBG1 expression (shaded in red) on top, and a comparison peak within the LCR (shaded in blue). Gene location and shaded regions indicating location of highlighted peaks are shown at bottom. c, Chromatin accessibility coverage tracks comparing accessibility between homozygous wildtype (blue), heterozygous (yellow), and homozygous mutant (red) cells within the EP1 cluster for the HBG1 and HBB genes. d, Quantification of the proportion of cells showing accessibility of the HBG1 gene (upper panel; Fisher exact test) or the imputed gene accessibility value for either homozygous wildtype, heterozygous or homozygous mutant cells (bottom panel; LMM followed by likelihood ratio test). e, BCL11A motif accessibility and HBG1 gene accessibility scores for longitudinal sample (Pt-01) from a patient progressing from polycythemia vera (PV) to myelofibrosis (MF). Wilcoxon rank sum test. f, Differential transcription factor motif accessibility between wildtype (n = 95 cells) and mutant (n = 542 cells) within the MkP cluster of untreated MF patients (n = 4). A subset of TFs that exhibited enrichment in mutant cells are highlighted by pathway involvement: JUN/FOS signaling in blue, TGF-b in purple. Horizontal dotted line represents FDR = 0.05; vertical dotted lines represent absolute Dz-score > 0.25. LMM followed by likelihood ratio test and Benjamini-Hochberg correction. g, Transcription factor footprinting for JUN and FOS comparing wildtype (blue) and mutant (red) in untreated (n = 4; top) and treated (n = 3; bottom) MF patient samples. h, Schematic representation of a Jak2RL mouse experiment110 in which bulk RNA-seq was performed on sorted MEP cells from Jak2^{V617F} and Jak2^{V617F}-deleted mice. i, Gene set enrichment analysis illustrating a depletion of JUN targets and heme metabolism pathway (Hallmark M5945) in Jak2^{V617F}-deleted compared to JAK2^{V617F} mouse MEPs (NES = normalized enrichment score).

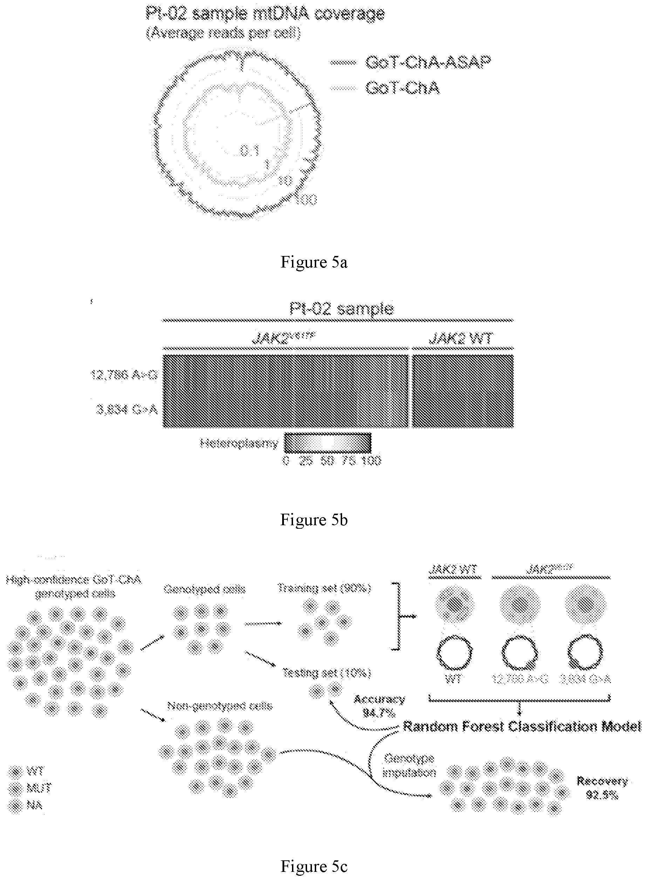

**Figures 5a-5k** show GoT-ChA integration with ASAP-seq enables mitochondrially informed genotype imputation and surface protein measurements in primary human samples. a, Depiction of the overall mitochondrial genome coverage from sample Pt-02 processed with GoT-ChA-ASAP compared to the same sample with the standard GoT-ChA protocol. The average number of reads per cell covering each region is shown. b, Heatmap of heteroplasmy of mitochondrial variants per cell for patient sample Pt-02, illustrating the presence of two variants (12,786 A>G and 3,834 G>A) that segregate in phase with GoT-ChA genotyping. c, Schematic representation detailing the process of genotype imputation using a random forest classifier. GoT-ChA genotyped cells were separated into a training set (90% of cells, downsampled for even numbers of mutant and wildtype cells) and a test set (10% of cells). The classifier resulted in an imputed genotyping accuracy of 94.7% on the test set and, when applied to nongenotyped cells in the same sample, increased the effective rate of genotyping in the sample from 23.2% to 92.5%. d, Percent of JAK2^{V617} genotyped cells across multiple studies and protocols utilizing high-throughput droplet-based technologies. Bars represent the mean; each dot represents a sample and error bars represent the standard deviation. e, Uniform manifold approximation and projection (UMAP) of cells from sample Pt-02 processed with GoT-ChA-ASAP, illustrating the expected stem cell and committed progenitor populations in early hematopoiesis (n = 11,457 cells). HSPC = hematopoietic stem and progenitor cells; MPP = multi-potent progenitors; GMP = granulocyte monocyte progenitors; MEP = megakaryocyte-erythrocyte progenitors; EP = erythroid progenitors; MkP = megakaryocytic progenitors; LMPP = lymphoid-myeloid pluripotent progenitors; Pre-B = precursor B cells; NK = natural killer cells; BP = basophil progenitors. f, UMAP as seen in e, colored by GoTChA genotyping of the JAK2^{V617} locus after mitochondrial-based imputation: wildtype (WT; n = 3,482 cells; blue), mutant (MUT; n = 6,697 cells; red), and not assignable (NA; n = 872 cells; grey). g, UMAP as seen in e, colored by protein expression of cell surface markers, illustrating successful measurement of protein expression with GoT-ChA-ASAP. h, Differential protein expression between wildtype and mutant cells within HSPC1 and HSPC2 clusters. Wilcoxon rank sum test followed by Bonferroni correction. Black dots represent differentially expressed (family wise error rate [FWER] < 0.05 and absolute Dzscore > 1) cell surface proteins. i, Scatter plot comparing protein expression of CD34 and CD90. Inset pie charts show the enrichment of JAK2^{V617F} mutant cells in each CD34+ quadrant. j, Chromatin accessibility coverage track of the CD90 gene (THY1) and a proximal upstream regulatory element is shown for either wildtype (blue) and mutant cells (red) from the HSPC1 and HSPC2 clusters. Wilcoxon rank sum test followed by Benjamini-Hochberg correction. k, CD90 (THY1 gene) chromatin accessibility scores of HSPCs (HSPC1 and HSPC2 clusters) for untreated MF samples (upper panel; P = 5.3 x 10⁻⁵; linear mixture model (LMM) modeling patient identity as random effects, followed by likelihood ratio test) excluding Pt-01 (PV) and sample Pt-02, or ruxolitinib-treated samples (P = 0.98; LMM modeling patient identity as random effects, followed by likelihood ratio test).

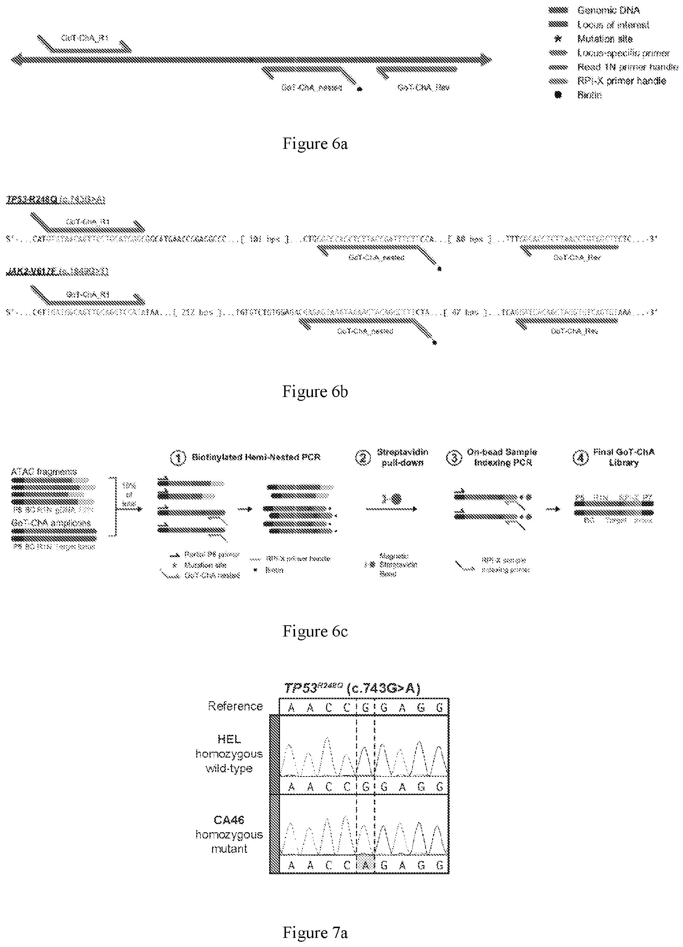

**Figures 6a-6c** show primer sequences, relative positions, and library construction schematic for GoT-ChA. a, Primer design schematic for GoT-ChA, indicating the relative position of GoT-ChA primers to one another as well as the targeted locus of interest. b, Primer binding sites for TP53R248 and JAK2V617 genotyping. Binding sites are highlighted in blue, with custom primer handles shown as indicated in panel a. c, Schematic representation detailing GoT-ChA library construction, comprised of a biotinylated hemi-nested PCR, a streptavidin-biotin pull-down, and an on-bead sample indexing PCR, ultimately resulting in the generation of a genotyping library compatible with Illumina sequencing. P5, Illumina sequencing handle; BC, unique cell barcode; R1N, Read 1 Nextera adapter; gDNA, genomic DNA; R2N, Read 2 Nextera adapter.

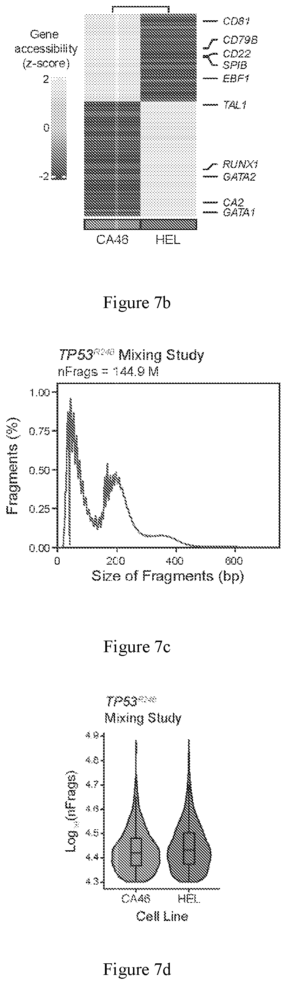

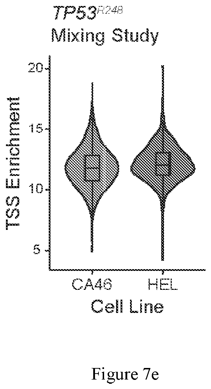

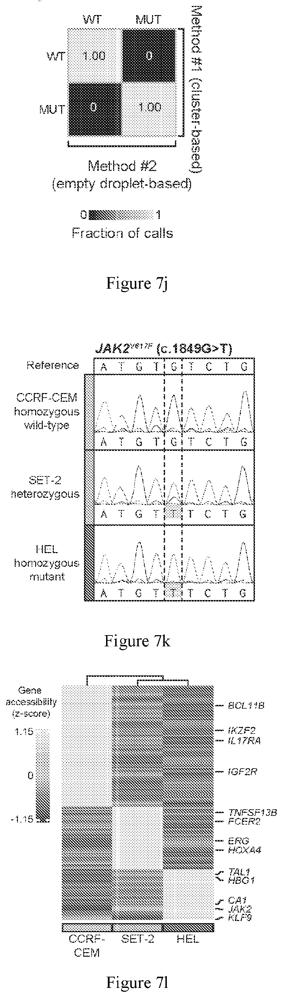

**Figures 7a-7p** show genotyping accuracy, quality control metrics, and GoT-ChA data processing of proof-of-principle mixing studies. a, Sanger sequencing confirming known homozygosity of TP53R248 wildtype HEL cells and TP53R248Q mutant CA46 cells. b, Differential gene accessibility score heat map illustrating the identification of the two human cell lines HEL and CA46 in the TP53R248 mixing study (FDR < 0.05 and log2FC > 1.25; Wilcoxon rank sum test followed by Benjamini-Hochberg correction). c, scATAC-seq library fragment size distribution for the TP53R248 mixing study, illustrating characteristic periodicity due to nucleosome positioning. d, Number of unique nuclear fragments per cell for each cell line in the TP53R248 mixing study, indicating adequate complexity of the scATAC-seq libraries. e, TSS enrichment scores per cell for each cell line in the TP53R248 mixing study, indicating a high signal-to-background ratio in the scATAC-seq data. f, Schematic representation illustrating the workflow of the analytical pipeline used for noise correction of the GoT-ChA data and for defining single cell genotype assignments. g, Histograms of WT (left panel) and MUT (right panel) number of reads per cell from the TP53R248 mixing study. Kernel density estimation (KDE) lines for overall data (red), background (yellow), and signal (pink) are shown for each genotype. h, Scatter plots comparing assigned genotypes from GoT-ChA processing (left panel) as compared to the true genotypes as determined by cell line identity (right panel). Dotted lines indicate the detected threshold for the distinction between background and signal before updated cluster assignments for both WT and MUT data. i, Comparison of the percentage of cells genotyped in alternative empty droplet-based noise correction methods (see materials and methods). j, Confusion matrix for genotype assignments for those cells with available genotyping in both alternative noise correction methods (see materials and methods). k, Sanger sequencing confirmation of known genotypes for the JAK2^{V617} mixing study: CCRF-CEM wildtype cells, SET-2 heterozygous cells, and HEL homozygous mutant cells. Note that SET-2 data confirm the known allelic ratio of 3:1 for mutated:wildtype alleles in this cell line. l, Differential gene accessibility score (FDR < 0.05 and log2FC > 1.25; Wilcoxon rank sum test followed by Benjamini-Hochberg correction) heat map indicating identification of the three human cell lines CCRFCEM, SET-2, and HEL used in the JAK2^{V617} mixing study. m, Fragment size distribution for the JAK2^{V617} mixing study scATAC-seq library, showing expected nucleosomal periodicity. n, Scatter plots showing the number of unique nuclear fragments per cell vs. the transcriptional start site (TSS) enrichment. Dotted lines indicate the selected thresholds based on the distribution. o, Histograms of WT (left) and MUT (right) read distributions from the JAK2^{V617} mixing study. KDE lines for overall data (red), background (yellow), and signal (pink) are shown for each genotype. p, Scatter plots comparing assigned genotypes from GoTChA processing (left panel) compared to the true genotypes (right panel) as determined by cell line identity. Dotted lines indicate the initial thresholds identified between background noise and signal for either WT (vertical line) or MUT (horizontal line) data before final genotype assignment after clustering (see materials and methods).

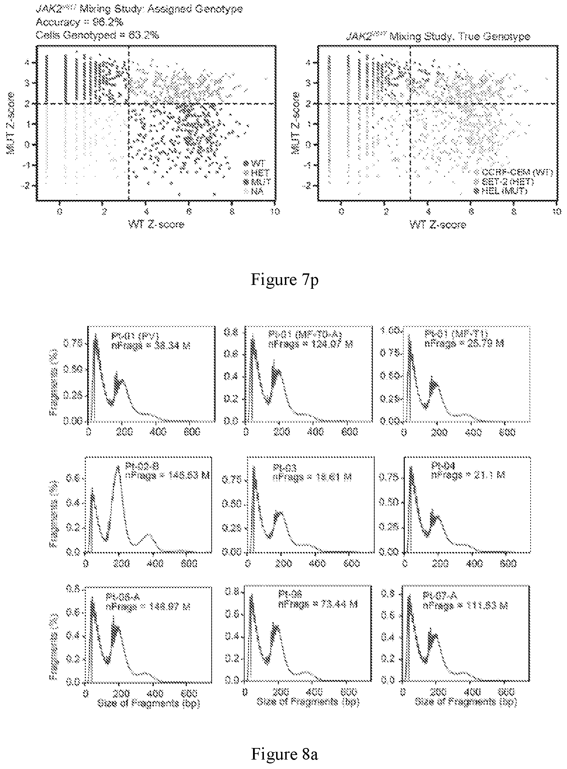

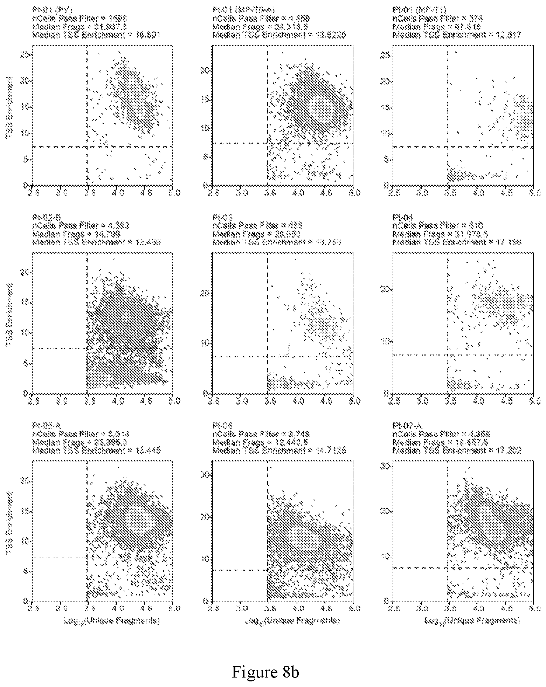

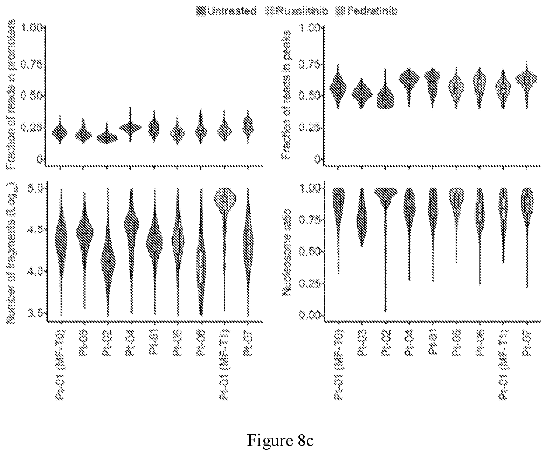

**Figures 8a-8i** show GoT-ChA results in high quality chromatin accessibility data in human patient samples, allowing progenitor subset annotation. a, Fragment size distribution plots per sample, showing characteristic periodicity due to nucleosome positioning across all samples. In samples for which multiple 10x lanes were run, a representative example is shown. b, Scatter plots showing the number of unique nuclear fragments versus TSS enrichment for each patient sample, with dotted lines indicating the thresholds used for identification of high-quality cells (TSS enrichment score > 8 and log10(unique fragments) > 3.5). The median values for unique nuclear fragments, median TSS enrichment score, and total number of cells that pass filters per patient sample are shown. c, Violin plots detailing quality metrics for scATAC-seq data, plotted per patient, and colored by JAK inhibitor therapy: fraction of reads in promoters (top left), fraction of reads in peaks (top right), number of unique fragments (bottom left), and nucleosome ratio (bottom right). Untreated samples are colored green, ruxolitinib-treated in yellow, and fedratinib-treated in purple. d, Integrated uniform manifold approximation and projection (UMAP) colored by patient sample, illustrating consistent distribution of cells after integration through reciprocal latent semantic indexing (see materials and methods). e, Heat map of gene accessibility scores across all progenitor subclusters, showing genes with differential accessibility (FDR < 0.05 and log2FC > 1; Wilcoxon rank sum test followed by Benjamini-Hochberg correction), and highlighting informative marker genes. f, Genomic distribution of peaks in distal regions (green), exonic regions (blue), intronic regions (purple), and promoters (yellow) for the union of peaks (n = 321,995 peaks). g, Differentially accessible peaks (FDR < 0.05 and log2FC > 1; Wilcoxon rank sum test followed by Benjamini-Hochberg correction) across progenitor clusters. h, Gene accessibility scores for key stem cell (HLF, AVP, CD34, CD38) marker genes across clusters HSPC1 and HSPC2 (Wilcoxon rank sum test), consistent with HSPC1 representing earlier HSPCs (higher HLF, AVP and CD34) and HSPC2 representing later HSPCs (higher CD38 and lower HLF, AVP and CD34). i, Confusion matrix between manually annotated cluster labels and predicted labels based on scRNA-seq reference via bridge integration mapping54 (see materials and methods).

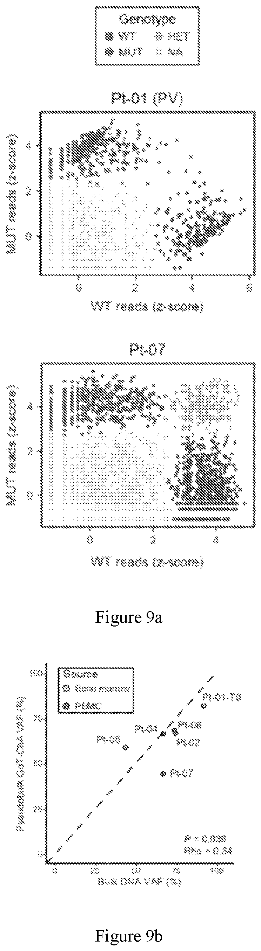

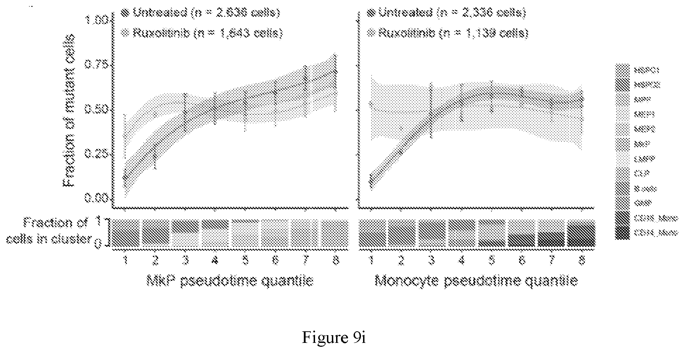

**Figures 9a-9i** show genotyping and distribution of mutant cells in patient samples. a, Scatter plots illustrating two representative examples of assigned genotypes from GoT-ChA processing. Final genotype assignment after clustering shown in dot color. Homozygous wildtype (WT; blue), homozygous mutant (MUT; red), heterozygous (HET; yellow) or not assignable (NA; grey). b, Scatter plot of pseudobulk GoT-ChA variant allele fraction (VAF) versus bulk DNA sequencing VAF reported clinically for those patients for which was available (n = 6 patients; no clinically reported VAF available for samples Pt-01 (PV), Pt-03, and Pt-01 (MF-T1)). Source of biological material for each patient sample used for GoT-ChA (bone marrow in yellow and peripheral blood in red) is indicated. Dotted line represents a slope of 1 with intersection 0 as reference. Correlation values are shown (Spearman correlation test). c, Fraction of genotyped cells and average JAK2 gene accessibility per cluster. Bars represent the mean; each point represents a patient sample and error bars represent the standard deviation (upper panel). JAK2 mean gene accessibility z-scores across cell clusters is shown (bottom panel) d, Chromatin accessibility coverage of the JAK2 gene across progenitor clusters. The ^{V617} hotspot is indicated by the region shaded red. e, Genotype calls projected onto the integrated uniform manifold approximation and projection (UMAP) per sample. WT = wildtype cells (blue); HET = heterozygous cells (yellow); MUT = JAK2^{V617F} mutant cells (red); NA = not assigned (grey). f, Relative fold change of mutant fraction in MkP and EP clusters compared to HSPCs across all patient samples. Samples for which more than one technical replicate was performed are shown separately. Statistical significance was determined by Fisher test followed by Bonferroni multiple hypothesis correction. Family-wise error rate (FWER) indicated as Padj; n.s. = not significant (Padj > 0.05). g, Relative fold change of mutant fraction in MkP and EP clusters compared to HSPC cluster for the fedratinib treated sample Pt-07. Statistical significance was determined by Fisher test followed by Bonferroni multiple hypothesis correction. Family-wise error rate (FWER) indicated as Padj; n.s. = not significant (Padj > 0.05). h, UMAP illustrating semi-supervised pseudotime estimation for hematopoietic lineages: megakaryocytic (left panel), and monocyte (right panel). i, Fraction of mutant cells along megakaryocyte or monocyte pseudotime for untreated or ruxolitinib-treated samples. Pseudotime was divided in 8 quantiles; each point represents the mean fraction of mutant cells, error bars indicate standard error, lines indicate the fit and shadowed areas represent the 95% confidence interval of the generalized additive model. The fraction of cells belonging to the cluster specified by color (HSPC = hematopoietic stem and progenitor cell; MPP = multipotent progenitor; MEP = megakaryocyte erythrocyte progenitor; MkP = megakaryocyte progenitor; LMPP = lymphoid-myeloid pluripotent progenitor; GMP = granulocyte monocyte progenitor; CD14 Mono = CD14+ monocyte progenitors; CD16 Mono = CD16+ monocytes) within the indicated pseudotime quantile is shown (bottom panel).

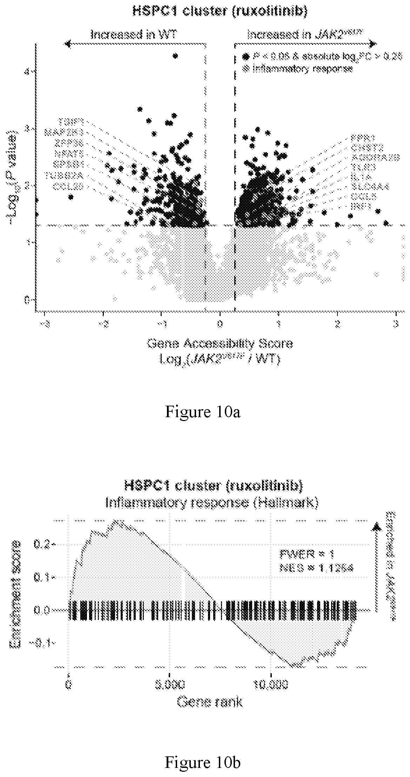

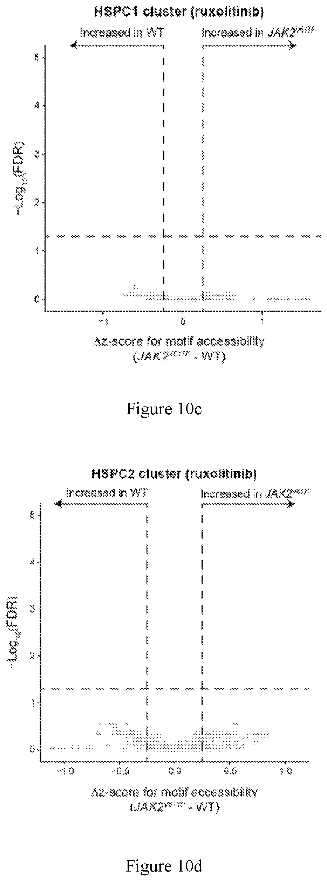

**Figures 10a-10e** show differential gene and transcription factor motif accessibility in ruxolitinib-treated HSPCs. a, Volcano plot illustrating differential gene accessibility scores between wildtype (n = 55 cells) and mutant (n = 87 cells) cells within the HSPC1 cluster of ruxolitinib-treated patients (n = 3). Horizontal dotted line represents P = 0.05; vertical dotted lines represent absolute log2FC > 0.25. Genes involved in the inflammatory response pathway (Hallmark M5932) are highlighted in green. Linear mixture model (LMM) followed by likelihood ratio test. b, Pre-ranked gene set enrichment of genes within the Inflammatory response pathway for wildtype vs JAK2^{V617F} HSPC1 gene accessibility scores in ruxolitinib-treated samples (Bonferroni multiple hypothesis correction; FWER = family wise error rate; NES = normalized enrichment score; Hallmark pathway M5932). c, Volcano plot of differential motif accessibility scores between wildtype and mutant cells within the HSPC1 cluster from patients treated with ruxolitinib JAK inhibitor therapy. Horizontal dotted lines indicate FDR = 0.25, vertical dotted lines indicate absolute Dz-score > 0.25. Linear mixture model followed by likelihood ratio test and Benjamini-Hochberg correction. d, Volcano plot of differential motif accessibility scores between wildtype and mutant cells within the HSPC2 cluster from patients treated with ruxolitinib JAK inhibitor therapy. Horizontal dotted lines indicate FDR = 0.25, vertical dotted lines indicate absolute Dz-score > 0.25. Linear mixture model followed by likelihood ratio test and Benjamini-Hochberg correction. e, Table showing the correlation values and Rho goodness of fit values for HSPCs (HSPC1 and HSPC2 clusters combined) in untreated MF samples; Spearman correlation test.

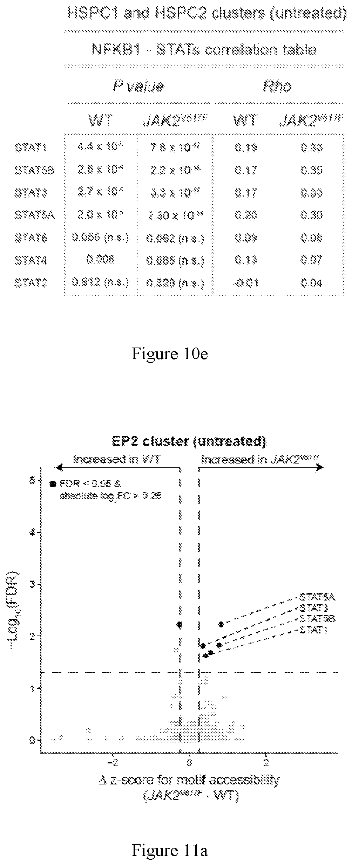

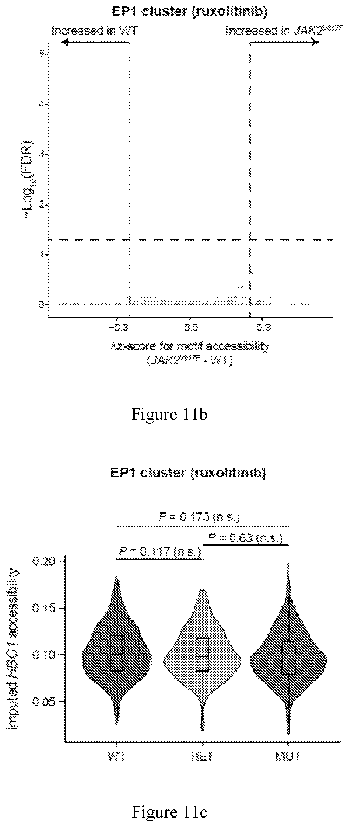

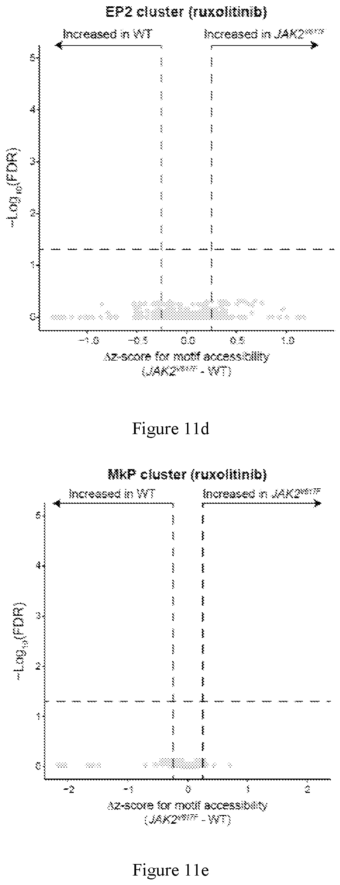

**Figures 11a-11e** show Differential gene and transcription factor motif accessibility in EPs and MkPs with and without ruxolitinib treatment. a, Differentially accessible transcription factor motifs between wildtype and mutant cells within the EP2 cluster of untreated MF patients (n = 4). For all panels unless stated otherwise, horizontal dotted line represents FDR = 0.05; vertical dotted lines represent absolute Dz-score> 0.25. Linear mixture model (LMM) followed by Benjamini-Hochberg correction. b, Differentially accessible transcription factor motifs between wildtype and mutant cells within the EP1 cluster of ruxolitinib-treated MF patients (n = 3). c, Quantification of the imputed gene accessibility value for either homozygous wildtype, heterozygous, or homozygous mutant cells in ruxolitinib-treated MF patients (n = 3) from the EP1 (n = 2,065 cells) cluster (LMM followed by likelihood ratio test). d, Differentially accessible transcription factor motifs between wildtype and mutant cells within the EP2 cluster of ruxolitinib-treated MF patients (n = 3). e, Differentially accessible transcription factor motifs between wildtype and mutant cells within the MkP cluster (n = 1,437 cells) of ruxolitinib-treated MF patients (n = 3).

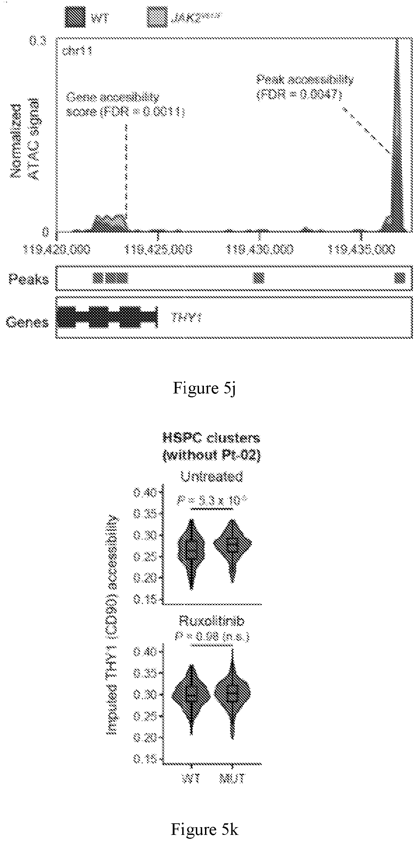

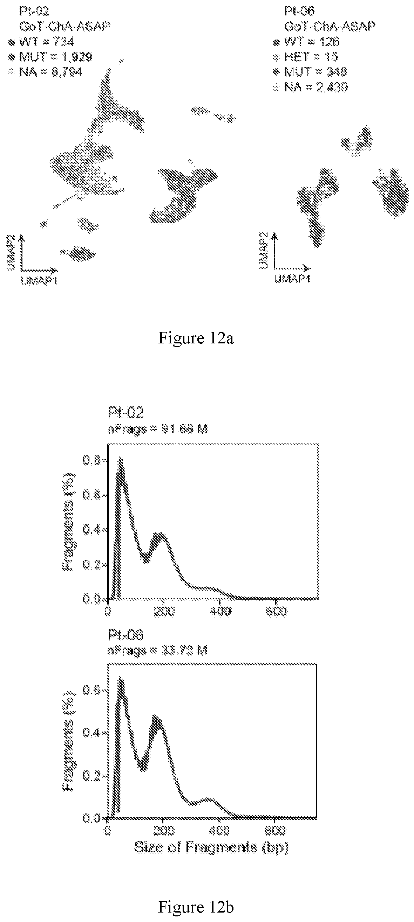

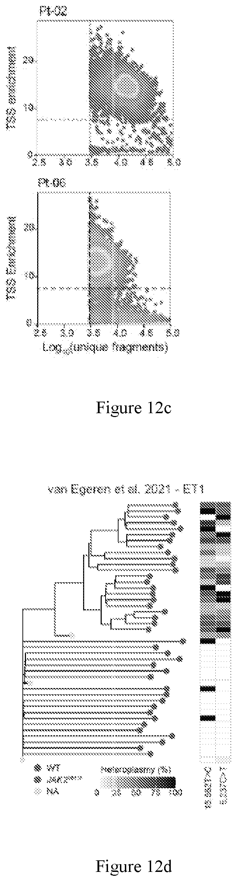

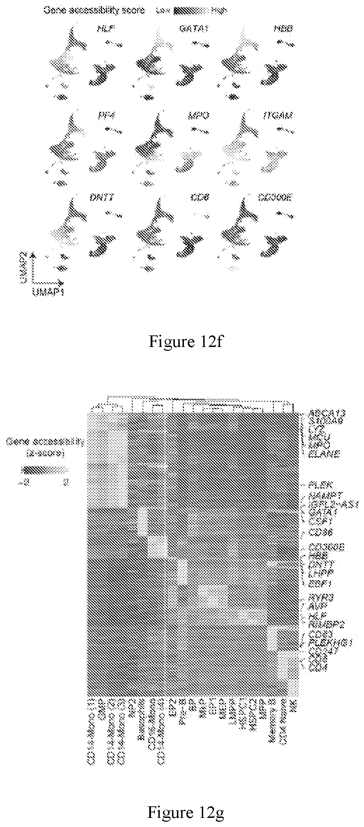

**Figures 12a-12i** show quality control and clustering of GoT-ChA-ASAP samples. a, Chromatin-based uniform manifold approximation and projection (UMAPs) colored by GoT-ChA-assigned JAK2^{V617} genotypes for Pt-02 and Pt-06 as homozygous wildtype (WT; blue), homozygous mutant (MUT; red), heterozygous (HET; yellow), and not assignable (NA; grey) prior to mitochondrial imputation. Cell numbers for each genotype are indicated per patient sample. b, Library fragment size distribution for samples Pt-02 and Pt-06, illustrating characteristic periodicity due to nucleosome positioning. c, Scatter plot showing the number of unique nuclear fragments versus TSS enrichment for samples Pt-02 and Pt-06, with dotted lines indicating the thresholds used for identification of high-quality cells (TSS enrichment score > 8 and log10[unique fragments] > 3.5). d, Lineage tree of HSPCs from a patient with essential thrombocythemia (ET)31. The phylogeny was built from 21,430 clonal SNVs detected within the single-cell expanded clones across the whole genome using CellPhy169. Terminal nodes are colored based on the mutation status of the JAK2 gene. Cell heteroplasmies for two mitochondrial mutations are shown in the heatmap at the right. e, Total number of cells pre and post mitochondrial imputation of genotyping assignments for sample Pt-02. f, UMAPs showing the gene accessibility scores of canonical marker genes used to identify progenitor cell type for each cluster for sample Pt-02. g, Heat map of differential gene accessibility scores (FDR < 0.05 and log2FC > 1) across all progenitor subclusters for Pt-02. h, Differential protein expression between wildtype and mutant cells within HSPC cluster of ruxolitinib-treated sample Pt-06. Black dots represent differentially expressed (family wise error rate [FWER] < 0.05 and absolute Dz-score > 1; Wilcoxon rank sum test followed by Bonferroni correction) cell surface proteins. i, CD90 (THY1 gene) chromatin accessibility scores of HSPCs (HSPC1 and HSPC2 clusters) per patient for either untreated or ruxolitinib-treated patients included in Fig. 5k.

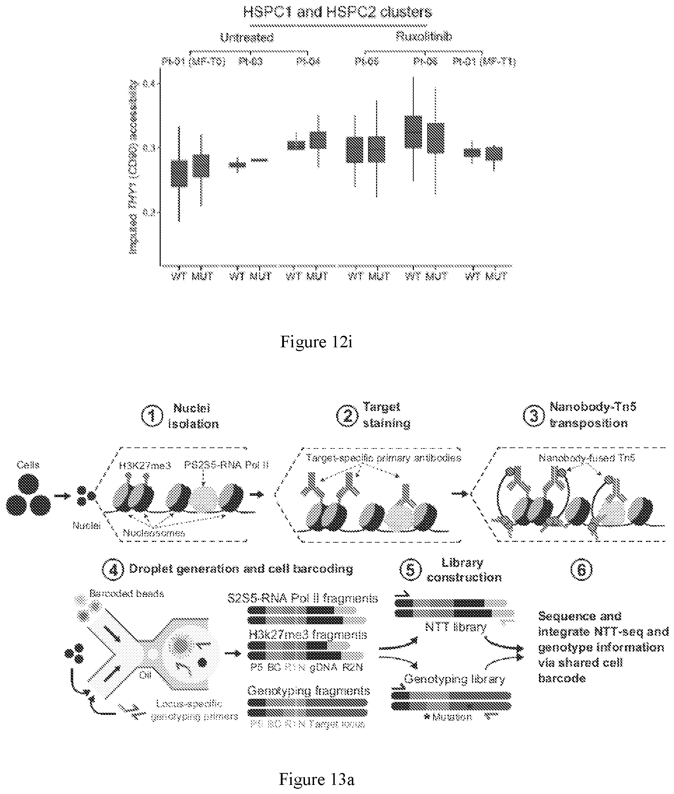

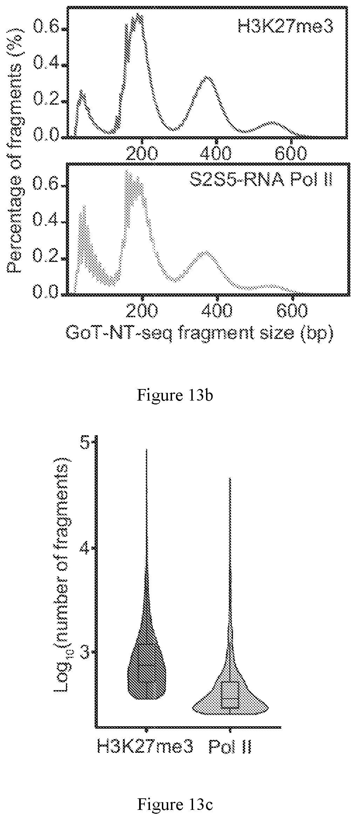

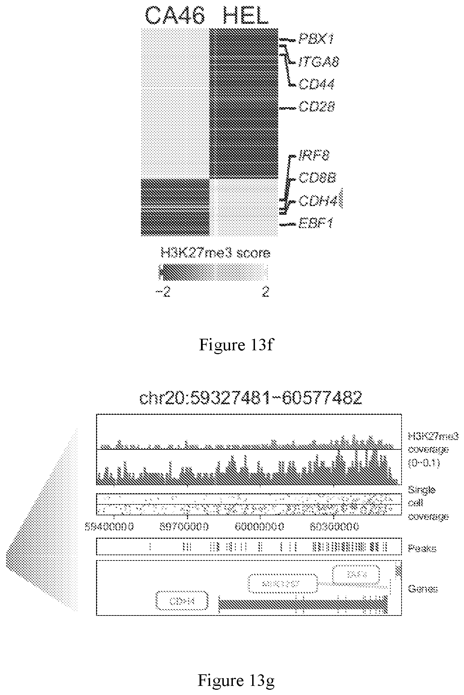

**Figures 13a-13h** shows development of GoT-NTT-seq. A) Schematic representation of the proposed method. B) Representative profiles of fragment size distribution for each target. C) Number of fragments per cell captured for each target. D) Normalized fragments per genomic 500 bp bin of H3K27me3 for each RNA Polymerase binding quantile (Wilcox rank sum test). E) Uniform manifold approximation (UMAP) in the H3K27me3 space. F) H3K27me3 scores for marker genes for each cell line (FDR < 0.05). G) Representative track of H3K27me3 signal for each cell line. H) UMAP with overlay genotyping information obtained by GoT-NTT-seq.

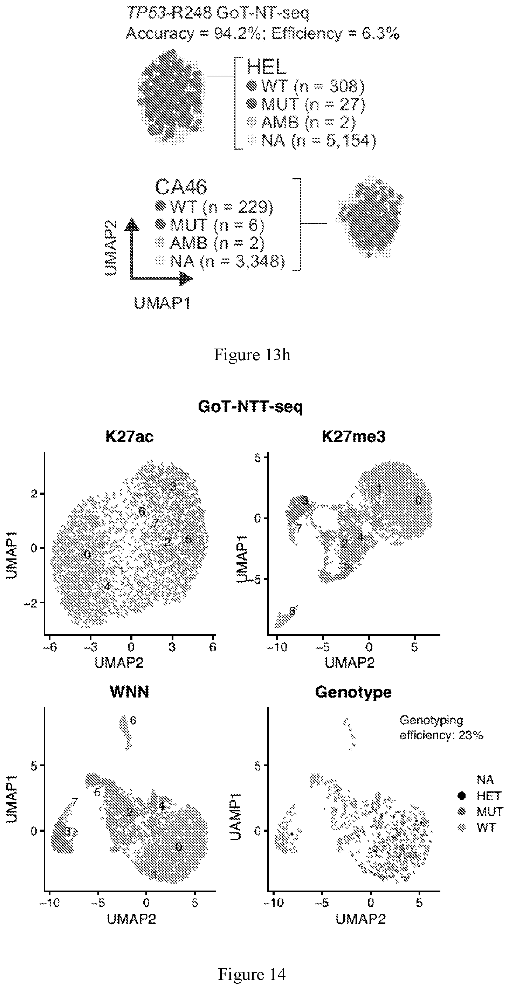

**Figure 14** shows schematic representation for the GoT-NTT-seq.

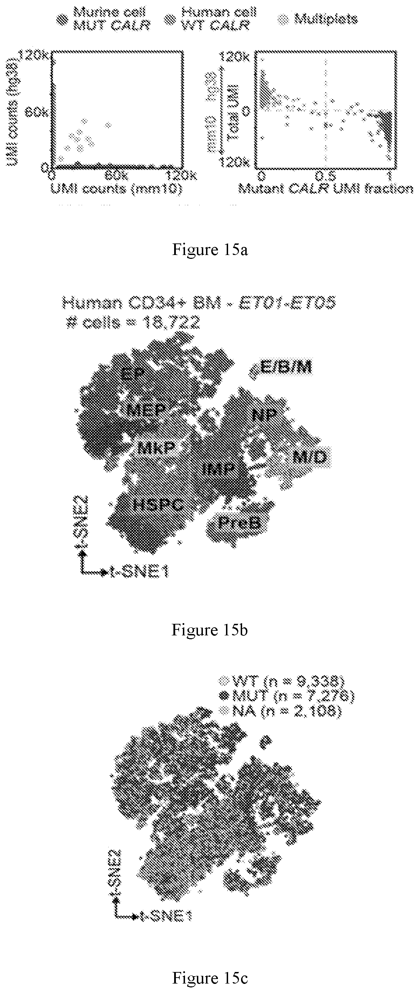

**Figures 15a-15h** show genotyping of transcriptomes. **A.** Species mixing shows accurate genotyping in genotyping of transcriptomes. **B-C.** CD34⁺ cells from ET patients clustered based on RNA expression (**B**), labeled with genotyping (**C**). **D.** Mutant cell frequency in progenitor subsets. **E.** Cell cycle expression in mutant and wildtype cells. **F.** Degree of cell cycle correlates with patient's platelet count. **G.** Volcano plots of differentially expressed genes between mutant and wildtype progenitors. **H.** GoT captures splice site of *XBP1.* Total *XBP1* and spliced *XBP1* in progenitors.

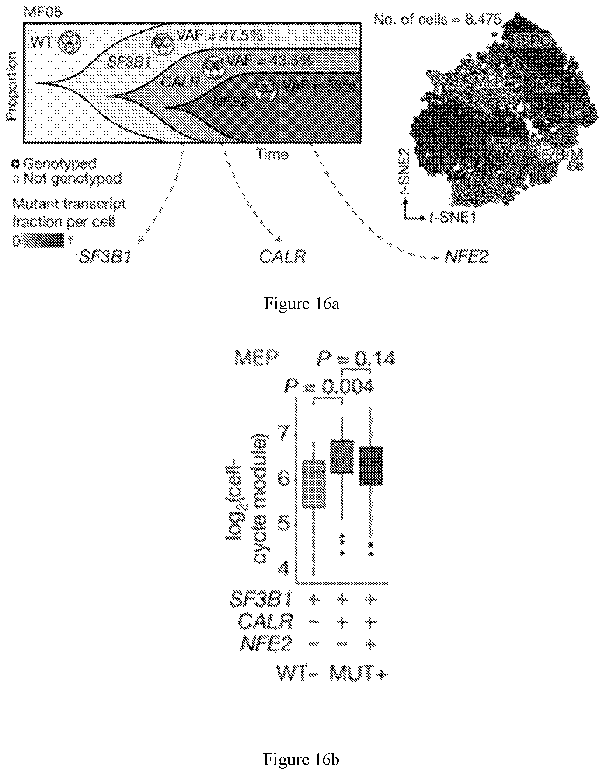

**Figures 16a-16b** shows targeting three mutations in parallel by bulk exon sequencing. **A.** Relative percentages of CALR, NFE2, SF3B1. **B.** *SF3B1*/*CALR-double* mutants exhibited increased proliferative advantage over *SF3B1*-single mutants while the triple-mutant did not increase cell cycle activation.

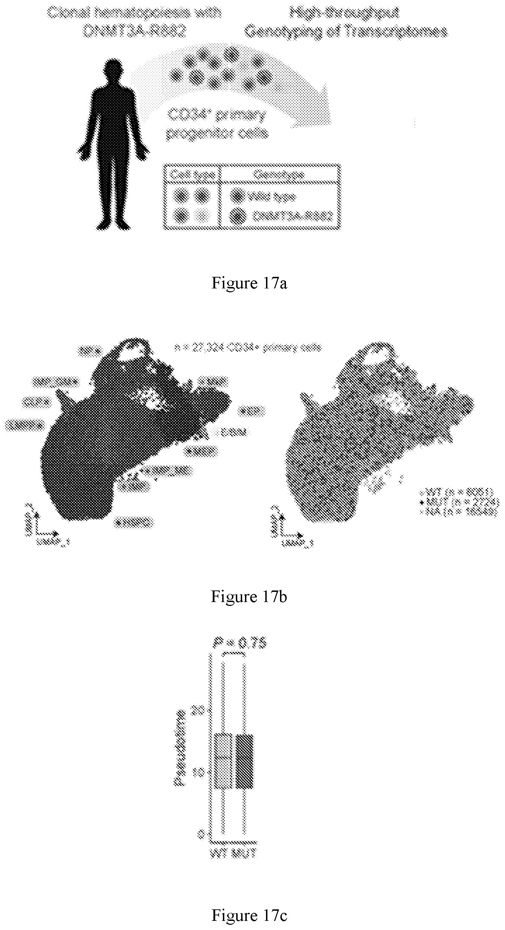

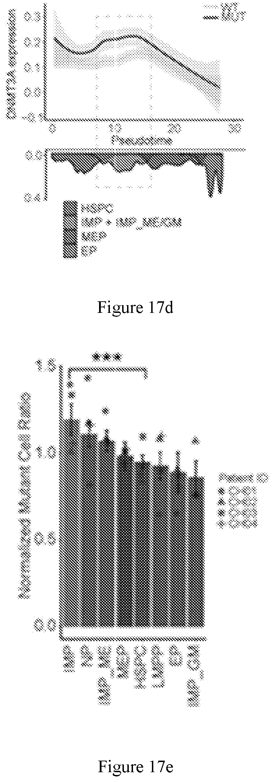

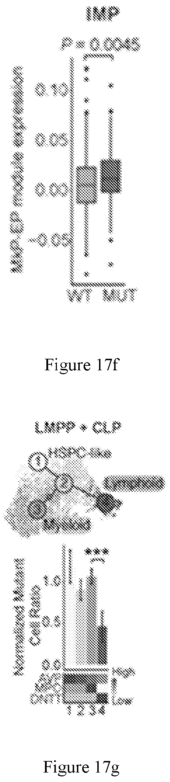

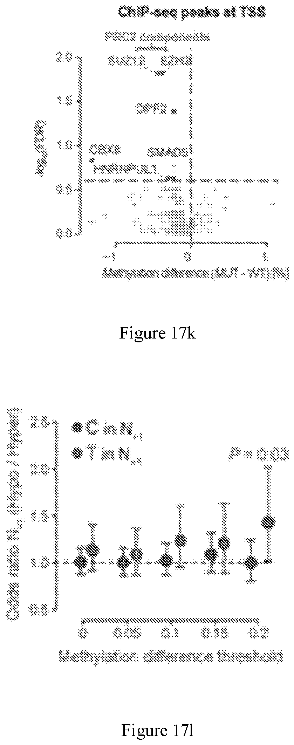

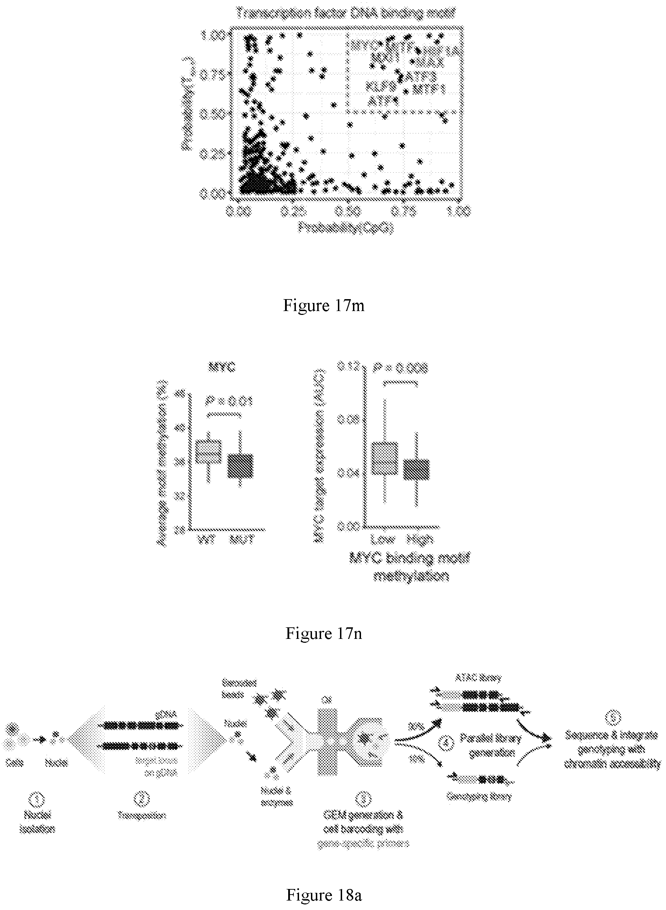

**Figures 17a-17n** show genotyping of transcriptomes. **A-B**. Genotyping of transcriptomes applied to DNMT3A human CD34+ samples, showing no global change in differentiation (**C**). **D-F**. DNMT3A in immature myeloid progenitors (IMPs) is associated with higher mutant cell frequency. **G**. DNMT3A mutant HSPCs show a bias towards lymphoid over myeloid differentiation. **H**. De novo differential expression analysis highlights aberrant transcriptional programs in DNMT3A CM. **I-J**. Single-cell methylomes show hypomethylation in DNMT3A mutants, particularly in CGI and PRC2 targets. **K**. *DNMT3A*^{R882} results in selective hypomethylation that preferentially impacts CpG islands and PRC2 targets **L-N.** DNMT3A-R882 shows preferential hypomethylation in CpGs followed by a T, a motif enriched in MYC binding with increase in transcriptional activity.

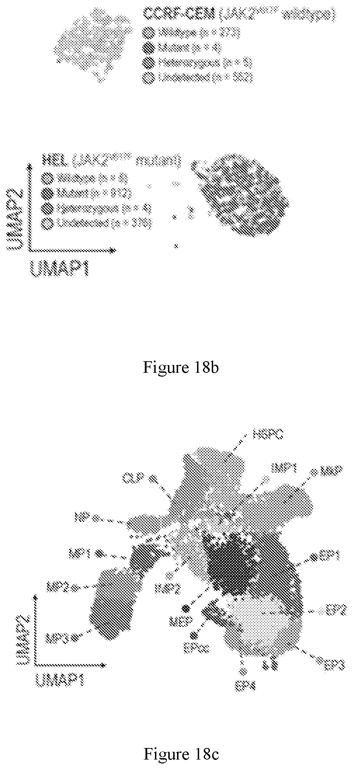

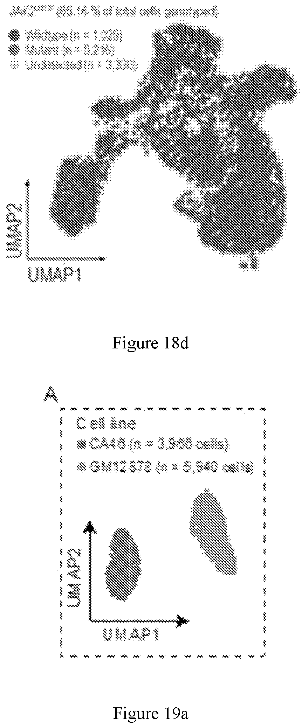

**Figures 18a-18d** show genotyping of specific loci. **A**. GoT-ChA captures scATAC-seq with genotyping of specific loci. **B**. Cell line mixing shows precision JAK2^{V617F} genotyping, with ~50% of cells genotyped. Application of GoT-ChA to JAK2^{V617F} MPN bone marrow sample shows high resolution of progenitor subsets using scATAC-seq information (**C**), together with high efficiency genotyping (**D**), demonstrating increased mutated cell frequency in erythroid progenitors.
**Figures 19a-19c** show GoT-ChA multi-plexed cell line. **A**. GoT-ChA multi-plexed cell line mixing showing accurate genotyping across 3 targets (**B-C**).

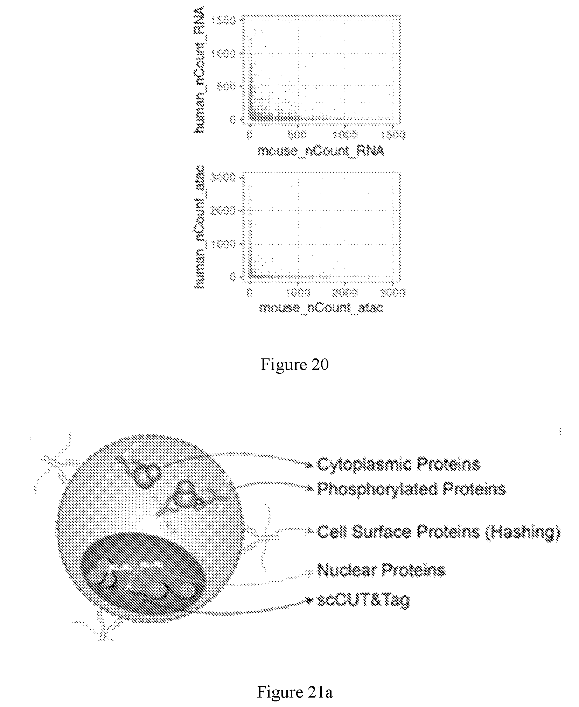

**Figure 20** shows species mixing plots for paired RNA and ATAC modalities for pilot ISSAAC-seq dataset.
**Figure 21a-21c** shows development of GoT-ChA-Pro. A) Schematic representation of the method for capturing intra-nuclear/cellular protein within the scATAC-seq framework. B) UMAP representation of 6,453 single cells from human brain organoids with paired profiles of chromatin accessibility and 64 intracellular protein panels. ATAC-based clustering reveals the presence of 10 neuronal lineages, while intracellular profiles characterize lineage-heterogeneity in both signaling proteins (pRPS6) and transcription factors (OTX2). C) Heterogeneity in protein.

### DETAILED DESCRIPTION

### General

The present disclosure is generally directed to methods for genotyping a locus. Such method comprises generating a plurality of biological particles, each biological particle comprising a plurality of nucleic acid fragments. The method further comprises generating a plurality of partitions, each partition comprising one of the plurality of biological particles comprising a plurality of nucleic acid fragments, each partition further comprising a primer pair and a barcoded bead. The primer pair may comprise a 5' primer-adapter nucleic acid molecule comprising a nucleotide sequence that is complementary to a sequence located at the 3' end of a specific locus within the nucleic acid fragment, and a 3' primer-adapter nucleic acid molecule comprising a nucleotide sequence that is complementary to a sequence located at the 5' end of the specific locus.

Currently, there are a small number of single-cell targeted genotyping strategies, which can be broadly split into two categories via the source material from which genotyping information is captured. The first group of methods identify wild type or mutant mRNA transcripts from the gene of interest to genotype individual cells. This approach pairs well with single-cell RNA sequencing (scRNA-seq) assays, as the material for genotyping is already captured and appended with unique cell barcodes with the standard protocol prior to any modifications made for targeted genotyping. These methods allow for direct assessment of the biological impact of somatic mutations in primary human patient samples, critically using a patient's own wild type cells for intra-sample comparisons and therefore accounting for potential technical or biological confounders. Given the low rate of transcript capture associated with droplet-based high-throughput methods, the associated genotyping methods are efficient only for targets that are highly expressed. In addition, current poly-A capture methods rely on reverse-transcription steps, and therefore capture only ~1.5 kb from the 3' end of the transcript. Therefore, mutations in genes that are lowly expressed, or whose mutation loci are located far from the ends of the molecule, the efficiency of these methods drops drastically.

The second group of methods utilizes genomic DNA as the source of genotyping information, and thus does not require mRNA transcripts to be captured (or even to be present) for successful genotyping. Notably, TARGET-seq, a plate-based targeted genotyping approach paired with single-cell RNA-seq, utilizes both mRNA and genomic DNA to great effect. As TARGET-seq is plate-based, gDNA that would normally not be barcoded in droplet-based scRNA-seq techniques is maintained in a separate physical well from other cells, allowing for greater flexibility for downstream targeted genotyping. However, this approach is applied at the expense of throughput, since increasing the scale of the production for capturing thousands of cells is labor-intensive and often not viable.

To surmount the challenges of resolving the admixture of wild type and mutant cells while simultaneously interrogating chromatin accessibility of thousands of single cells at a time, we developed Genotyping of Targeted loci with single cell Chromatin Accessibility (GoT-ChA). Due to the clear benefits of targeted genotyping using genomic DNA, we developed GoT-ChA to capture genotyping information directly from the genome rather than using mRNA transcripts. GoT-ChA fundamentally relies upon an in-droplet PCR reaction using custom primers to amplify a genomic region of interest, with nascent transcripts sequentially amplified with barcoded oligonucleotides prior to emulsion breakage. As such, GoT-ChA is adaptable to any system that utilizes in-droplet PCR to append cell barcodes onto nucleic acids of interest. For example, GoT-ChA is compatible with methods such as single-cell Cut&Tag, providing DNA-bound protein information or the genomic distribution of specific histone marks, transcription factors, or other aspects of chromatin that can be probed using an antibody approach (e.g., DNA breaks, R-Loops) along with single-cell genotyping. GoT-ChA is also compatible with various multi-omic methods, such as ASAP-seq, that are able to simultaneously obtain chromatin accessibility information, mitochondrial genome coverage, and both intra- and extracellular protein expression measurements via oligonucleotide-tagged antibodies. Critically, the additional multitude of mitochondrial variants captured with such methods may enable further genotyping imputation, provided adequate coverage of mitochondrial variants that are in-phase with GoT-ChA genotyping. Therefore, GoT-ChA can synergize with multiple single cell multi-omic methods to enable a multitude of differential analyses interrogating the effects of somatic mutations on various modalities of biological information, directly in human patient samples.

At the current time, GoT-ChA is the only high-throughput droplet-based single cell method that obtains targeted genotyping from gDNA along with chromatin accessibility or even histone modifications or DNA-binding proteins profiles. Critically, GoT-ChA captures the genotype information directly from genomic DNA, and thus obviates limiting dependencies in current methods, such as target localization and expression level, allowing high-throughput somatic genotype-to-phenotype mapping. GoT-ChA provides critical insights into the epigenetic features that shape cellular fates and how that regulation may be disrupted via somatic mutations, ultimately advancing our knowledge of how such mutations impact human health from benign maladies to overt malignancies.

In hematopoiesis, changes in chromatin accessibility define priming and commitment of hematopoietic precursors towards cellular fates. In turn, somatic mutations in hematopoietic stem and progenitor cells (HSPCs) drive the onset and progression of myeloid disorders, such as myeloproliferative neoplasms (MPNs), and reshape differentiation topologies.

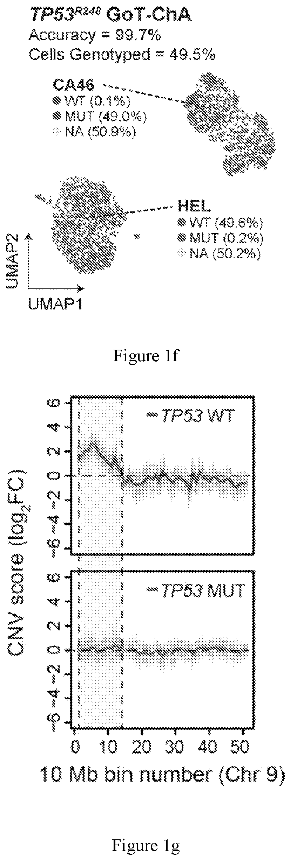

To chart how somatic mutations disrupt the epigenetic landscape in human clonal outgrowths, we developed Genotyping of Targeted loci with Chromatin Accessibility (GoT-ChA), linking genotypes to chromatin accessibility across thousands of single cells. Crucially, GoT-ChA captures genotypes directly from genomic DNA (**Figure 1a**), and thus independently of the genomic location or expression of the target. We tested GoT-ChA via cell line mixing studies targeting either *TP53^{R248Q}* (**Figure 1f**), or *JAK2^{V617F}* (**Figure 1f**), assigning cell line identity solely based on chromatin accessibility. Notably, GoT-ChA resulted in genotyping of 54% of cells with 96.9% accuracy for *TP53^{R248Q}* and 60% of cells with 99.7% accuracy for *JAK2^{V617F}.* These data show the transformative advantage of targeting DNA directly, as prior high-throughput droplet methods to target the lowly expressed *JAK2* via cDNA resulted in genotyping of only ~7% of cells (Nam et al, Nature, 2019).

Next, we applied GoT-ChA to CD34⁺ cells from *JAK2^{V617F}*-mutant myelofibrosis (MF) samples. We clustered cells based on chromatin profiles, revealing the expected cell populations in hematopoiesis, and then projected genotyping status onto the differentiation map. In further validation of genotyping accuracy, copy number inference showed a sample that contained a partial deletion of chromosome 20 concordant with our genotyping. Furthermore, GoT-ChA can be integrated with recent protocols to allow for high mitochondrial genome coverage (Lareau et al, Nature Biotechnology, 2020). We observed mitochondrial mutations that were highly concordant with *JAK2^{V617F}*, allowing genotyping of >85% of cells.

Within MPN samples, wildtype (WT) and mutated (MUT) cells were intermingled across the differentiation topology. Nonetheless, we observed an increase in the mutant fraction within erythroid progenitors (EP). Moreover, pseudo-temporal ordering of chromatin accessibility revealed that the mutant cell fraction increased along erythroid or megakaryocyte differentiation in untreated MPN, in line with clinical phenotypes.

Chromatin accessibility profiles can provide clues to the underlying regulatory network through transcription factor (TF) motif accessibility. Uniquely, GoT-ChA enables *de novo* differential motif accessibility, directly comparing WT and MUT cells coexisting within the same bone marrow. Mutant HSPCs showed increased motif accessibility (FDR < 0.05) for TFs associated with erythropoiesis, suggestive of increased erythroid priming. Within EP clusters, we observed increased motif accessibility of STAT5A and STAT5B, downstream targets of JAK2. These data demonstrated a cell-type specific effect of the *JAK2^{V617F}* mutation.

Ruxolitinib is a frontline JAK1/2 inhibitor for MF. Despite improvements in quality of life, ruxolitinib does not clearly target the MPN clone or prevent progression of disease. In ruxolitinib-treated patients the MUT cell fraction was uniformly distributed along the differentiation, demonstrating an abrogation of the fitness advantage of *JAK2^{V617F}* in committed progenitors, but not in HSPCs. Consistently, STAT5A motif accessibility remained increased in MUT cells at intermediate stages of erythroid maturation but decreased to similar levels as WT cells at later stages.

Overall, GoT-ChA radically expands the single-cell multi-omics toolkit and obviates limiting dependencies on target gene transcription, allowing high throughput somatic genotype-to-phenotype mapping. Applied to *JAK2^{V617F}*-mutated MPN, GoT-ChA uncovered a cell-type specific fitness advantage with erythroid commitment, that was reversed upon JAK2 inhibitor treatment. The reshaping of the differentiation topography traced back to differential transcription factor activity driving uncommitted vs. committed JAK2^{V617F} progenitors. Thus, single-cell multi-omics with GoT-ChA enables to chart the epigenetic underpinnings of hematopoietic clonal outgrowth.

As will be appreciated by those of skill in the art, GoT-ChA is generally compatible with any sequencing or genotyping approach that uses partitions (such as droplets or beads) and barcodes to sequence multiple genomes in parallel, such as single-cell sequencing techniques. In certain embodiments, GoT-ChA may be used in combination with Cut&Tag (Nat Commun. 2019 Apr 29;10(1):1930), ISSAAC-seq (world wide web at doi.org/10.1101/2022.01.16.476488), scifi-RNAseq (Nat Methods. 2021 Jun;18(6):635-642.), Single cell CRISPR screening, and Single cell lineage tracing.

### Clonal mosaicism (CM).

Clonal driver mutations fuel clonal outgrowths in normal tissue mosaicism. Somatic evolution has long been appreciated to be a central feature of malignancy, enabling adaptation to therapeutic pressures. Recently, exciting data showed that somatic evolution is ubiquitously found in normal human tissues, with massive clonal expansions, harboring somatic mutations in known cancer drivers (e.g., DNMT3A and SF3B1 in clonal hematopoiesis(CH);TP53 and NOTCH1 in esophageal mosaicism). Thus, somatic driver mutations provide fitness advantage allowing clonal outgrowth, not only in cancer, but also in normal tissue mosaicism. We therefore posit that the ability to link clonal genotypes to cellular phenotypes is a central challenge in clonal mosaicism (CM), critical to the understanding of how driver mutations provide fitness advantage leading to selection and clonal outgrowth.

Linking clonal genotypes to fitness-enhancing phenotypes in human samples is curtailed by current technological limitations. While approaches such as cell culture and murine models have enabled mechanistic elucidation of how somatic mutations confer growth advantage, models are lacking in many tissue types and may not reflect the evolutionary processes that occur in humans over decades. These limitations have inspired the research community to pursue large-scale profiling of primary human tissues with high-throughput platforms such as exome, RNA and epigenomics sequencing. However, these approaches are challenged in addressing the question of clonal fitness advantage, as clonal admixtures are not captured by bulk sequencing methods. Thus, to date, efforts to chart clonal outgrowths in normal human tissues have been limited to genotyping, and therefore we have little information directly from human samples about how mutations drive clonal growth. This is due in part to the fact that clones in normal tissues often affect only a small fraction of cells, and lack distinguishing surface markers amenable to enrichment by flow sorting. While recent single-cell RNA-seq (scRNA-seq) technologies have partially overcome this limitation by mapping differentiation processes at single-cell resolution, these methods cannot concurrently capture the genotype to allow isolation of specific mutants for analysis. Thus, there is an urgent unmet need in the field of clonal mosaicism to develop methods for multi-omics profiling in single cells to overlay somatic mutational genotyping with downstream epigenome, transcriptome or protein information.

Single-cell multi-omics technology innovation links somatic mutations to downstream epigenetic and transcriptional phenotypes. To address this challenge, we have developed plate-based methods for multi-omic single-cell technologies capable of capturing multiple layers of information from the same single cells. To address the specific challenge of genotyping in scRNA-seq at the high throughput needed for the study of CM, we developed genotyping of transcriptomes. This method genotypes transcripts (cDNA-based) containing somatic mutations together with scRNAseq, providing the ability to study the transcriptional impact of somatic mutations at single-cell resolution. Importantly, it turns the admixture of mutant and wildtype cells from a limitation to an advantage, enabling the direct comparison of mutant and wildtype cells within the same individual, overcoming individual-specific confounders in human studies. Applied to CALR mutation-driven clonal outgrowths in the human bone marrow, our studies revealed the differential fitness impact of the CALR mutations as a function of cell identity, discoveries that could only have been made due to this unique technology. We have also applied this platform to clonal mosaicism samples in hematopoiesis to define the impact of DNMT3A and SF3B1 mutations on human hematopoietic differentiation topography. Collectively, joint capture of somatic genotypes and phenotypes enables charting the effect of mutations on fitness as a function of cell identity.

Expanding the Genotyping of Targeted loci (GoT) toolkit to study clonal heterogeneity in human sample CM. The GoT toolkit is expanded to enable comprehensive study of the phenotypic consequences of somatic clonal genotypes in human normal tissues. First, given the high frequency of epigenetic and transcription factor mutations in CM, deciphering the mechanisms underlying clonal outgrowth requires genotype-aware high-throughput single-cell chromatin accessibility studies. We therefore developed a prototype to targeted single-cell genotyping (gDNA-based) in the context of Chromatin Accessibility (GoT-ChA) to jointly genotype driver mutations in single-cell Assay for Transposase-Accessible Chromatin by sequencing (scATAC-seq). GoT-ChA has two unique advantages in the context of CM: (i) as genotyping is done with gDNA it obviates limiting dependencies on targeted locus transcription, expanding the reach of single-cell multi-omics to any mutations in the genome, and (ii) it is compatible with single nuclei studies, critical for scalable application to solid human tissues. GoT-ChA is optimized to a CM across tissues and add multiplexing capabilities to target multiple mutations. We leverage the transposase-enabled aspect of GoT-ChA as a backbone to enable profiling across other epigenetic marks such as histone modification leveraging single cell Cut & Tag approaches. Last, to expand genotype-phenotype linkage, we integrate GoT-ChA with single cell transcriptome and protein profiling. We develop the molecular and analytic tools to enable production scale capabilities for the GoT toolkit. Collectively, our work allows linking CM genotypes with broad phenotyping across epigenetic, transcriptomic and protein profiling.

### Definitions

As used herein the specification, "a" or "an" may mean one or more. As used herein in the claim(s), when used in conjunction with the word "comprising", the words "a" or "an" may mean one or more than one. For example, reference to an "antibody" is a reference from one to many antibodies. As used herein "another" may mean at least a second or more.

An "*isolated*" molecule or cell is a molecule or a cell that is identified and separated from at least one contaminant molecule or cell with which it is ordinarily associated in the environment in which it was produced. Preferably, the isolated molecule or cell is free of association with all components associated with the production environment. The isolated molecule or cell is in a form other than in the form or setting in which it is found in nature. Isolated molecules therefore are distinguished from molecules existing naturally in cells; isolated cells are distinguished from cells existing naturally in tissues, organs, or individuals.

An "*isolated*" nucleic acid molecule is a nucleic acid molecule that is identified and separated from at least one contaminant nucleic acid molecule with which it is ordinarily associated in the environment in which it was produced. Preferably, the isolated nucleic acid is free of association with all components associated with the production environment. The isolated nucleic acid molecules encoding the polypeptides and antibodies herein is in a form other than in the form or setting in which it is found in nature. Isolated nucleic acid molecules therefore are distinguished from nucleic acids encoding any polypeptides and antibodies herein that exist naturally in cells.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term "biological sample" encompasses a clinical sample, and also includes cells in culture, cell supernatants, cell lysates, serum, plasma, biological fluid, and tissue samples. The term "biological sample" includes urine, saliva, cerebrospinal fluid, interstitial fluid, ocular fluid, synovial fluid, blood fractions such as plasma and serum, and the like. The term "biological sample" also includes solid tissue samples, tissue culture samples, and cellular samples.

The term "sample," as used herein, generally refers to a biological sample of a subject. The biological sample may comprise any number of macromolecules, for example, cellular macromolecules. The sample may be a cell sample. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears.

The term "biological particle," as used herein, generally refers to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or include a chromosome or other portion of a genome. The biological particle may be or may include a matrix (e.g., a gel or polymer matrix) comprising a cell or one or more constituents from a cell (e.g., cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix.

The term "*vector*," as used herein, is intended to refer to a nucleic acid molecule capable of transporting another nucleic acid to which it has been linked. One type of vector is a "plasmid," which refers to a circular double stranded DNA into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors," or simply, "expression vectors." In general, expression vectors useful in recombinant DNA techniques are often in the form of plasmids. In the present specification, "plasmid" and "vector" may be used interchangeably as the plasmid is the most commonly used form of vector.

"*Polynucleotide*," or "*nucleic acid*," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.*, methyl phosphonates, phosphotriesters, phosphoamidates, carbamates, *etc.*) and with charged linkages (*e.g.,* phosphorothioates, phosphorodithioates, *etc*.)*,* those containing pendant moieties, such as, for example, proteins (*e.g.,* nucleases, toxins, antibodies, signal peptides, ply-L-lysine, *etc*.), those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc*.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR₂ ("amidate"), P(O)R, P(O)OR', CO, or CH₂ ("formacetal"), in which each R or R' is independently H or substituted or unsubstituted alkyl (1-20 C) optionally containing an ether (-O-) linkage, aryl, alkenyl, cycloalkyl, cycloalkenyl or aralkyl. Not all linkages in a polynucleotide need be identical. The preceding description applies to all polynucleotides referred to herein, including RNA and DNA.

*A* "*host cell*" includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected *in vivo* with a polynucleotide(s) of this disclosure.

The term "*subject*" as used herein refers to a living mammal and may be interchangeably used with the term "patient". Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. The term does not denote a particular age or gender.

The term "culturing" refers to the in vitro propagation of cells or organisms on or in media of various kinds. It is understood that the descendants of a cell grown in culture may not be completely identical (i.e., morphologically, genetically, or phenotypically) to the parent cell. By "expanded" is meant any proliferation or division of cells.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The term "genome," as used herein, generally refers to genomic information from a subject, which may be, for example, at least a portion or an entirety of a subject's hereditary information. A genome can be encoded either in DNA or in RNA. A genome can comprise coding regions (e.g., that code for proteins) as well as non-coding regions. A genome can include the sequence of all chromosomes together in an organism. For example, the human genome ordinarily has a total of 46 chromosomes. The sequence of all of these together may constitute a human genome.

The terms "adaptor(s)," "adapter(s)," "adaptor molecule(s)," and "tag(s)" may be used synonymously. An adaptor or tag can be coupled to a polynucleotide sequence to be "tagged" by any approach, including ligation, hybridization, or other approaches. Adaptors may also be used to refer to a nucleic acid sequence or segment, such as a functional sequence. These adaptors may comprise nucleic acid sequences that may add a function, e.g., spacer sequence, primer sequencing site, barcode sequence, unique molecular identifier sequence, etc.

The terms "primer sequencing site" and "sequencing primer sequence" may be used interchangeably herein. Primer sequencing sites generally refer to nucleic acid sequences that can be used for sequencing.

The term "sequencing," as used herein, generally refers to methods and technologies for determining the sequence of nucleotide bases in one or more polynucleotides. Polynucleotides may comprise nucleic acid molecules, such as deoxyribonucleic acid (DNA) or ribonucleic acid (RNA), including variants or derivatives thereof (e.g., single-stranded DNA). Sequencing can be performed by various systems currently available, such as, without limitation, a sequencing system by Illumina^{®}, Pacific Biosciences (PacBio^{®}), MGI, Complete Genomics, Oxford Nanopore^{®}, or Life Technologies (Ion Torrent^{®}). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. Sequencing can comprise short-read sequencing or long-read sequencing, or both. In some situations, systems and methods provided herein may be used with proteomic information.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The term "real time," as used herein, can refer to a response time of less than about 1 second, a tenth of a second, a hundredth of a second, a millisecond, or less. The response time may be greater than 1 second. In some instances, real time can refer to simultaneous or substantially simultaneous processing, detection or identification.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

### Methods of genotyping a locus

The present disclosure is generally directed to methods for genotyping a locus. Such method comprises generating a plurality of biological particles, each biological particle comprising a plurality of nucleic acid fragments. The method further comprises generating a plurality of partitions, each partition comprising one of the plurality of biological particles comprising a plurality of nucleic acid fragments, each partition further comprising a primer pair and a barcoded bead. The primer pair may comprise a 5' primer-adapter nucleic acid molecule comprising a nucleotide sequence that is complementary to a sequence located at the 3' end of a specific locus within the nucleic acid fragment, and a 3' primer-adapter nucleic acid molecule comprising a nucleotide sequence that is complementary to a sequence located at the 5' end of the specific locus.

The present disclosure is generally directed to methods for generating a barcoded nucleic acid fragment. The method comprises contacting a plurality of biological particles comprising chromatin with a transposase to generate a plurality of nucleic acid fragments of said chromatin. The method may further comprise partitioning said plurality of biological particles comprising said plurality of nucleic acid fragments, a plurality of primer pairs, and a plurality of barcoded beads into a plurality of partitions. A partition of said plurality of partitions may comprise a biological particle of said plurality of biological particles, one or more primer pair of the plurality of primer pairs, and a barcoded bead of said plurality of barcoded beads. Said biological particle may comprise a nucleic acid fragment of said plurality of nucleic acid fragments. Said barcoded bead may be attached to a plurality of nucleic acid barcode molecules comprising a common barcode sequence. The primer pairs comprises: a 5' primer-adapter nucleic acid molecule comprising a nucleotide sequence that is complementary to a sequence located at the 3' end of a specific locus within the nucleic acid fragment, and a 3' primer-adapter nucleic acid molecule comprising a nucleotide sequence that is complementary to a sequence located at the 5' end of the specific locus. The method may further comprise amplifying the specific locus. The method may further comprise generating a barcoded nucleic acid fragment using the amplified specific locus and a nucleic acid barcode molecule of said plurality of nucleic acid barcode molecules.

A critical challenge in studying the phenotypes of clonal expansions, in both healthy tissues and overt malignancies, is that primary human samples are often composed of an admixture of wildtype and mutant cells. Thus, precision mapping of genotypes to phenotypes is obscured. Additionally, bulk population measurements largely aggregate heterogeneous groups of cells, hindering the identification of cell type- or cell state-specific phenotypes arising from the presence of somatic mutations. To circumvent these limitations, single cell simultaneous capture of genotypes together with phenotypic information is required, enabling intrasample, cell type-specific mutant to wildtype comparisons. Application of such single cell multi-omic approaches have shown that somatic mutations in human tissues exert a differing phenotypic effect as a function of cell state.

While droplet-based single cell technologies allow high-throughput linkage of cDNA-captured mutated genotypes with phenotypes, similar high-throughput methods for linking somatic genotypes with epigenetic profiles are lacking. Furthermore, cDNA based capture results in limiting dependencies on target gene expression and the distance of the locus from transcript end. For example, these dependencies led to inefficient capture of the lowly expressed JAK2^{V617F} locus (<10% of cells), requiring gDNA capture via lower throughput plate-based single cell sequencing. The dependency on gene expression also limits the application to archival frozen tissues, as nuclei isolation further decreases the number of available mRNA molecules for genotyping. Collectively, these limitations restrict the ability to profile key lowly expressed mutations, as well as mutations leading to nonsense-mediated mRNA decay or affecting non-coding regions.

GoT-ChA addresses this challenge by delivering droplet-based, broadly available, high-throughput joint capture of genotypes and chromatin accessibility. We further show that GoT-ChA can be readily integrated with protein and mitochondrial DNA capture, enabling robust linkage of somatic genotypes to a variety of signals at single cell resolution. As GoT-ChA is based on gDNA rather than cDNA capture, it also obviates the limiting dependencies on mutated locus expression and location. Thus, GoT-ChA enables the interrogation of somatic mutations throughout the genome, and radically expands the range of human biological phenomena that can be investigated for epigenetic deregulation due to somatic mutations. Importantly, the ability to apply GoTChA to nuclei opens the possibility for application to archived frozen solid tissues or tumors, critical for the exciting emerging field of clonal mosaicism across human tissues.

To leverage the unique ability to chart the impact of somatic mutation on epigenetic differentiation landscapes, we focused on JAK2^{V617F}-driven clonal expansions in primary human samples from PV and MF patients. These data revealed that the epigenetic consequences of the JAK2^{V617F} mutation are highly cell state dependent. Indeed, the frequency of mutated cells expanded at the stage of committed erythroid and megakaryocytic progenitors, consistent with clinical phenotypes, and demonstrates that the clonal representation of this mutation varies by differentiation stage and fate. Notably, the ability to profile single-cell chromatin landscapes allowed to examine patterns of HSPC priming and demonstrated that increases in the frequencies of mutated committed progenitors were heralded by increased accessibility of erythroid transcription factor motifs already in mutated HSPCs, consistent with aberrant lineage priming in MPN initiating cells.

JAK2V617F myeloproliferation is characterized by the presence of an inflammatory microenvironment, driving bone marrow fibrosis and extramedullary hematopoiesis. Previous work has linked JAK2- mediated activation of STAT1 and STAT3 to increased NF-kB signaling in mouse models, and highlighted cell-extrinsic effects of the microenvironment. Here, we show that the JAK2^{V617F} mutant HSPCs also display epigenetic profiles that are consistent with cell-intrinsic pro-inflammatory phenotypes, with increased motif accessibility of NF-kB-, AP-1-, and TGF-b-associated transcription factors in mutant cells. The observation that pro-inflammatory phenotypes are, at least to some degree, linked directly to JAK2^{V617F} in a cell-intrinsic fashion opens an avenue for potential combined therapeutic strategies for mutant-specific targeting, aimed at both JAK2^{V617F} constitutive activation as well as pro-inflammatory signaling in mutant HSPCs. In another striking demonstration of cell-type specificity in mutational impact, JAK2^{V617F} megakaryocytic progenitors, which produce mature megakaryocytes thought to drive characteristic marrow fibrosis through pro-fibrotic cytokine signaling, showed a proinflammatory landscape specific for AP-1 transcription factor activity, which has been linked with the fibrotic clinical phenotype of MF. In contrast, early HSPCs (HSPC1) showed broad, pro-inflammatory signature characterized not only by increased AP-1-, but also NF-kB- and TGF-b-associated transcription factor motif accessibility. The study of paired samples from a PV patient who progressed to MF showed that many of the changes described above, including increased proinflammatory NF-kB and AP-1 motif accessibility in HSPCs and aberrant regulation of the g-globin locus in EPs, are already evident long before significant marrow fibrosis occurs, providing human data support to the causal role of JUN/FOS in inducing fibrosis. These data suggest that JAK2^{V617F}-mediated inflammation and fibrosis results from a complex interplay between cell-extrinsic and cell-intrinsic effects that vary across different progenitor populations.

Interestingly, we observed a near complete loss of differential accessibility signals between mutant and wildtype cells within patients treated with ruxolitinib therapy, including reversion of intrinsic proinflammatory phenotypes and differentiation biases. However, ruxolitinib treatment does not eliminate JAK2^{V617F} mutant HSPCs. This is in line with the observation that while ruxolitinib reduces splenomegaly and disease symptoms resulting in overall improvement in quality of life, it fails to prevent disease progression or eliminate the mutated clones in MPNs. Thus, while ruxolitinib treatment appears to abrogate JAK2^{V617F}-mediated shifts in the epigenetic landscape of HSPCs, mutated cells may continue to promote disease progression through clonal evolution, even in the context of JAK inhibition. Thus, improved JAK2 inhibition for elimination of mutated cells may be critical for the prevention of disease progression.

While our cell mixing study directly testing heterozygous genotyping demonstrated a 34% rate of complete allelic capture, incomplete capture of targeted alleles during in-droplet genotyping reduces the capacity for classification of heterozygous cells. While MF patients tend to have homozygous JAK2^{V617F} mutations, allelic dropout will result in misclassification of heterozygous cells as either homozygous wildtype or mutant. Nonetheless, genotype misclassifications would dilute the strength of the biological differences between genotypes, and thus the differential epigenomic alterations reported here likely serve as a lower bound to effect sizes. Further, application of GoT-ChA-ASAP in samples with in-phase mitochondrial variants may allow for inference genotyping, decreasing the impact of allelic dropout. In addition, although our multi-modal approach expanding GoT-ChA allows to link genotype to both epigenetic changes and to protein expression levels, it does not provide simultaneous capture of transcriptional information, thus limiting the assessment of mutational impact on gene expression. While the 10x Genomics Multiome platform obtains both chromatin accessibility and gene expression information, the cell barcoding reaction does not utilize in-droplet PCR and thus precludes the usage of GoT-ChA. However, development of alternative multiomic technologies that retain an indroplet barcoding PCR, such as ISSAAC-seq that utilizes the 10x Genomics scATAC-seq platform as a foundation for simultaneous scRNA-seq, provides promising avenues for linking gene expression changes to mutation-specific epigenetic alterations assayed via GoT-ChA. Furthermore, given that the transposase-enabled scATAC-seq has been leveraged to expand the range of assayable epigenomic profiles to other modalities such as histone modifications, GoT-ChA has the potential to link genotypes with additional epigenetic features and therefore provide a more comprehensive understanding of the cellular phenotypes driven by somatic mutations.

Collectively, disclosed herein is a powerful novel single-cell multiomic approach that allows for the direct investigation of the impact of somatic mutations on chromatin accessibility in primary human patient samples. These data show that the JAK2^{V617F} somatic mutation, central to MPN pathogenesis, leads to epigenetic rewiring, in a cell-intrinsic and cell typespecific manner. These results, thus, demonstrate the power of joint single-cell capture of genotypes and epigenomes for a high-resolution study of clonal outgrowths in primary human tissue. GoT-ChA may be of particular importance to the emerging field of clonal mosaicism, now understood to be ubiquitous across the human body. Clonal mosaicism arises when a post-zygotic mutational event is detectable in subpopulations of cells as an alternative genotype while not present in the germline genome. In non-malignant clonal mosaicism, previous investigations have been largely limited to genotyping, due to the inability to separate admixtures of wildtype and mutant cells for genotype-phenotype inferences in primary human samples. We envision that GoT-ChA will thus serve as a foundation for broad future explorations to uncover the critical link between mutated somatic genotypes and epigenetic alterations across human clonal outgrowths in malignant and non-malignant contexts.

"Transposase" is an enzyme that binds to the end of a transposon and catalyses its movement to another part of the genome by a cut and paste mechanism or a replicative transposition mechanism. Any suitable transposase may be used in the method described herein, such as Tn5. For example, a transposase may be a fusion protein with various functional domains in addition to the endogenous accessible chromatin binding/cutting activity. Some examples may include Tn5 fusions with dead Cas9 enzymes for genomic targeting of transposase activity, or Tn5 fusions with protein A/G which can be used in Cut&Tag assays to tagment regions where primary antibodies against histone marks or transcription factors are bound, providing information about genome-wide histone mark distribution or TF activity in conjunction with targeted genotyping.

A "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes.

Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, strand of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

A "probe" or a "target," when used in reference to a nucleic acid or sequence of a nucleic acids, is intended as a semantic identifier for the nucleic acid or sequence in the context of a method or composition, and does not limit the structure or function of the nucleic acid or sequence beyond what is expressly indicated.

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, e.g., spacer sequences, primer sequences/sites, barcode sequences, unique molecular identifier sequences.

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

A "primer" is a single-stranded nucleic acid sequence having a 3' end that can be used as a chemical substrate for a nucleic acid polymerase in a nucleic acid extension reaction. RNA primers are formed of RNA nucleotides, and are used in RNA synthesis, while DNA primers are formed of DNA nucleotides and used in DNA synthesis. Primers can also include both RNA nucleotides and DNA nucleotides (e.g., in a random or designed pattern). Primers can also include other natural or synthetic nucleotides described herein that can have additional functionality. In some examples, DNA primers can be used to prime RNA synthesis and vice versa (e.g., RNA primers can be used to prime DNA synthesis). Primers can vary in length. For example, primers can be about 6 bases to about 120 bases. For example, primers can include up to about 25 bases.

A "ligation" is a method of ligating two (or more) nucleic acid sequences that are in proximity with each other through enzymatic means (e.g., a ligase). In some embodiments, ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Pat. No. 7,264,929).

A wide variety of different methods can be used for ligating nucleic acid molecules, including (but not limited to) "sticky-end" and "blunt-end" ligations. Additionally, single-stranded ligation can be used to perform ligation on a single-stranded nucleic acid molecule. Sticky-end ligations involve the hybridization of complementary single-stranded sequences between the two nucleic acid molecules to be joined, prior to the ligation event itself. Blunt-end ligations generally do not include hybridization of complementary regions from each nucleic acid molecule because both nucleic acid molecules lack a single-stranded overhang at the site of ligation.

The terms "detectable label," "optical label," and "label" are used interchangeably herein to refer to a directly or indirectly detectable moiety that is associated with (e.g., conjugated to) a molecule to be detected, e.g., a capture probe or analyte. The detectable label can be directly detectable by itself (e.g., radioisotope labels or fluorescent labels) or, in the case of an enzymatic label, can be indirectly detectable, e.g., by catalyzing chemical alterations of a chemical substrate compound or composition, which chemical substrate compound or composition is directly detectable. Detectable labels can be suitable for small scale detection and/or suitable for high-throughput screening. As such, suitable detectable labels include, but are not limited to, radioisotopes, fluorophores, chemiluminescent compounds, bioluminescent compounds, and dyes.

The detectable label can be qualitatively detected (e.g., optically or spectrally), or it can be quantified. Qualitative detection generally includes a detection method in which the existence or presence of the detectable label is confirmed, whereas quantifiable detection generally includes a detection method having a quantifiable (e.g., numerically reportable) value such as an intensity, duration, polarization, and/or other properties. In some embodiments, the detectable label is bound to a feature or to a capture probe associated with a feature. For example, detectably labeled features can include a fluorescent, a colorimetric, or a chemiluminescent label attached to a bead (see, for example, Raj eswari et al., J. Microbiol Methods 139:22-28, 2017, and Forcucci et al., J. Biomed Opt. 10:105010, 2015.)

### Preparation of Biological Samples

A variety of steps can be performed to prepare a biological sample for analysis. Except where indicated otherwise, the preparative steps described below can generally be combined in any manner to appropriately prepare a particular sample for analysis.

### Tissue Sectioning

A biological sample can be harvested from a subject (e.g., via surgical biopsy, whole subject sectioning), grown in vitro on a growth substrate or culture dish as a population of cells, or prepared as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 micrometers thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 micrometers. Thicker sections can also be used if desired or convenient, e.g., at least 70, 80, 90, or 100 micrometers or more. Typically, the thickness of a tissue section is between 1-100 micrometers, 1-50 micrometers, 1-30 micrometers, 1-25 micrometers, 1-20 micrometers, 1-15 micrometers, 1-10 micrometers, 2-8 micrometers, 3-7 micrometers, or 4-6 micrometers, but as mentioned above, sections with thicknesses larger or smaller than these ranges can also be analysed.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

### Freezing

In some embodiments, the biological sample (e.g., a tissue section as described above) can be prepared by deep freezing at a temperature suitable to maintain or preserve the integrity (e.g., the physical characteristics) of the tissue structure. Such a temperature can be, e.g., less than -20° C., or less than -25° C., -30° C., -40° C., -50° C., -60° C., -70° C., -80° C. -90° C., -100° C., -110° C., -120° C., -130° C., -140° C., -150° C., -160° C., -170° C., -180° C., -190° C., or -200° C. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15° C., less than -20° C., or less than -25° C. A sample can be snap frozen in isopentane and liquid nitrogen. Frozen samples can be stored in a sealed container prior to embedding.

### Formalin Fixation and Paraffin Embedding

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

### Fixation

As an alternative to formalin fixation described above, a biological sample can be fixed in any of a variety of other fixatives to preserve the biological structure of the sample prior to analysis. For example, a sample can be fixed via immersion in ethanol, methanol, acetone, formaldehyde (e.g., 2% formaldehyde), paraformaldehyde-Triton, glutaraldehyde, or combinations thereof.

In some embodiments, acetone fixation is used with fresh frozen samples, which can include, but are not limited to, cortex tissue, mouse olfactory bulb, human brain tumor, human post-mortem brain, and breast cancer samples. In some embodiments, a compatible fixation method is chosen and/or optimized based on a desired workflow. For example, formaldehyde fixation may be chosen as compatible for workflows using IHC/IF protocols for protein visualization. As another example, methanol fixation may be chosen for workflows emphasizing RNA/DNA library quality. Acetone fixation may be chosen in some applications to permeabilize the tissue. When acetone fixation is performed, pre-permeabilization steps (described below) may not be performed. Alternatively, acetone fixation can be performed in conjunction with permeabilization steps.

### Embedding

As an alternative to paraffin embedding described above, a biological sample can be embedded in any of a variety of other embedding materials to provide a substrate to the sample prior to sectioning and other handling steps. In general, the embedding material is removed prior to analysis of tissue sections obtained from the sample. Suitable embedding materials include, but are not limited to, waxes, resins (e.g., methacrylate resins), epoxies, and agar.

### Staining

To facilitate visualization, biological samples can be stained using a wide variety of stains and staining techniques. In some embodiments, a sample can be stained using any number of biological stains, including but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin.

The sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the biological sample can be stained using a detectable label (e.g., radioisotopes, fluorophores, chemiluminescent compounds, bioluminescent compounds, and dyes) as described elsewhere herein. In some embodiments, a biological sample is stained using only one type of stain or one technique. In some embodiments, staining includes biological staining techniques such as H&E staining. In some embodiments, staining includes identifying analytes using fluorescently-conjugated antibodies. In some embodiments, a biological sample is stained using two or more different types of stains, or two or more different staining techniques. For example, a biological sample can be prepared by staining and imaging using one technique (e.g., H&E staining and brightfield imaging), followed by staining and imaging using another technique (e.g., IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destined by washing the sample in HCl. In some embodiments, destaining can include 1, 2, 3, or more washes in HCl. In some embodiments, destaining can include adding HCl to a downstream solution (e.g., permeabilization solution). As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905.

### Systems and Methods for Sample Compartmentalization

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion. A partition may comprise one or more other partitions.

A partition may include one or more particles. A partition may include one or more types of particles. For example, s partition of the present disclosure may comprise one or more biological particles and/or macromolecular constituents thereof. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead. A cell bead can be a biological particle and/or one or more of its macromolecular constituents encased inside of a gel or polymer matrix, such as via polymerization of a droplet containing the biological particle and precursors capable of being polymerized or gelled. Unique identifiers, such as barcodes, may be injected into the droplets previous to, subsequent to, or concurrently with droplet generation, such as via a microcapsule (e.g., bead), as described elsewhere herein. Microfluidic channel networks (e.g., on a chip) can be utilized to generate partitions as described herein. Alternative mechanisms may also be employed in the partitioning of individual biological particles, including porous membranes through which aqueous mixtures of cells are extruded into non-aqueous fluids.

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295 .

Emulsion systems for creating stable droplets in non-aqueous or oil continuous phases are described in, for example, U.S. Patent Application Publication No. 2010/0105112.

In the case of droplets in an emulsion, allocating individual particles to discrete partitions may in one non-limiting example be accomplished by introducing a flowing stream of particles in an aqueous fluid into a flowing stream of a non-aqueous fluid, such that droplets are generated at the junction of the two streams. Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In some cases, partitions among a plurality of partitions may contain at most one biological particle (e.g., bead, DNA, cell or cellular material). In some embodiments, the various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

### Beads

A partition may comprise one or more unique identifiers, such as barcodes. Barcodes may be previously, subsequently or concurrently delivered to the partitions that hold the compartmentalized or partitioned biological particle. For example, barcodes may be injected into droplets previous to, subsequent to, or concurrently with droplet generation. The delivery of the barcodes to a particular partition allows for the later attribution of the characteristics of the individual biological particle to the particular partition. Barcodes may be delivered, for example on a nucleic acid molecule (e.g., an oligonucleotide), to a partition via any suitable mechanism. Barcoded nucleic acid molecules can be delivered to a partition via a microcapsule. A microcapsule, in some instances, can comprise a bead. Beads are described in further detail below.

A bead may be porous, non-porous, solid, semi-solid, semi-fluidic, fluidic, and/or a combination thereof. In some instances, a bead may be dissolvable, disruptable, and/or degradable. In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer (µm), 5 µm, 10 µm, 20 µm, 30 µm, 40 µm, 50 µm, 60 µm, 70 µm, 80 µm, 90 µm, 100 µm, 250 µm, 500 µm, 1 mm, or greater. In some cases, a bead may have a diameter of less than about 10 nm, 100 nm, 500 nm, 1 µm, 5 µm, 10 µm, 20 µm, 30 µm, 40 µm, 50 µm, 60 µm, 70 µm, 80 µm, 90 µm, 100 µm, 250 µm, 500 µm, 1 mm, or less. In some cases, a bead may have a diameter in the range of about 40-75 µm, 30-75 µm, 20-75 µm, 40-85 µm, 40-95 µm, 20-100 µm, 10-100 µm, 1-100 µm, 20-250 µm, or 20-500 µm.

A bead may comprise natural and/or synthetic materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (e.g., co-polymers) thereof. Beads may also be formed from materials other than polymers, including lipids, micelles, ceramics, glass-ceramics, material composites, metals, other inorganic materials, and others.

In some instances, the bead may contain molecular precursors (e.g., monomers or polymers), which may form a polymer network via polymerization of the molecular precursors. In some cases, a precursor may be an already polymerized species capable of undergoing further polymerization via, for example, a chemical cross-linkage. In some cases, a precursor can comprise one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers. In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carbon-carbon bonds, thioether bonds, or carbon-heteroatom bonds.

Cross-linking may be permanent or reversible, depending upon the particular cross-linker used. Reversible cross-linking may allow for the polymer to linearize or dissociate under appropriate conditions. In some cases, reversible cross-linking may also allow for reversible attachment of a material bound to the surface of a bead. In some cases, a cross-linker may form disulfide linkages. In some cases, the chemical cross-linker forming disulfide linkages may be cystamine or a modified cystamine.

In some cases, disulfide linkages can be formed between molecular precursor units (e.g., monomers, oligomers, or linear polymers) or precursors incorporated into a bead and nucleic acid molecules (e.g., oligonucleotides). Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible cross-linkers). The disulfide linkages may permit the bead to be degraded (or dissolved) upon exposure of the bead to a reducing agent.

In some cases, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

In some cases, a bead may comprise an acrydite moiety, which in certain aspects may be used to attach one or more nucleic acid molecules (e.g., barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment can be reversible, such that when the disulfide bond is broken (e.g., in the presence of a reducing agent), the attached species is released from the bead. In other cases, an acrydite moiety can comprise a reactive hydroxyl group that may be used for attachment.

Functionalization of beads for attachment of nucleic acid molecules (e.g., oligonucleotides) may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule (e.g., oligonucleotide), which may include a priming sequence (e.g., a primer for amplifying target nucleic acids, random primer, primer sequence for messenger RNA) and/or one or more barcode sequences. The one more barcode sequences may include sequences that are the same for all nucleic acid molecules coupled to a given bead and/or sequences that are different across all nucleic acid molecules coupled to the given bead. The nucleic acid molecule may be incorporated into the bead.

In some cases, the nucleic acid molecule can comprise a functional sequence, for example, for attachment to a sequencing flow cell, such as, for example, a P5 sequence for Illumina^{®} sequencing. In some cases, the nucleic acid molecule or derivative thereof (e.g., oligonucleotide or polynucleotide generated from the nucleic acid molecule) can comprise another functional sequence, such as, for example, a P7 sequence for attachment to a sequencing flow cell for Illumina sequencing. In some cases, the nucleic acid molecule can comprise a barcode sequence. In some cases, the primer can further comprise a unique molecular identifier (UMI). In some cases, the primer can comprise an R1 primer sequence for Illumina sequencing. In some cases, the primer can comprise an R2 primer sequence for Illumina sequencing. Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (e.g., barcoded oligonucleotides), the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., a nucleic acid molecule, e.g., barcoded oligonucleotide) may result in release of the species from the bead.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent can break the various disulfide bonds, resulting in bead degradation and release of the barcode sequence into the aqueous, inner environment of the droplet. In another example, heating of a droplet comprising a bead-bound barcode sequence in basic solution may also result in bead degradation and release of the attached barcode sequence into the aqueous, inner environment of the droplet.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing nucleic acid molecule (e.g., oligonucleotide) bearing beads.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)). A bond may be cleavable via other nucleic acid molecule targeting enzymes, such as restriction enzymes (e.g., restriction endonucleases), as described further below.

Numerous chemical triggers may be used to trigger the degradation of beads. Examples of these chemical changes may include, but are not limited to pH-mediated changes to the integrity of a component within the bead, degradation of a component of a bead via cleavage of cross-linked bonds, and depolymerization of a component of a bead.

### Reagents

In accordance with certain aspects, biological particles may be partitioned along with lysis reagents in order to release the contents of the biological particles within the partition. In such cases, the lysis agents can be contacted with the biological particle suspension concurrently with, or immediately prior to, the introduction of the biological particles into the partitioning junction/droplet generation zone (e.g., junction 210), such as through an additional channel or channels upstream of the channel junction.

### Kits

Also described herein are kits comprising partitioning fluid, a barcoded bead, and a primer pair comprising: a 5' primer-adapter nucleic acid molecule comprising a nucleotide sequence that is complementary to a sequence located at the 3' end of a specific locus within the nucleic acid fragment, and a 3' primer-adapter nucleic acid molecule comprising a nucleotide sequence that is complementary to a sequence located at the 5' end of the specific locus. The kits may include one or more of the following: one, two, three, four, five or more, up to all of partitioning fluids, including both aqueous buffers and non-aqueous partitioning fluids or oils, nucleic acid barcode libraries that are releasably associated with beads, as described herein, microfluidic devices, reagents for disrupting cells amplifying nucleic acids, and providing additional functional sequences on fragments of cellular nucleic acids or replicates thereof, as well as instructions for using any of the foregoing in the methods described herein.

### EXAMPLES

The invention now being generally described, it will be more readily understood by reference to the following examples which are included merely for purposes of illustration of certain aspects and embodiments of the present invention, and are not intended to limit the invention.

### Example 1: Materials and Methods

### Cell lines.

Human CA46 (ATCC, #CRL-1648), HEL (ATCC, #TIB-180), and CCRF-CEM (ATCC, #CRM-CCL-119) cell lines were maintained according to standard procedures in RPMI-1640 (Thermo Fisher Scientific, #11-875-119) with 10% (or 20% for CA46 cells) FBS (Thermo Fisher Scientific, #10-437-028) at 37°C with 5% CO₂.

### Patient sample processing and CD34⁺ cell sorting.

Patient samples were obtained either as fresh peripheral blood or cryopreserved mononuclear cells isolated from bone marrow biopsies or peripheral blood. For fresh peripheral blood, mononuclear cells were isolated within 48 hours of blood collection utilizing a Ficoll (Thermo Fisher Scientific, #45-001-750) gradient according to manufacturer's recommendations. Isolated mononuclear cells were then resuspended in staining buffer (Biolegend, #420201) and incubated with Human TruStain FxC (10 minutes at 4°C; Biolegend, #422302) to block Fc receptor-mediated binding. Cells were then stained with a CD34-PE-Vio770 antibody (20 minutes at 4°C; Miltenyi Biotec, clone AC136, #130-113-180) and DAPI (Invitrogen, #D1306). The samples were then sorted for DAPI-negative, CD34-positive cells using a BD Influx cell sorter at the Weill Cornell Medicine Flow Cytometry Core.

### Single nucleus ATAC-seq with GoT-ChA.

*Nuclei isolation.* Cells of interest were first subjected to nuclei isolation. Briefly, cells were resuspended with lysis buffer (10 mM Tris-HCL (pH 7.4), 10 mM NaCl, 3 mM MgCl₂, 0.1% Tween-20, 0.1% Nonidet P40 Substitute, 0.01% Digitonin, 1% BSA) and incubated on ice (3 minutes for patient samples, 5 minutes for cell lines), followed by adding chilled wash buffer (10 mM Tris-HCL (pH 7.4), 10 mM NaCl, 3 mM MgCl₂, 1% BSA, 0.1% Tween-20) and centrifuging to pellet isolated nuclei. Nuclei were then resuspended in a Tris-based buffer for subsequent tagmentation and counted using trypan blue and a Countess II FL Automated Cell Counter.

*Tagmentation.* For a target recovery of 10,000 cells, 16,000 nuclei were tagmented in bulk using standard Nextera-loaded Tn5 transposase on a thermocycler with the following program: 37°C for 60 minutes, hold at 4°C.

*Single-cell emulsion generation.* After tagmentation, partitioning oil, uniquely barcoded gel beads, nuclei, and PCR reagent mixture containing the two locus-specific GoT-ChA primers (at a final concentration of 300 nM) were loaded onto a microfluidics chip to create a droplet-based single-cell emulsion. The two locus-specific GoT-ChA primers used are: GoT-ChA_R1N (primer with a Read 1N handle sequence: TCGTCGGCAGCGTCAGATGTGTATAAGAGACAG - [22bp locus specific]) and GoT-ChA_IS2 (locus-specific primer with an IS2 handle sequence: AGCAAGTGAGAAGCATCGTGTC - [22bp locus specific]). Critically, the gel beads are each coated with oligonucleotides that begin with the P5 Illumina sequencing adapter followed by a 16-nucleotide unique cell barcode and ending with a partial Read 1 Nextera sequence

(AATGATACGGCGACCACCGAGATCTACACNNNNNNNNNNNNNNNNNTCGTCGG
CAGCGTC).

*In-droplet cell barcoding.* After generation, the single-cell emulsion was transferred to a pre-chilled PCR strip tube and placed on a thermocycler for the following program: 72°C for 5 minutes; 98°C for 30 seconds; 12 cycles of 98°C for 10 seconds, 59°C for 30 seconds, 72°C for 1 minute; hold at 15°C.

*Emulsion cleanup and sample allocation for library construction.* After cell barcoding, the emulsion is broken and barcoded nucleic acids are cleaned twice using magnetic beads. The first cleanup step utilizes Dynabeads MyOne SILANE, while the second utilizes SPRIselect beads at a 1.2X size selection ratio of beads: sample, eluting in a total volume of 45 µL. 5 µL of sample is set aside for GoT-ChA library construction, while the remaining 40 µL are used for standard ATAC-seq library construction.

*ATAC-seq library construction.* The 40 µL set aside for ATAC-seq is subjected to a sample indexing PCR, utilizing a partial P5 primer (AATGATACGGCGACCACCGAGA) and a unique indexing primer beginning with the P7 Illumina sequencing adapter followed by an 8-nucleotide sample index and ending with a partial Read 2 Nextera sequence (CAAGCAGAAGACGGCATACGAGATXXXXXXXXGTCTCGTGGGCTCGG, "X" denotes user-defined sample index), with the following thermocycler program: 98°C for 45 seconds; 9 cycles (if targeting 10,000 cells; more cycles needed if a lower nuclei input was used) of 98°C for 20 seconds, 67°C for 30 seconds, 72°C for 20 seconds; 72°C for 1 minute, 4°C hold. After the indexing PCR, the sample is subjected to a 0.4X / 1.2X double-sided SPRIselect cleanup, eluting in a final volume of 20 µL.

*GoT-ChA library construction: hemi-nested PCR and streptavidin-biotin pull-down.* To generate the GoT-ChA library, two PCRs are performed, using the 5 µL set aside after the cell barcoding PCR cleanup step. The first PCR utilizes a hemi-nested primer design with partial P5 (binds the P5 Illumina sequencing handle: AATGATACGGCGACCACCGAGATCTACAC) and GoT-ChA_nested (a nested, biotinylated, locus specific primer with a TruSeq Small RNA Read 2 handle: /5BiosG/CCTTGGCACCCGAGAATTCCA-[22bp locus specific sequence, proximal to the mutation site relative to the GoT-ChA_IS2 primer binding site]) primers with the following thermocycler program: 95 °C for 3 min; 15 cycles of 95 °C for 20 s, 65°C for 30 s and 72°C for 20 s; followed by 72°C for 5 min and ending with hold at 4°C. After a 1.2X SPRIselect clean up, biotinylated PCR product is bound and isolated using Dynabeads M-280 Streptavidin magnetic beads (Thermo Fisher Scientific, #11206D). Briefly, beads are washed three times with 1X sodium chloride-sodium phosphate-EDTA buffer (SSPE, VWR, #VWRV0810-4L), added to the purified PCR product, and incubated at room temperature for 15 minutes. The beads are then washed twice with 1X SSPE buffer and once with 10 mM Tris-HCl (pH 8.0) before resuspending in water.

*GoT-ChA library construction: on-bead sample indexing.* The bead-bound fragments are then amplified and sample indexed using P5 and RPI-X (binds the TruSeq Small RNA Read 2 handle and adds a sample index and P7 Illumina sequencing handle: CAAGCAGAAGACGGCATACGAGATXXXXXXXXGTGACTGGAGTTCCTTGGCAC CCGAGAATTCCA, "X" denotes user-defined sample index) primers with the following thermocycler program: 95 °C for 3 min; 6-10 cycles of 95 °C for 20 s, 65°C for 30 s and 72°C for 20 s; followed by 72°C for 5 min and ending with hold at 4°C.

*Library QC and Sequencing Parameters.* Final libraries were quantified using a Qubit dsDNA HS Assay Kit (Thermo Fisher Scientific, #Q32854) and a High Sensitivity DNA chip (Agilent Technologies, #5067-4626) run on a Bioanalyzer 2100 system (Agilent Technologies) and sequenced on a NovaSeq 6000 at the Weill Cornell Medicine Genomics Resources Core Facility with the following parameters: paired-end 50 cycles; Read 1N 50 cycles, i7 Index 8 cycles, i5 Index 16 cycles, Read 2N 50 cycles. ATAC libraries were sequenced to a depth of 25,000 read pairs per nucleus, and GoT-ChA libraries were sequenced to 5,000 read pairs per nucleus.

### Single nucleus ATAC-seq with GoT-ChA (Modification).

Cells were subjected to nuclei isolation according to the Nuclei Isolation for Single Cell ATAC Sequencing protocol (version CG000169 Rev D, 10x Genomics). Briefly, cells were resuspended with lysis buffer (10 mM Tris-HCL (pH 7.4), 10 mM NaCl, 3 mM MgCl₂, 0.1% Tween-20, 0.1% Nonidet P40 Substitute, 0.01% Digitonin, 1% BSA) and incubated on ice (3 minutes for patient samples, 5 minutes for cell lines), followed by adding chilled wash buffer (10 mM Tris-HCL (pH 7.4), 10 mM NaCl, 3 mM MgCl₂, 1% BSA, 0.1% Tween-20) and centrifuging to pellet isolated nuclei. Nuclei were then resuspended in 1X diluted nuclei buffer (10x Genomics) and counted using trypan blue and a Countess II FL Automated Cell Counter.

Nuclei were subsequently processed according to the Chromium Next GEM Single Cell ATAC Solution user guide (version CG000209 Rev F, 10x Genomics) with the following modifications:
1. During the GEM Generation and Barcoding reaction (Step 2.1), 1 µL of 22.5 µM GoT-ChA primer mix was added to the barcoding reaction mixture. The primers used are GoT-ChA_R1N (locus-specific primer with a Read 1N handle sequence: TCGTCGGCAGCGTCAGATGTGTATAAGAGACAG - [22bp locus specific]) and GoT-ChA_IS2 (locus-specific primer with an IS2 handle sequence: AGCAAGTGAGAAGCATCGTGTC - [22bp locus specific]).
2. During the Post GEM Incubation Cleanup (Step 3.2), 45.5 µL of Elution Solution I is used to elute material from SPRIselect beads. 5 µL is used for GoT-ChA library construction, and the remaining 40 µL are used for ATAC library construction as indicated in the standard protocol.
3. To generate the GoT-ChA library, two additional PCRs were performed on the 5 µL set aside during Step 3.2 (Fig. 6c). The first PCR aims to amplify genotyping fragments prior to sample indexing and uses P5 (binds the P5 Illumina sequencing handle: AATGATACGGCGACCACCGAGATCTACAC) and GoT-ChA_nested (a nested, biotinylated, locus specific primer with a TruSeq Small RNA Read 2 handle: /5BiosG/CCTTGGCACCCGAGAATTCCA-[22bp locus specific]) primers with the following thermocycler program: 95 °C for 3 min; 15 cycles of 95 °C for 20 s, 65°C for 30 s and 72°C for 20 s; followed by 72°C for 5 min and ending with hold at 4°C. After a 1.2X SPRIselect clean up, biotinylated PCR product is bound and isolated using Dynabeads M-280 Streptavidin magnetic beads (Thermo Fisher Scientific, #11206D). Briefly, beads are washed three times with 1X sodium chloride-sodium phosphate-EDTA buffer (SSPE, VWR, #VWRV0810-4L), added to the purified PCR product, and incubated at room temperature for 15 minutes. The beads are then washed twice with 1X SSPE buffer and once with 10 mM Tris-HCl (pH 8.0) before resuspending in water. The bead-bound fragments are then amplified and sample indexed using P5 and RPI-X (binds the TruSeq Small RNA Read 2 handle and adds a sample index and P7 Illumina sequencing handle: CAAGCAGAAGACGGCATACGAGATXXXXXXXXGTGACTGGAGTTCCTT GGCACCCGAGAATTCCA, "X" denotes user-defined sample index) primers with the following thermocycler program: 95 °C for 3 min; 6-10 cycles of 95 °C for 20 s, 65°C for 30 s and 72°C for 20 s; followed by 72°C for 5 min and ending with hold at 4°C.

Final libraries were quantified using a Qubit dsDNA HS Assay Kit (Thermo Fisher Scientific, #Q32854) and a High Sensitivity DNA chip (Agilent Technologies, #5067-4626) run on a Bioanalyzer 2100 system (Agilent Technologies) and sequenced on a NovaSeq 6000 at the Weill Cornell Medicine Genomics Resources Core Facility with the following parameters: paired-end 50 cycles; Read 1N 50 cycles, i7 Index 8 cycles, i5 Index 16 cycles, Read 2N 50 cycles. ATAC libraries were sequenced to a depth of 25,000 read pairs per nucleus, and GoT-ChA libraries were sequenced to 5,000 read pairs per nucleus.

### GoT-ChA data processing.

Raw sequencing data for GoT-ChA libraries were demultiplexed using cellranger-ATAC mkfastq. The GoT-ChA sequencing reads were then input into a series of custom pre-processing functions designed to result in a genotype-per-cell output.

First, the "FastqSplit" function takes input FASTQ files and splits them into smaller files with a user-defined n reads for later parallelized processing. Next, "FastqFiltering" runs on each newly generated split FASTQ file and identifies read pairs that do not pass a set of user-defined parameters for base quality filtering. Default usage was designed to identify poor quality bases at or surrounding a SNV site, though the function includes parameters to easily adjust for filtering of all base pairs in paired read sequences or of only a single read for each pair. "BatchMutationCalling" is then run, which first identifies whether a read contains the expected sequence of the nested primer used during library construction. If so, then each read's paired cell barcode is matched to a provided whitelist, within a Hamming distance of two. All reads that pass these two criteria are then assessed for whether the read contains a specified wildtype or mutant sequence at a designated position. This process is performed for each read in each split and filtered FASTQ file via parallelized processing through a slurm workload manager, ultimately outputting a cell barcode by genotyped reads matrix for each FASTQ file processed. Finally, "MergeMutationCalling" takes all matrices generated from each split FASTQ file and merges them together, grouping by cell barcode and summarizing the counts of reads that were identified to be wildtype, mutant, or neither.

The summarized genotyping data must then be integrated with the chromatin accessibility information via shared cell barcodes. Further, the genotyping data must be corrected for background noise. These two steps are achieved via a single function, "AddGenotypingArchR" that is compatible with the ArchR scATAC-seq pipeline. This function separates genotyping data from barcodes that match true cells present in the ATAC dataset from barcodes that are deemed "non-cells" from empty droplets. The quantity of signal for each genotype is quantified in empty droplets and, using a percentile of that noise, is corrected from genotyping signals in true cells. Next, both the raw and noise corrected genotyping data is added to the ArchR project's metadata by matching shared cell barcodes. Finally, the "FilterGenotyping" function is used to implement a minimum number of reads that is required for high confidence genotyping calls.

### Antibody-oligo conjugation for intracellular protein detection.

Purified antibodies were covalently conjugated to oligonucleotides containing unique barcodes for use in ASAP-seq experiments, as previously described (Van Buggennum et al., Sci Rep 6, 22675, 2016). Briefly, custom oligonucleotides with a sequence designed to mimic that of TotalSeq-B (Biolegend) that also contain a 5' amino modifier are labelled with a trans-cyclooctene (TCO) polyethylene glycol (PEG, 4 units) N-hydroxysuccinimide (NHS) linker. Separately, purified antibodies (without any carrier proteins such as BSA or gelatin) against desired intracellular proteins are labelled with a methyltetrazine (mTz)-PEG4-NHS linker. Modified oligos and antibodies were then conjugated at 4°C overnight, after which TCO-PEG4-glycine was used to quench residual tetrazine reaction sites on the antibodies. Conjugates were then pooled if desired and remaining unbound oligonucleotides were removed with ammonium sulfate precipitation and PBS washes using 50kDa amicon filters (Millipore Sigma, #UFC505008).

### Single cell ASAP-seq with GoT-ChA.

Samples were processed in a similar fashion to that described previously for standard scATAC-seq, with a few key differences as noted by the original authors (Mimitou et al., Nature Biotechnology, 2021, 39, pages1246-1258)) and additional minor modifications for incorporation of GoT-ChA:
1. Prior to FACS, cells were additionally stained with a panel of TotalSeq-A antibodies against various cell surface markers (Biolegend). This staining was done concurrently with fluorescent antibody labelling and followed previously described methodology.
2. After sorting, instead of isolating nuclei, cells were fixed with methanol-free paraformaldehyde (final concentration 1%), quenched with glycine (final concentration 0.125 M), and permeabilized using a modified lysis buffer (10 mM Tris-HCL (pH 7.4), 10 mM NaCl, 3 mM MgCl₂, 0.1% Nonidet P40 Substitute, 1% BSA).
3. If intracellular protein expression measurements are desired, fixed and permeabilized whole cells were then incubated with an intracellular block solution (Biolegend #900002577 with 10% Human TruStain FxC - Biolegend #422302 and 10% True-Stain Monocyte Blocker - Biolegend # 426101) for 15 minutes on ice prior to incubation with homemade TotalSeq-B antibodies against a panel of various intracellular and intranuclear proteins (Biolegend) for an additional 30 minutes on ice. Cells are then washed twice with intracellular wash buffer (Biolegend #900002577) and once with 1X chilled nuclei buffer (10X Genomics #2000207).
4. During the GEM Generation and Barcoding reaction (Step 2.1), 1 µL of 22.5 µM GoT-ChA primer mix is added to the barcoding reaction mixture, just as was done for scATAC-seq. Additionally, 0.5 µL of 1 µM bridge oligonucleotides, corresponding to the variety of capture sequences present on the antibody-oligos used to stain for extracellular and intracellular antibodies, are added to the barcoding reaction mixture. The bridge oligonucleotide sequences are as follows: BOA (bridge oligo for TSA):
   TCGTCGGCAGCGTCAGATGTGTATAAGAGACA-GNNNNNNNNNVTTTTTTTTTTTTTTTTTTTTTTTTTTTTTT/3InvdT/ and BOB (bridge oligo for TSB): TCGTCGGCAGCGTCAGATGTGTATAAG AGACAGTTGCTAGGACCGGCCTTAAAGC/3InvdT/. These oligonucleotides serve as templates for antibody-oligos to obtain partial Read 1 Nextera, thus allowing subsequent interaction with the 10x gel bead oligonucleotide and acquisition of both the unique cell barcode and the P5 Illumina sequencing handle.
5. During GEM Incubation (Step 2.5), an additional thermocycler step of 5 minutes at 40°C was added at the beginning of the protocol (prior to the 5 minute incubation at 72°C).
6. During the Post GEM Incubation Cleanup - Dynabeads (step 3.2), resuspend beads in 43.5 µL of Elution Solution I to recover 43 µL, 3 µL of which is removed to a separate tube to use as input in the protein tag library construction PCR. The remaining 40 µL proceeded to SPRI clean up as directed in the standard protocol.
7. During Post GEM Incubation Cleanup - SPRIselect (step 3.2d), the supernatant was saved and subsequently cleaned at a 2.0X ratio of SPRI beads to reaction volume, to be combined with the 3 µL from Step 3.1o and used as input for the protein tag library construction PCR.
8. During the Post GEM Incubation Cleanup (Step 3.2), 45.5 µL of Elution Solution I is used to elute material from SPRIselect beads. 5 µL is used for GoT-ChA library construction, and the remaining 40 µL are used for ATAC library construction as indicated in the standard protocol. GoT-ChA library construction proceeded as described above.
9. To generate the protein tag library, TotalSeq-specific PCRs were set up using P5 and RPI-X primers for TSA antibodies, and P5 and D7-XX primers for TSB antibodies with the following program: 95 °C for 3 min; 14-16 cycles of 95 °C for 20 s, 60°C for 30 s and 72°C for 20 s; followed by 72°C for 5 min and ending with hold at 4°C. Examples of an RPI-X primer: (binds the TruSeq Small RNA Read 2 handle and adds a sample index and P7 Illumina sequencing handle: CAAGCAGAAGACGGCATACGAGATXXXXXXXXGTGACTGGAGTTCCTT GGCACCCGAGAATTCCA, "X" denotes user-defined sample index). Example of a D7-XX primer: (binds the TruSeq Read 2 handle and adds a sample index and P7 Illumina sequencing handle: CAAGCAGAAGACGGCATACGAGATXXXXXXXXGTGACTGGAGTTCAGA CGTGTGC, "X" denotes user-defined sample index).

Final libraries were quantified using a Qubit dsDNA HS Assay Kit (Thermo Fisher Scientific, #Q32854) and a High Sensitivity DNA chip (Agilent Technologies, #5067-4626) run on a Bioanalyzer 2100 system (Agilent Technologies) and sequenced on a NovaSeq 6000 at the Weill Cornell Medicine Genomics Resources Core Facility with the following parameters: paired-end 50 cycles; Read 1N 50 cycles, i7 Index 8 cycles, i5 Index 16 cycles, Read 2N 50 cycles. ATAC libraries were sequenced to a depth of 25,000 read pairs per cell, and both GoT-ChA and any protein tag libraries were sequenced to 5,000 read pairs per cell.

### Other targets that are tested

TP53 M133
   LM062_TP53_F4_M133_R1N 
TCGTCGGCAGCGTCAGATGTGTATAAGAGACAGCTACAGTACTCCCCTGC*C*C*
T
   LM063_TP53_R4_M133_IS2
      AGCAAGTGAGAAGCATCGTGTCACCATCGCTATCTGAGC*A*G*C
   LM064_TP53_N4_M133_ADT
      /5BiosG/CCTTGGCACCCGAGAATTCCATCACAACCTCCGTCATGTGC
NRAS
   LM-059_NRAS_Q61_F1_R1N 
TCGTCGGCAGCGTCAGATGTGTATAAGAGACAGTTGGTCTCTCATGGCAC*T*G*
T
   LM-060_NRAS_Q61_R1_IS2
      AGCAAGTGAGAAGCATCGTGTCGGGACAAACCAGATAGGCA*G*A*A
   LM-061_NRAS_Q61_N1_ADT
      /5BiosG/CCTTGGCACCCGAGAATTCCAAGCATTGCATTCCCTGTGGT
NOTCH1 
TCGTCGGCAGCGTCAGATGTGTATAAGAGACAGGAAACGAACCCTGCCTCCAG
CC
 
AGCAAGTGAGAAGCATCGTGTCAGTGCATCAACACGCTGGGCTC
CCTTGGCACCCGAGAATTCCACTCGAACCCGTGCCAGAACGAC
FOXO1

TCGTCGGCAGCGTCAGATGTGTATAAGAGACAGAGGTTGCTCATGAAGTCGGC
GC

AGCAAGTGAGAAGCATCGTGTCTCCCCCAAATCTCGGACCGTCC
CCTTGGCACCCGAGAATTCCAAGATCGACCCGGACTTCGAGCC

### Example 2: Somatic genotyping in high-throughput droplet scATAC-seq with GoT-ChA

To link genotypes to chromatin accessibility profiles at a single-cell resolution, we developed GoT-ChA by modifying the 10x Genomics single cell ATAC-seq (scATAC-seq) platform (**Figure 1a**). After nuclei isolation and bulk transposition, two GoT-ChA primers are added to the PCR reaction mixture prior to loading the microfluidics chip. These primers are designed to flank a specific region of interest, with one primer containing a partial Read 1 Nextera handle that is analogous to the capture sequence on the 10x Genomics scATAC-seq Gel Bead oligonucleotides. During in-droplet PCR, the GoT-ChA primers bind to and amplify the genomic region of interest, generating an amplicon that contains the partial Read 1 Nextera sequence, which then allows for further priming and amplification with the 10x scATAC-seq Gel Bead oligonucleotides (**Figure 1a**). In this manner, both tagmented gDNA fragments and GoT-ChA amplicons simultaneously obtain the unique cell barcode sequence and the P5 Illumina sequencing handle prior to breakage of the oil emulsion. Of note, while in-droplet amplification of tagmented genomic fragments is linear, in-droplet amplification of GoT-ChA fragments is exponential by design, resulting in an increased relative abundance of genotyping product. After the barcoded fragments are cleaned, 10% of the total sample is used for genotyping library construction, while the remainder is utilized for the scATAC-seq library. During genotyping library construction, target fragments are specifically amplified using a hemi-nested PCR strategy and a biotin-streptavidin pulldown prior to sample indexing. Both the scATAC and GoT-ChA final libraries can then be sequenced together, with chromatin accessibility and genotype information linked via shared cell barcodes. Of note, tagmentation of the targeted site is not required for genotype capture via GoT-ChA, as target amplification solely requires primer binding. To make GoT-ChA more easily implementable, we developed an R package (to be publicly available upon manuscript publication) that provides start-to-end processing of GoT-ChA data and its integration with the scATAC data.

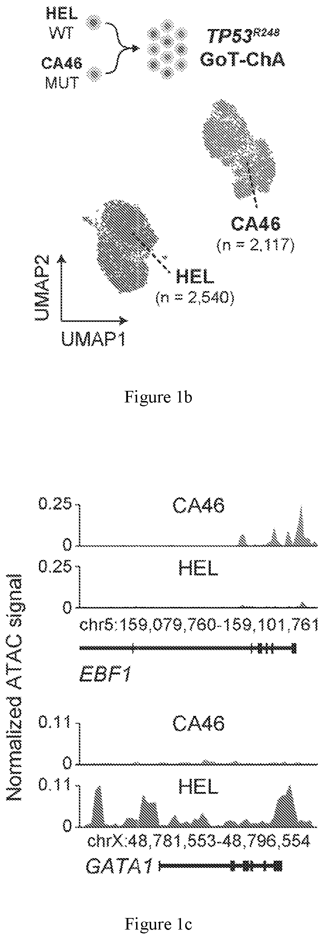

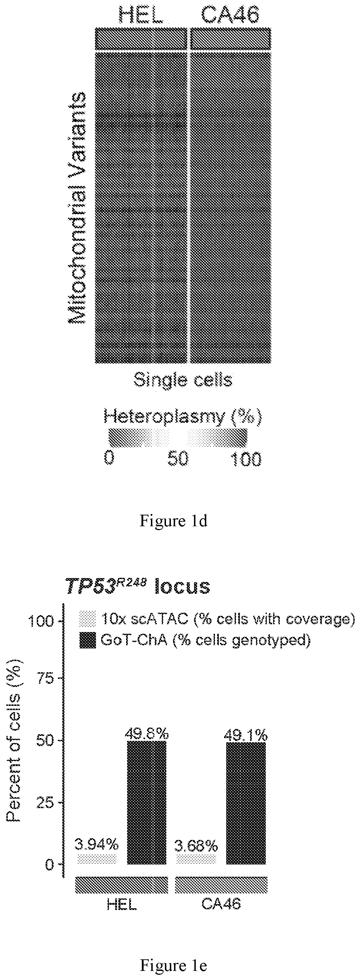

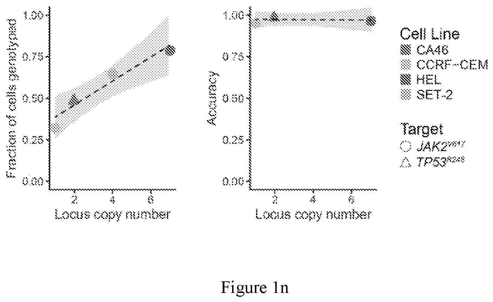

To test the ability of GoT-ChA to accurately capture genotype along with chromatin accessibility information we performed cell line mixing studies. Two human cell lines of discrete cell types (CA46, a Burkitt's lymphoma cell line, and HEL, an erythroleukemic cell line) and homozygous genotypes (**Figure 7a**) for the target site of interest, TP53-R248, were mixed at a 1:1 ratio and profiled with GoT-ChA. Using scATAC information alone, cells clustered into two distinct populations (**Figure 1b**) which were clearly identified as either cell line via chromatin accessibility signal at key marker genes (**Figure 1c**) as well as the presence of mutually exclusive mitochondrial variants (**Figure 1d**) and distinct copy number differences (**Figure 1g**) in a manner agnostic to genotyping. However, the unprocessed genotyping data illustrated a high degree of background noise resulting in intermediate mutant fractions despite known homozygosity of these cells. We reasoned that background noise likely arises from leakage of DNA molecules into the cell suspension media, similar to what is observed in other droplet-based methods, and therefore leveraged genotyping data from empty droplets to measure of experimental noise. By correcting for noise and implementing a threshold for minimum coverage of the mutation site (see materials and methods), we achieved genotyping of the TP53-R248 locus in 54.2% of cells with an accuracy of 96.9% (**Figure 1f**). Of note, while 82% of cells contained sequencing reads from the scATAC library within the TP53 gene, only 4% of cells had at least one read covering the locus of interest. Conversely, GoT-ChA resulted in high depth coverage of the mutation site, capturing genotyping information for 54% of cells (**Figure 1e**). We further tested GoT-ChA against a different genomic locus, the JAK2-V617 hotspot, with a separate mixing study. Consistent with successful GoT-ChA implementation at various sites in the genome, we observed similar results regarding accuracy and recovery. While scATAC information alone provided coverage of the JAK2 gene in most cells, only 1% of cells had scATAC reads covering the targeted locus. Implementation of the GoT-ChA pipeline achieved genotyping of the JAK2-V617 locus in 59.9% of cells with an accuracy of 99.7% (**Figure 1f**). Of note, while the number of cells with genotyping information was positively correlated with allele copy number, the accuracy remained above 95% independently of target copy number (**Figure 1n**).

Thus, GoT-ChA allows for high-throughput simultaneous capture of genotypes and chromatin accessibility profiles in single cells, with high accuracy and cell recovery independent of expression level and genomic localization of the targeted region.

### Example 3: Droplet-based high-throughput simultaneous capture of genotypes and chromatin accessibility in single cells

To integrate genotyping into scATAC-seq, we modified the broadly utilized 10x Genomics platform by adding two custom primers (GoT-ChA primers) to the cell barcoding PCR reaction mixture prior to loading the microfluidics chip for droplet generation (Fig. 1a). These primers are designed to flank a specific region of interest, with one primer containing a Read 1 Nextera (R1N) handle (Figs. 6a-6b). During in-droplet PCR, the GoT-ChA primers bind to and amplify the genomic region of interest, generating an amplicon that is complementary to the 10x scATAC-seq Gel Bead oligonucleotides, allowing for cell barcoding and further amplification. In this manner, both tagmented gDNA fragments and GoT-ChA amplicons simultaneously obtain the unique cell barcode sequence and the P5 Illumina sequencing handle prior to breakage of the single-cell emulsion (Fig. 1a). Of note, tagmentation of the targeted site is not required for genotype capture, as target amplification with GoT-ChA primers results in amplicons already containing the R1N capture sequence. Moreover, while in-droplet amplification of tagmented genomic fragments is linear, GoT-ChA amplicons are exponentially amplified by design, resulting in an increased relative abundance of genotyping amplicons. Then, 10% of the total product is used for genotyping library construction, while the remainder is utilized for the scATAC-seq library. During genotyping library construction, GoT-ChA fragments are specifically amplified using a hemi-nested PCR strategy and a biotinstreptavidin pulldown prior to sample indexing (Fig. 6c). Both the scATAC-seq and GoTChA libraries can then be sequenced together, with chromatin accessibility and genotype information linked via shared cell barcodes. To encourage the broad implementation of GoT-ChA, we developed an R package (Gotcha R package) that provides start-to-end processing of GoT-ChA data and its integration with the corresponding scATAC-seq profiles.

To test the ability of GoT-ChA to accurately capture genotype along with chromatin accessibility information, we performed a cell line mixing study. Two human cell lines of discrete cell types (CA46, a B lymphocyte cell line; and HEL, an erythroblast cell line) and differing genotypes for the TP53R248 locus (CA46, TP53R248Q homozygous mutant; HEL, TP53R248 homozygous wildtype; Fig. 7a) were mixed at a 1:1 ratio and profiled with GoT-ChA (Fig. 1b, upper panel). Using chromatin accessibility information alone, cells clustered into two distinct populations (Fig. 1b, bottom panel). The two clusters were readily annotated as the expected mixed cell lines via chromatin accessibility signals at key marker genes (Fig. 1c, Fig. 7b), as well as mutually exclusive mitochondrial variants (Fig. 1d). Fragment size distribution, total number of fragments per cell, and enrichment of fragments mapping to transcriptional start sites (TSS) all reflect high quality scATAC-seq data, unaffected by the inclusion of GoT-ChA primers during droplet generation (Figs. 7c-7e).

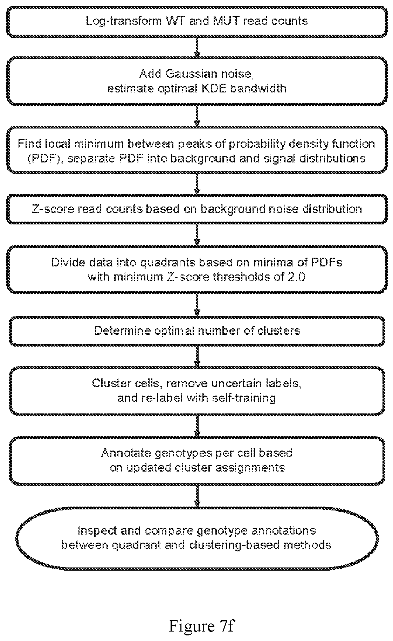

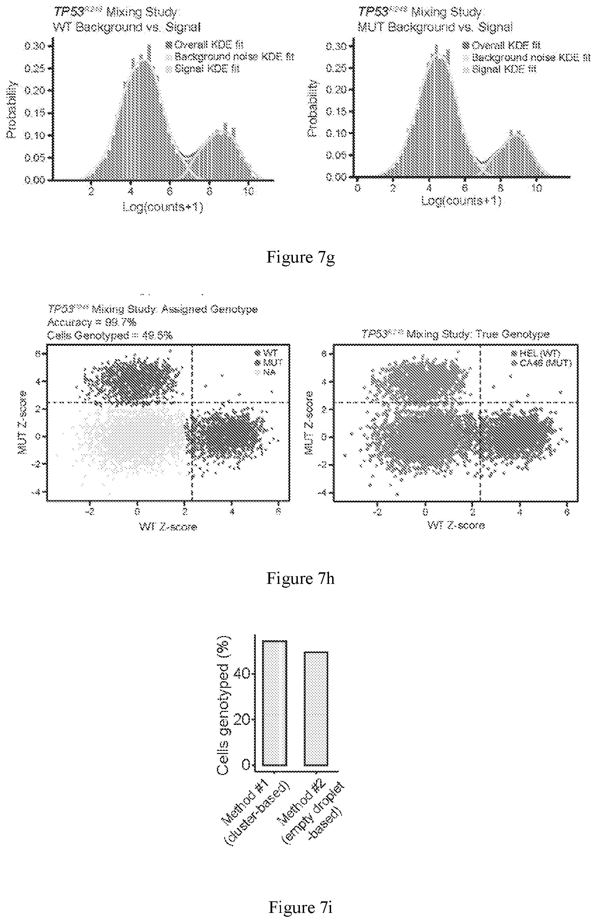

Analysis of the matching unprocessed genotyping data showed the presence of two distinct modes in the distribution of genotyping reads per cell. We reasoned that these distributions reflect cells for which genotyping was successfully captured versus cells displaying background noise from the genotyping library. To address this aspect, we developed a computational framework (Fig. 7f) using kernel density estimation (KDE) to define the boundaries between background noise and genotyping signal (Fig. 7g), followed by clustering and genotype assignment based on the z-scores of genotyping read counts (Fig. 7h). This approach was orthogonally validated using an alternative framework leveraging recent work46 in single-cell genomics that proposed estimating the background noise with empty droplets (Fig. 7i-j; see materials and methods; both approaches for noise correction and genotype calling are included in the Gotcha R package). While less than 4% of cells had at least one scATAC-seq read covering the TP53R248 locus of interest in either cell line, GoT-ChA resulted in high confidence genotyping for 49.8% and 49.1% of HEL and CA46 cells, respectively (Fig. 1e). By correcting for background noise, we achieved genotyping of the TP53R248 locus in 49.5% of all cells with an accuracy of 99.7% (Fig. 1f). As an additional orthogonal validation of our genotyping, we compared wildtype and mutant cell CNV scores inferred from the chromatin accessibility profiles (see materials and methods) across chromosome 9, for which the TP53R248 wildtype HEL cell line carries an amplification. Consistent with our observed genotyping, wildtype cells as defined by GoT-ChA genotyping exhibited an increased CNV score relative to mutant cells, further confirming successful single-cell genotyping (Fig. 1g).

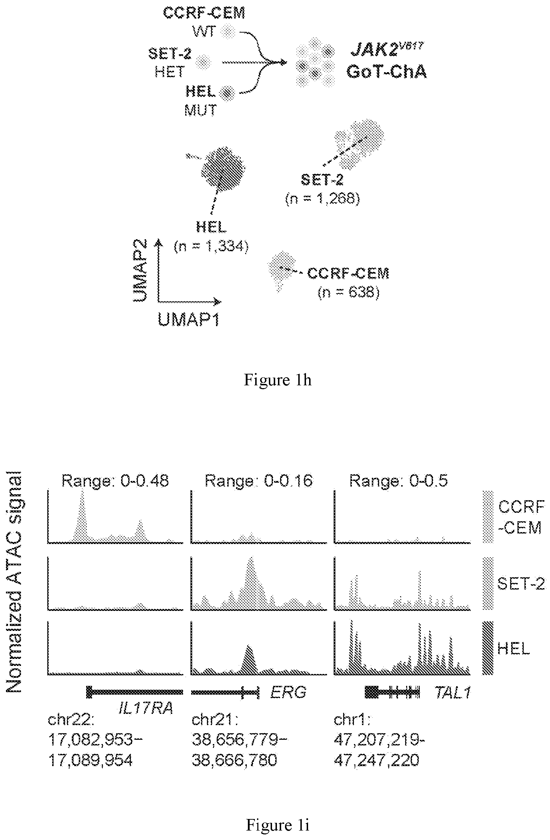

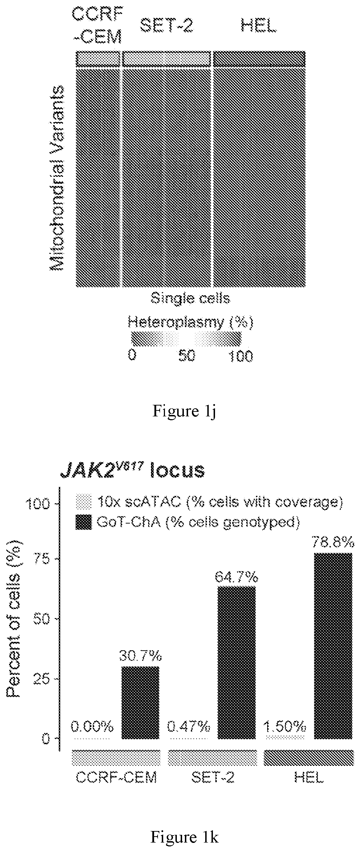

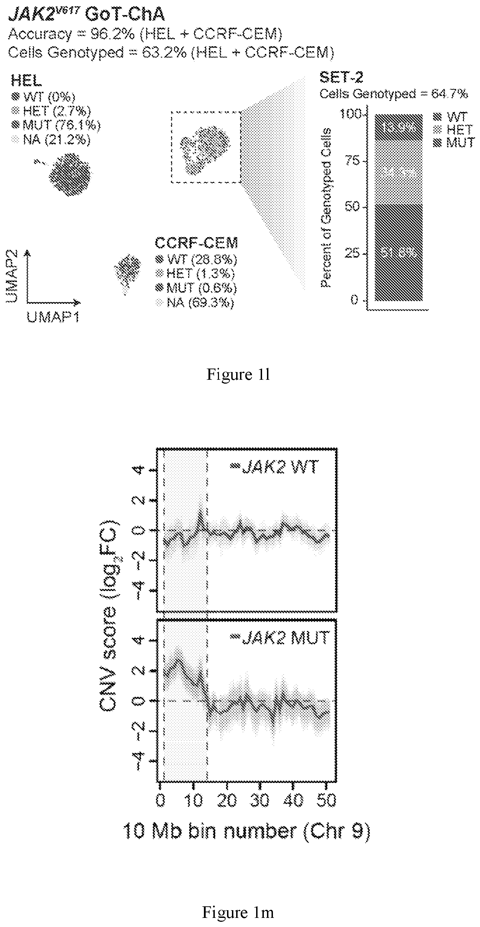

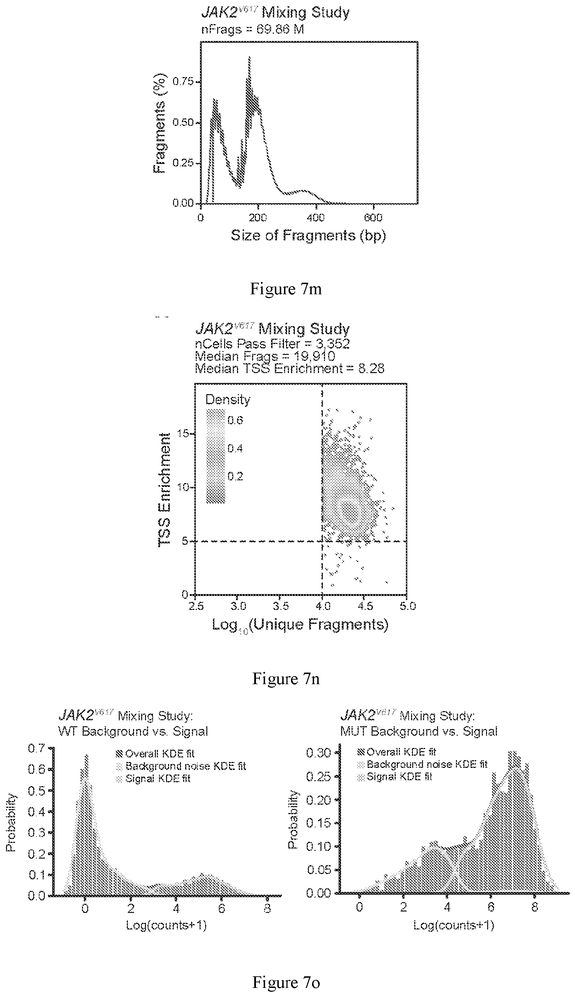

We further tested GoT-ChA targeting a different genomic locus, the JAK2V617 hotspot, with a separate mixing study to directly address heterozygous genotyping. Three human cell lines (HEL, an erythroblast cell line; SET-2, a megakaryoblast cell line; CCRF-CEM, a T lymphoblast cell line) with discrete JAK2V617 genotypes (HEL, JAK2^{V617F} homozygous mutant; SET-2, JAK2^{V617F} heterozygous; CCRF-CEM, JAK2V617 homozygous wildtype; Fig. 7k) were mixed at a 1:1:1 ratio and profiled with GoT-ChA. As with the homozygous mixing study targeting TP53R248 shown above, clustering agnostic to genotyping utilizing only the scATAC-seq data resulted in discrete populations of cells (Fig. 1h) that were annotated via differential chromatin accessibility signals at key marker genes (Fig. 1i; Fig. 7l) and unique mitochondrial variants (Fig. 1j), while again confirming unaffected quality of the scATAC-seq data (Fig. 7m, n). The genotyping data was processed as described above, using KDE applied to log-read distributions to define the boundaries between background noise and genotyping signal for wildtype and mutant reads (Fig. 7o), followed by clustering on zscore read counts (Fig. 7p). While less than 2% of cells had at least one scATAC-seq read covering the JAK2V617 locus, genotyping by GoT-ChA captured 30.7% of CCRF-CEM, 64.7% of SET-2 and 78.8% of HEL cells (Fig. 1k). In the two homozygous cell lines (wildtype CCRF-CEM and mutant HEL), genotyping of the JAK2V617 locus was achieved in 63.2% of cells with an accuracy of 96.2% (Fig. 1l, left panel). In the SET-2 heterozygous cell line, 64.7% of cells were successfully genotyped; 34.3% of genotyped cells were correctly identified as heterozygous, confirming bi-allelic capture, with the remaining genotyped cells split between homozygous wildtype and mutant assignments, suggesting incomplete capture of the heterozygous genotype (Fig. 1l, right panel, note that SET-2 cells have a 3:1 ratio of mutated: wildtype alleles (Fig. 7k), likely underlying the higher fraction of mutated versus wildtype calls in non-heterozygous genotype classification). CNV scores for homozygous wildtype and mutant cells along the chromosome 9 amplification present in the JAK2^{V617F} homozygous mutant HEL cell line orthogonally validated successful genotyping of the JAK2V617 locus (Fig. 1m). We note that variation in genotyping efficiency across cell lines is likely related to the copy number variation for the JAK2 locus. Indeed, the proportion of genotyped cells positively correlated with the copy number of the JAK2 locus (Fig. 1n, left panel, Pearson correlation; P = 0.011; R2 = 0.91; F-test), suggesting that additional copies of the target locus improve the genotype capture rate. Importantly, genotyping accuracy remained consistent regardless of target copy number differences (Fig. 1n, right panel).

Altogether, these data demonstrate that GoTChA allows for high-throughput simultaneous capture of genotypes and chromatin accessibility profiles in single cells, with high accuracy and cell recovery independent of expression level and genomic localization of the targeted region.

### Example 4: GoT-ChA of primary human JAK2^{V617F} myelofibrosis reveals cell type-specific mutant cell predominance in erythroid and megakaryocytic progenitors

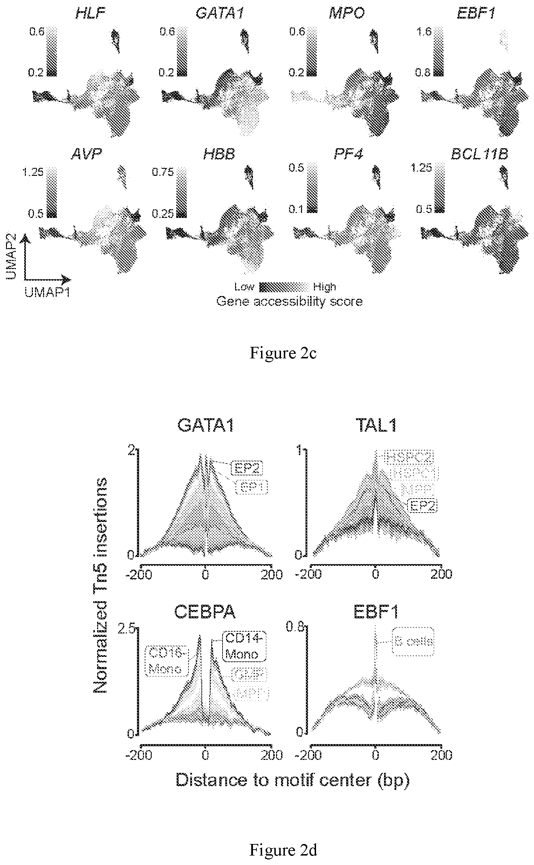

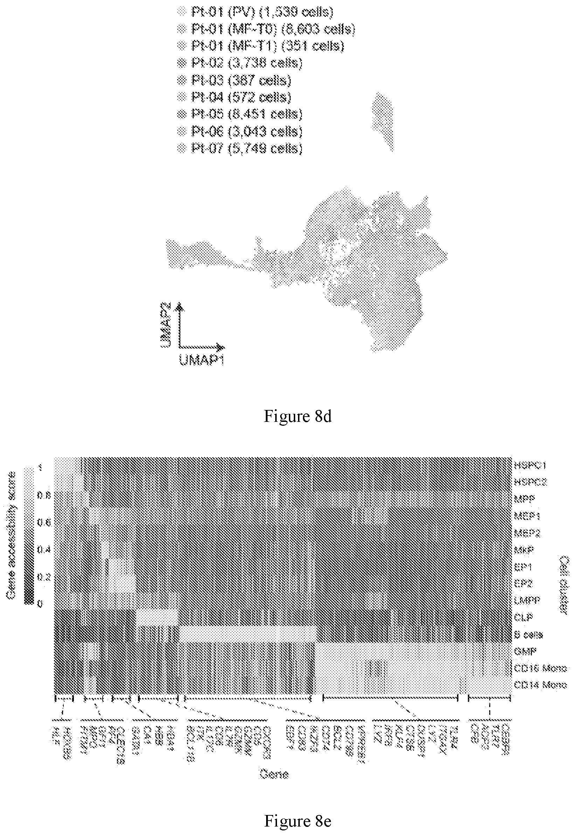

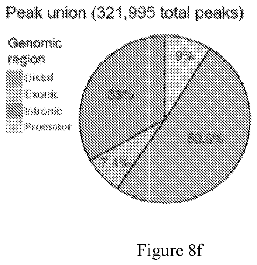

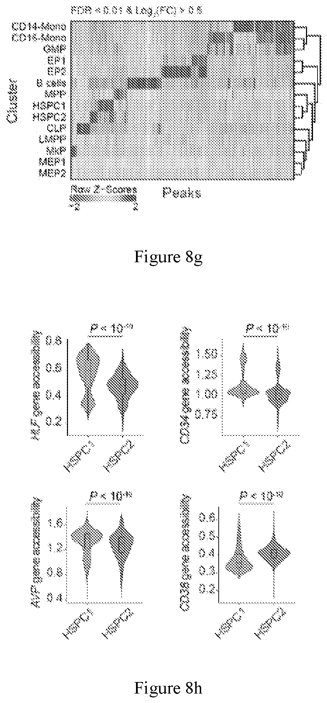

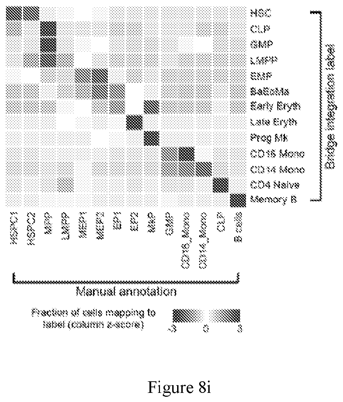

The JAK2^{V617F} mutation has a central role in the pathogenesis of myeloproliferative neoplasms. We sought to explore how this mutation disrupts the regulatory chromatin landscape that determines cellfate decisions of HSPCs. To address this question, we applied GoT-ChA to CD34+ sorted progenitor cells from seven patients with JAK2^{V617F}-mutated MF with no additional mutations, who had either not been treated with JAK inhibition or were being treated with ruxolitinib, a JAK1/2 inhibitor, or fedratinib, a JAK2-specific inhibitor at the time of sample collection. The quality of the scATAC-seq data was not affected by removal of a small portion of the sample for GoT-ChA genotyping library construction (Fig. 8a-c). We then performed cell clustering in a manner agnostic to genotyping information, based solely on chromatin accessibility (Fig. 2a). Reciprocal latent semantic indexing applied to the binarized genomic bin by cell matrix was used for sample integration to correct for patient-specific batch effects (Fig. 2b; Fig. 8d; see materials and methods). Individual cluster identities were assigned using gene accessibility scores of key marker genes for hematopoietic lineages (Fig. 2c; Fig. 8e) and confirmed via transcription factor footprinting (Fig. 2d) and peak calling (Fig. 8f), followed by differential enrichment of marker peaks (Fig. 8g). We identified the expected progenitor subtypes, along with a population of mature monocytic cells characterized by CD14 expression and lack of CD34 expression, often observed in CD34+ sorting of human bone marrow. The presence of two distinct HSPC subclusters, early (HSPC1) and late (HSPC2), was validated via gene accessibility scores of multiple markers of hematopoietic stem cells (Fig. 8h). Cluster assignments were further supported via orthogonal annotation utilizing cell mapping through a novel multiomic bridge integration approach (Fig. 8i; see materials and methods).

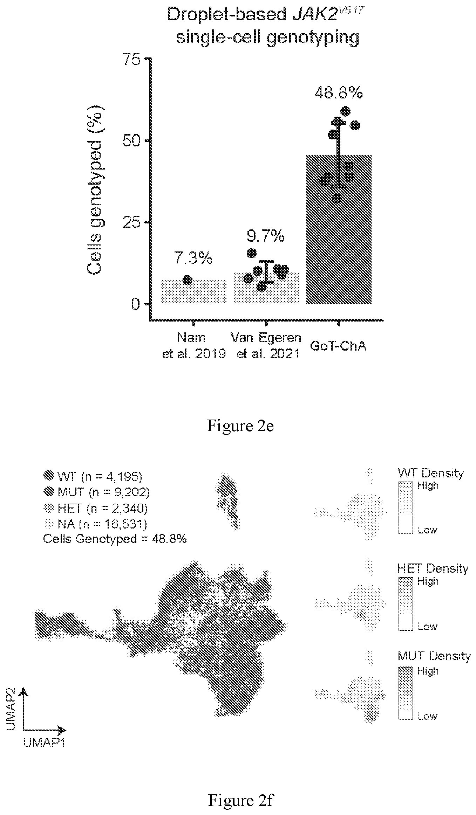

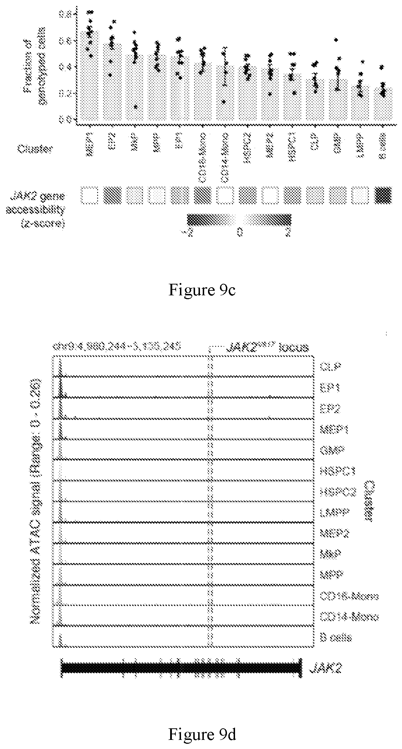

Analysis of the GoT-ChA genotyping data (Fig. 9a) resulted in JAK2V617 genotyping information for 15,737 out of 32,268 total cells (48.8% ± 9.64%; mean ± standard deviation across samples), compared to 7-10% of cells in previously reported droplet-based scRNA-seq cDNA-based genotyping (Fig. 2e), demonstrating the increased ability of GoTChA to genotype loci with relatively low expression in single cells. Pseudo-bulked GoT-ChA variant allele frequencies (VAFs) were positively correlated with the reported VAFs from clinical targeted panels (P = 0.036; Rho = 0.84; Spearman correlation; Fig. 9b). We noted variability in genotyping efficiency across cell types (range: 23.7% to 66.6%), which may be related to locus accessibility (Fig. 9c). Nonetheless, by design, GoT-ChA is not strictly dependent on accessibility, allowing genotyping across cell types despite minimal locus accessibility (Fig. 9d).

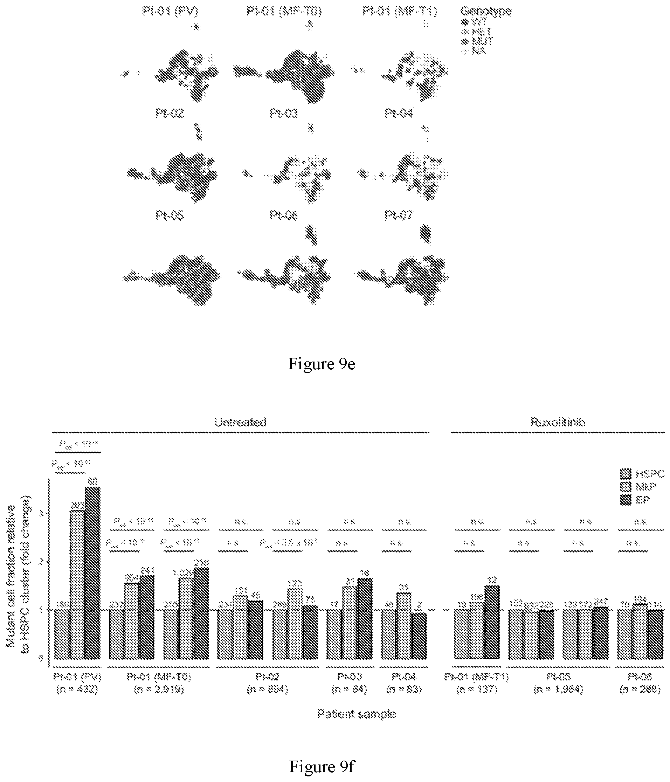

Projection of genotypes onto the cell differentiation map demonstrated intermingling of JAK2 wildtype and mutant cells throughout HSPCs and myeloid progenitor clusters, while the common lymphoid progenitors (CLPs) and B cell clusters were mainly comprised of wildtype cells (Fig. 2f, left panel; Fig. 9e), consistent with previous studies of hematopoietic colonies/subsets from MPN patients. The intermingling of mutated and wildtype progenitors reinforced the inability of chromatin accessibility profiles alone to distinguish wildtype from mutant cells, highlighting the need for single-cell multiomics for genotype-epigenome inferences.

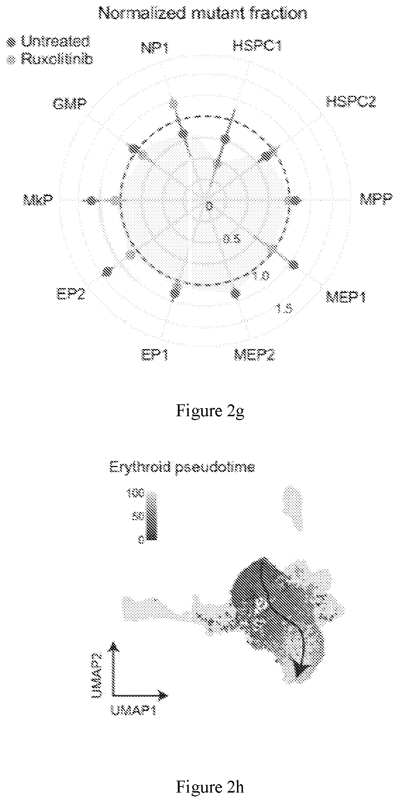

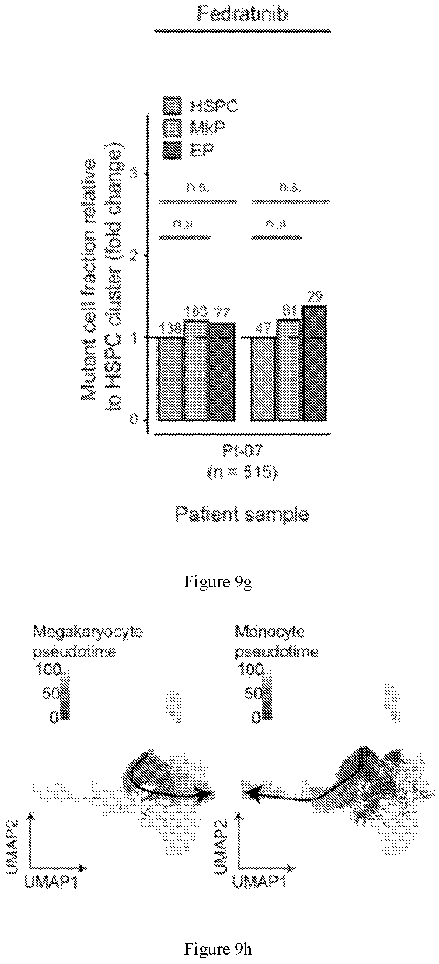

Nonetheless, projection of genotype densities onto the differentiation map suggested an uneven distribution of mutant cells across cell subtypes (Fig. 2f, right panel). Indeed, while HSPCs and committed myeloid progenitor clusters were composed of an admixture of wildtype and JAK2^{V617F} mutant cells, their frequencies varied greatly. The normalized mutant fraction was increased in the late erythroid progenitor cluster (EP2), as well as in the megakaryocytic progenitor (MkP) cluster (Fig. 2g; Fig. 9f). In contrast, patients treated with the JAK1/JAK2 inhibitor ruxolitinib displayed a more homogeneous distribution of mutant cells across cell types (Fig. 2g; see Fig. 9f for per sample analysis), suggesting that JAK inhibition alters the relative contribution of JAK2^{V617F} to these cellular lineages, but does not eliminate the mutated clone. Treatment with fedratinib, a JAK2-specific inhibitor, showed a similar distribution to ruxolitinib across cell types (Fig. 9g). Consistently, pseudotemporal ordering of cells along the erythroid differentiation trajectory (Fig. 2h) showed a steady increase in the fraction of mutant cells in untreated patients, while ruxolitinib treatment resulted in a more uniform distribution of mutant cells along erythroid differentiation (Fig. 2i). We observed a similar pattern of mutant cell fraction increase with MkP differentiation, and to a lesser extent in the monocyte differentiation trajectory (Fig. 9h-i). Thus, high resolution mapping of the JAK2^{V617F} mutation across human hematopoietic differentiation demonstrated a progenitor-specific mutant cell predominance in myelofibrosis that is eliminated by therapeutic JAK2 inhibition.

### Example 5: JAK2V^{617F} HSPCs show a cell-intrinsic proinflammatory phenotype and myeloid/erythroid epigenetic priming.

Inflammatory disruption of the bone marrow microenvironment has been extensively documented in myelofibrosis. Indeed, secretion of inflammatory cytokines is a central feature of MPN pathophysiology and has been shown to provide a supportive niche for the expansion of mutant clones in various disease states, including MPNs and clonal hematopoiesis. However, defining how wildtype and JAK2^{V617F} mutant early progenitors differ regarding cell-intrinsic epigenetic profiles in human myelofibrosis remains unknown due to the inability to directly compare mutated and wildtype cells within primary human samples.

To delineate the effects of JAK2^{V617F} on chromatin accessibility, we first compared gene accessibility scores (as the number of cells profiled varied between samples, linear mixture model (LMM) was used with patient sample identity explicitly modeled as random effect to account for inter-patient variability, followed by likelihood ratio test; see materials and methods) between wildtype and JAK2^{V617F}-mutated cells within the HSPC1 cluster (Fig. 3a) as a proxy for gene expression. These data revealed a cellintrinsic proinflammatory phenotype in JAK2^{V617F} mutant HSPCs, with increased gene accessibility of critical inflammatory genes (Fig. 3a-b). Proinflammatory genes more accessible in JAK2^{V617F} HSPC1 cells included members of the tumor necrosis factor (TNF) family, such as the receptor TNFRS9 (CD137) and its ligand TNFS9, as well as the cytokine CD70, which acts as ligand of the TNFRSF27 (CD27) receptor. This suggests an increase in levels of both ligands and their corresponding receptors in mutant HSPCs, which could result in increased downstream activation of NF-kB signaling64. In addition, both colony stimulating factors 1 and 2 (CSF1 and CSF2) show increased accessibility in JAK2^{V617F} HSPC1 cells. CSF1 can promote differentiation of HSPCs towards the myeloid lineage and CSF2 has been shown to be required for differentiation of precursor cells towards the myeloid/erythroid fate. We also observed increased accessibility of the CCL2 gene, a key cytokine mediating monocyte recruitment to inflammatory sites, which has been reported to be increased in MF.

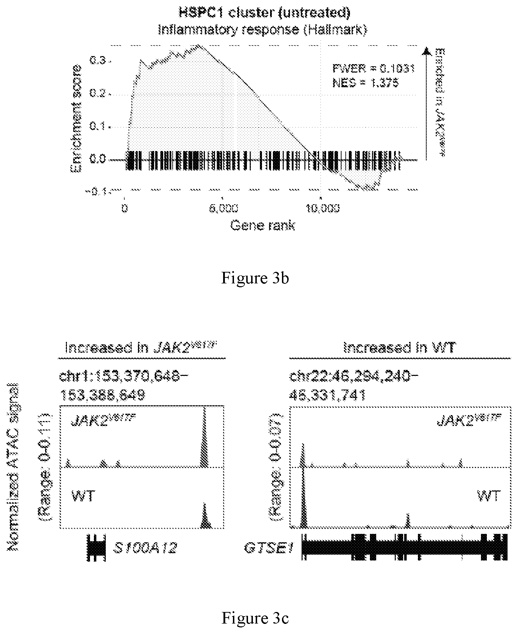

Consistently, gene pathway analysis revealed an enrichment in the inflammatory response pathway in mutant HSPC1 cells (Family-wise error rate [FWER] = 0.1031; normalized enrichment score [NES] = 1.375; Hallmark pathway M5932; Fig. 3b). In addition to the genes involved in the inflammatory pathway, we observed increased gene accessibility of S100A12 (Fig. 3c, left panel, decreased accessibility of GTSE1 gene is shown for comparison) encoding a protein involved in pro-inflammatory cytokine upregulation, including TNF through activation of NF-kB signaling. Notably, the proinflammatory signature observed in mutant cells was lost upon ruxolitinib treatment (Fig. 10a-b), consistent with ruxolitinib resulting in lowered circulating cytokine levels in patients.

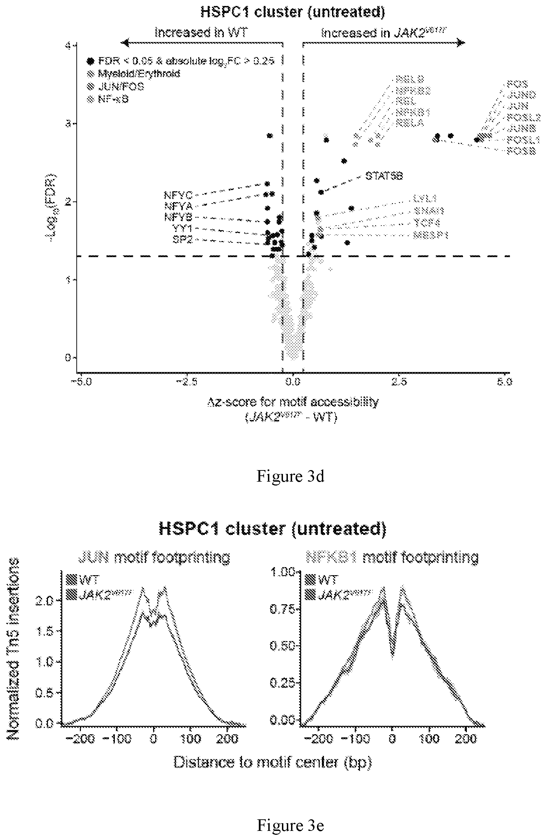

To further explore the regulatory underpinning of inflammatory phenotypes in HSPCs, we leveraged chromatin accessibility to infer transcription factor activity based on the accessibility of their DNA binding motifs (see materials and methods). Comparing wildtype and JAK2^{V617F}-mutated cells within the HSPC1 cluster, we uncovered a subset of transcription factors that show increased motif accessibility (false discovery rate [FDR] < 0.05 and Δz-score > 0.25) in early mutant HSPCs (Fig. 3d). Transcription factors involved in NF-kB signaling (NFKB 1, NFKB2, REL, RELA, and RELB) and in the AP-1 complex (JUN, JUNB, JUND, FOS, FOSB, FOSL1, FOSL2) showed increased accessibility in mutant cells, suggesting that the JAK2^{V617F} mutation already primes early HSPCs towards a pro-inflammatory cellular state. These findings are consistent with recent studies that have identified NF-kB and STAT3 as essential co-regulatory drivers of inflammation in MPN murine models, resulting in aberrant cytokine signaling from both mutant and non-mutant cells. Additionally, NF-kB signaling has been implicated in the regulation of AP-1 transcription factors, and along with STAT3, act as important mediators of an inflammatory regulatory network driving complex transcriptional programs in a variety of human cancers. Transcription factor footprinting within the HSPC1 cluster showed increased accessibility surrounding JUN and NFKB1 motifs in JAK2^{V617F} mutant cells (Fig. 3e).

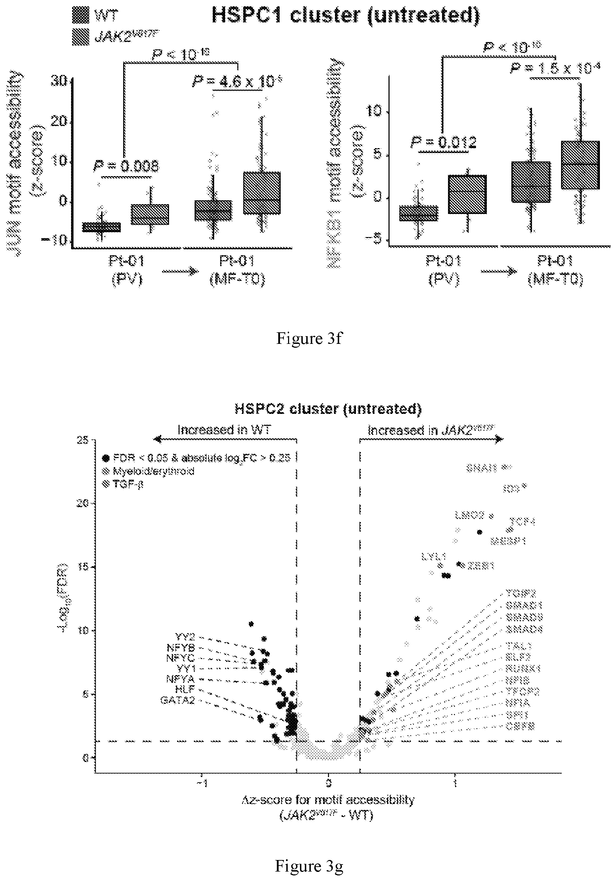

By leveraging longitudinal sampling obtained from an untreated patient with PV that later progressed to MF (Pt-01), we explored whether the pro-inflammatory phenotype as measured by JUN and NFKB1 motif accessibility preceded the increase in bone marrow fibrosis observed in MF. Indeed, we observed that both JUN and NFKB1 motif accessibilities were increased in JAK2^{V617F} mutant early HSPCs already at the PV stage (Fig. 3f). Importantly, JUN and NFKB1 transcription factor motif accessibility was increased in MF relative to PV (Fig. 3f), consistent with an increasingly pro-inflammatory bone marrow environment in the progression from PV to MF. Thus, while proinflammatory phenotypes have been linked to extrinsic effects, and highlighted to affect transcriptional profiles in committed erythroid progenitors, our high-resolution mapping of chromatin accessibility in early mutant human HSPCs showed that JAK2^{V617F} promotes cell-intrinsic proinflammatory gene accessibility and transcription factor activity, well before commitment towards the erythroid fate.

Myeloid/erythroid differentiation (LYL1, SNAI1, TCF4, and MESP1) also exhibited increased accessibility of their DNA-binding motifs in mutant cells (Fig. 3d), suggesting that epigenetic priming towards a myeloid cell fate is present at early stages of JAK2^{V617F}mutated hematopoiesis. We also observed modestly decreased motif accessibility for a group of transcription factors involved in stem cell quiescence (NFYA/B/C, YY1, and SP2) in mutant cells (Fig. 3d). The decrease in potential activity of these transcription factors may underlie the increased hematopoietic output of mutant cells relative to their wildtype counterparts, as suggested by increased mutant fractions in more differentiated progenitor clusters in the myeloid lineage.

To uncover further changes in transcription factor activity within later HSPCs, we performed differential transcription factor motif accessibility between wildtype and mutant cells within the HSPC2 cluster (Fig. 3g). Consistent with our observations in the earlier HSPC1 cluster, differential transcription factor motif accessibility highlighted increased activity of transcription factors associated with myeloid/erythroid differentiation (e.g., ID3, MESP1, SNAI1, LMO2, TCF4, ZEB1, and LYL1) in mutant cells, suggesting that the erythroid bias is strengthened in later HSPCs, likely underlying erythroid priming6. Notably, LMO2, a key factor in erythroid differentiation, has been postulated as a direct non-signaling target of JAK2 through nuclear translocation and histone modulation, thus serving as a potential direct link between the mutated genotype and erythroid-biased HSPC priming. A second group of transcription factors also associated with myeloid and erythroid differentiation showed a more modest yet significant increase in JAK2^{V617F} mutant HSPCs (e.g. TAL1, RUNX1, TFCP2, NFIA, NFIB, and CBFB; Fig. 3g). Of note, RUNX1 is known to drive the initial commitment towards myeloid differentiation in hematopoiesis and may underlie the myeloid priming observed in myelofibrosis.

Consistent with mutant cells in the HSPC1 cluster, HSPC2 mutant cells also exhibited a decrease in the accessibility of transcription factor motifs implicated in stem cell quiescence (NFYA/B/C, YY1, YY2, GATA2 and HLF; Fig. 3g). Additionally, HSPC2 mutant cells showed an increase in accessibility of transcription factor motifs within the TGF-b signaling pathway (TGIF2, SMAD1, SMAD4, and SMAD9), which is thought to play an important role in the development of marrow fibrosis. Of note, ruxolitinib treatment brogated the cell-intrinsic differences observed in transcription factor motif accessibility between wildtype and JAK2^{V617F}-mutated HSPCs (Fig. 10c-d), demonstrating that the changes observed in untreated patients are mediated by the JAK2^{V617F} constitutive activation.

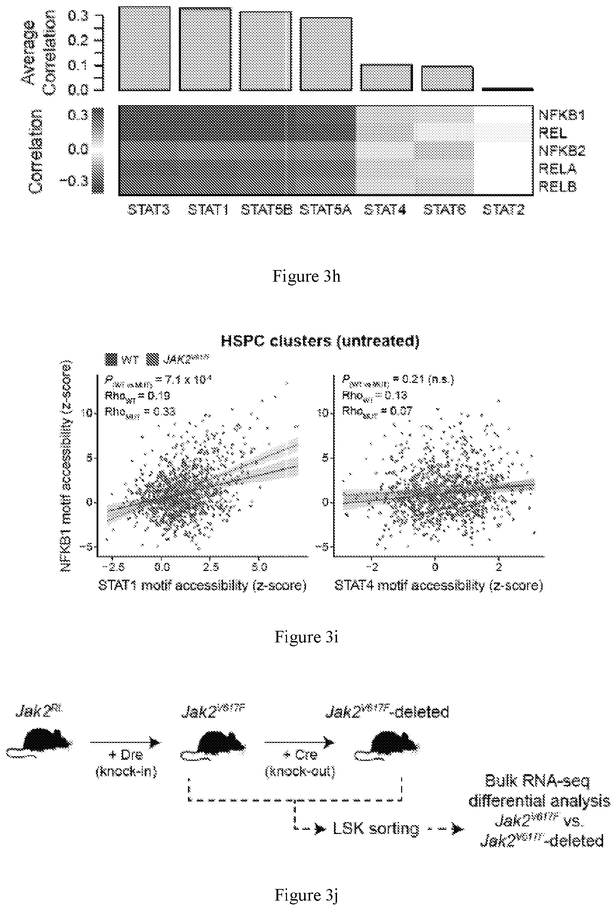

To link the activation of NF-kB signaling to canonical JAK2 downstream targets, we correlated NFkB- related transcription factor motif accessibility with members of the STAT family transcription factor motifs in HSPCs. We found increased correlation of STAT1, STAT3, STAT5A, and STAT5B motif accessibility with NF-kB factor motif accessibility, consistent with JAK2 activation of canonical STAT targets (Fig. 3h; Fig. 10e). In contrast, non-canonical JAK2 targets STAT2, STAT4, and STAT6 showed reduced correlation values with NFKB1 compared to canonical JAK2 target STATs (Fig. 3h; Fig. 10e), suggesting that JAK2-mediated activation underlies the increased activity of the NF-kB-associated transcription factors. Indeed, although both wildtype and JAK2^{V617F} mutant HSPCs show a positive correlation between STAT1 and NFKB1 motif accessibility, mutant cells display a higher correlation coefficient (Fig. 3i, left panel; P = 7.1 x 10⁻⁴; Spearman correlation), while no differences were observed in the correlation between STAT4 and NFKB1 (Fig. 3i, right panel; P = 0.21; Spearman correlation). Thus, activation of STAT1, STAT3, STAT5A and STAT5B downstream of JAK2^{V617F} might drive the observed NF-kB signal.

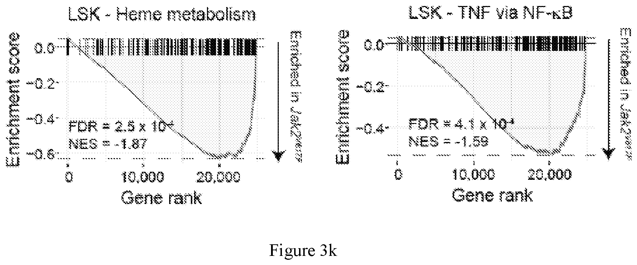

To validate the inflammatory and myeloid/erythroid signatures observed in mutant HSPCs, we leveraged bulk RNA-seq data of Lin-, Sca-1+, c-Kit+ (LSK) progenitor cells from a novel Dre-rox, Crelox dual recombinase Jak2^{V617F} mouse model that allows for sequential knock-in followed by knock-out of the mutated allele (Jak2Rox/Lox/Jak2RL; Fig. 3j). Consistent with our findings in human patient samples showing erythroid priming and a cell-intrinsic pro-inflammatory signals, gene set enrichment analysis revealed an enrichment of the heme metabolism gene set which includes key erythroid transcription factors (FDR = 2.5 x 10⁻⁴; NES = -1.87), and the tumor necrosis factor (TNF) signaling via NF-kB gene set (FDR = 4.1 x 10⁻⁴; NES = -1.59) in Jak2^{V617F} compared to Jak2^{V617F}-deleted murine LSK cells (Fig. 3k), supporting the causality of the JAK2^{V617F} mutation in driving the observed phenotypes.

Collectively, gene accessibility and transcription factor motif accessibility comparisons revealed a subset of early HSPCs displaying cell-intrinsic proinflammatory phenotypes, as well as erythroid lineage priming in JAK2^{V617F} mutant versus wildtype human HSPCs.

### Example 6: JAK2^{V617F} results in cell type-specific epigenetic changes in committed erythroid and megakaryocytic progenitors.

We next sought to define the epigenetic changes in the erythroid and megakaryocytic progenitors, the cell types undergoing significant clonal expansion of JAK2^{V617F}-mutated cells (Fig. 2g). When assessing transcription factor motif accessibility changes in the EP1 cluster, we found increased STAT5A and STAT5B transcription factor motifs (Fig. 4a), with increased accessibility of STAT1, STAT3, STAT5A, and STAT5B motifs in the late erythroid progenitor (EP2) cluster (Fig. 11a). These results highlight the cell type-specific degree of activation of canonical downstream targets of JAK2, varying between HSPCs and between early and late EPs, which might drive the increased fitness of mutated erythroid progenitors. In addition, we observed increased accessibility of multiple myeloid/erythroid transcription factor binding motifs (TCF4, ID3, MESP1, TFCP2, POU2F1, THRB, THRA, ZEB1, LMO2, and DDIT3; Fig. 4a), consistent with our previous observations in early HSPCs, pointing to a JAK2^{V617F} effect that remains consistent across erythroid differentiation. In contrast, PU.1 (SPI1), a critical regulator of cell fate decisions between myeloid and lymphoid lineages, exhibited decreased motif accessibility in mutant cells. Recent work has shown that downregulation of PU.1 induces gene expression changes that promote myeloid-biased stem cells and erythroid differentiation. Thus, increased erythroid-associated and STAT transcription factor activity may result in increased fitness underlying the expansion of mutated erythroid progenitors.

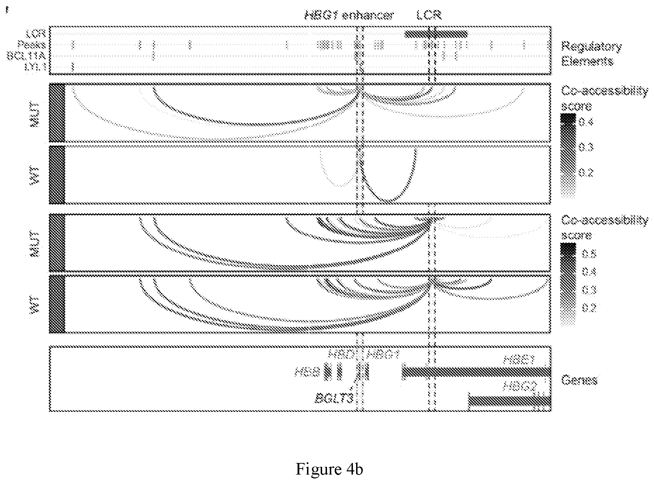

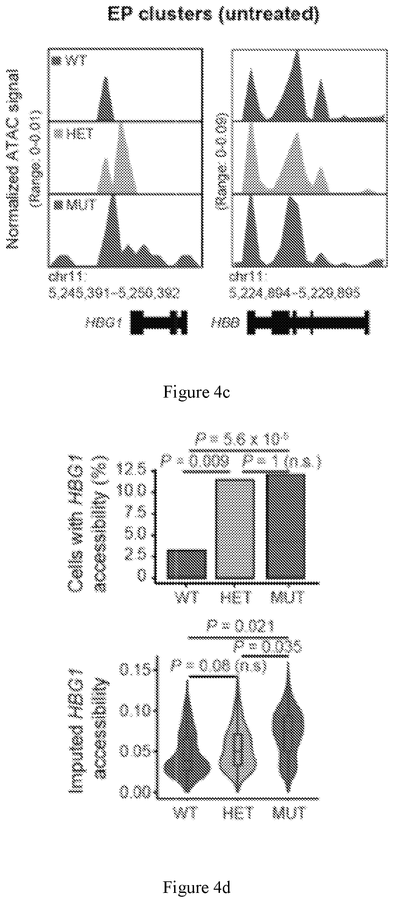

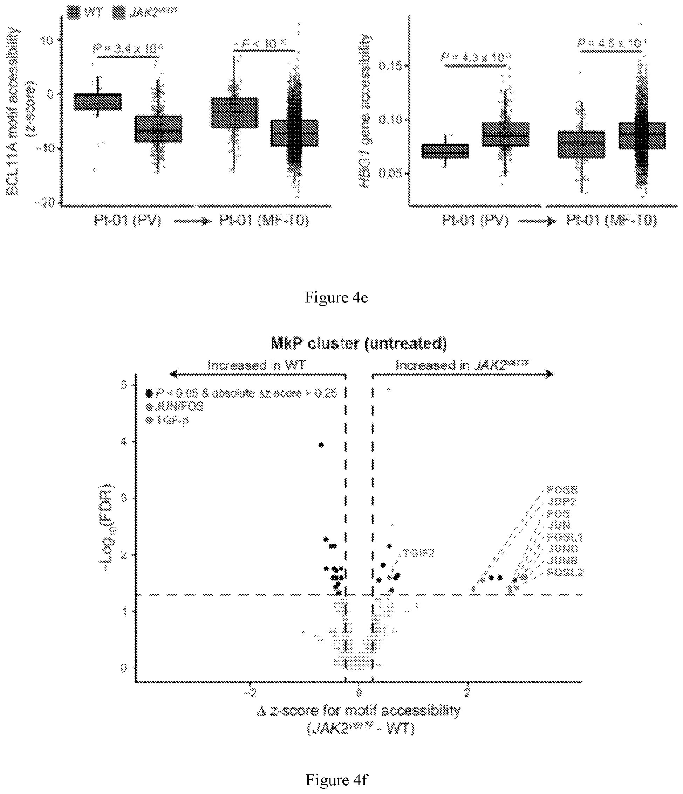

Of note, BCL11A was one of the top transcription factors noted to have decreased motif accessibility in mutated erythroid progenitors (FDR = 0.0032, Dz-score = -2.84; Fig. 4a). BCL11A is required for repression of the g-globin gene (HBG1), and loss of BCL11A is sufficient for increased expression of HBG, a component of fetal hemoglobin (HbF). To further explore the changes in the regulatory elements controlling the hemoglobin locus, scATAC-seq data is particularly well suited for defining pairs of coaccessible regions within the same single cell, which reflect coordinated activation of regulatory elements. By utilizing single-cell genotyping with GoT-ChA, we performed co-accessibility analysis of the hemoglobin locus region in either wildtype or JAK2^{V617F} mutant erythroid progenitors. We found that an enhancer region (ENCODE accession EH38E1516933) upstream of HBG1 showed increased co-accessibility in JAK2^{V617F} mutant erythroid progenitors when compared to their wildtype counterparts (Fig. 4b, red highlighted region), while another enhancer region located within the locus control region124 controlling the b-globin expression showed consistent co-accessibility across wildtype and JAK2^{V617F} mutant cells (Fig. 4b, grey highlighted region). These data provide further insight into reports that have shown an increase in HbF in MPN patients. By leveraging GoT-ChA and comparing genotyped cells, we observed a consistent increase in the accessibility of HBG1 (Fig. 4c) proportional to the JAK2^{V617F} allelic burden, while no apparent changes were observed in accessibility of the b-globin gene (HBB; Fig. 4c). Indeed, we observed an increase in the proportion of mutant cells in the EP1 cluster showing accessibility of the HBG1 gene (Fig. 4d, upper panel; Fisher exact test), while HBG1 gene accessibility increased proportionally to the mutated allele burden (Fig. 4d, bottom panel; LMM followed by likelihood ratio test). By leveraging longitudinal sampling of Pt-01 (PV ^{®} MF), we observed that decreased BCL11A motif accessibility as well as increased HBG1 gene accessibility are already present at the PV stage (Fig. 4e; Wilcoxon rank sum test) and remain upon progression to MF. While increased HbF levels often result from defective erythropoiesis, our data reveals that cell-intrinsic regulatory changes in JAK2^{V617F} mutant erythroid progenitors may also promote HbF and precede the onset of marrow fibrosis. In further support of a cell-intrinsic mechanism, these changes were reverted by ruxolitinib treatment (Fig. 11b-d), indicating the requirement of constitutive JAK2^{V617F} activity for the increased accessibility of HBG1 and decreased accessibility of BCL11A binding motifs.

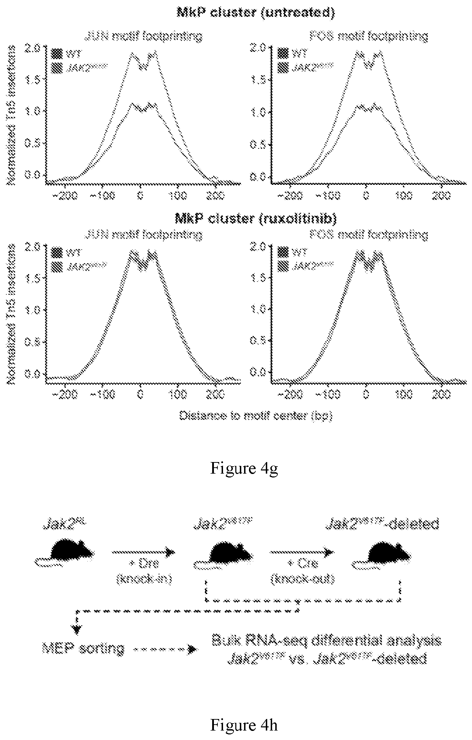

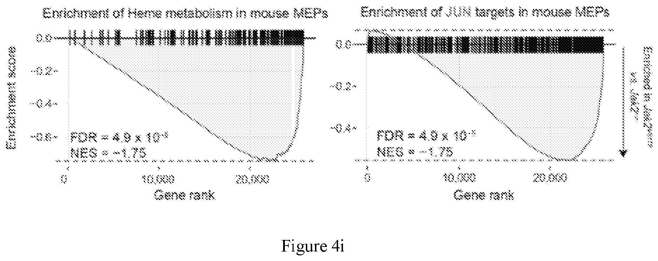

A key clinical feature of myelofibrosis is a dramatic increase in megakaryocytes, which are thought to be one of the main cellular drivers of marrow fibrosis via pro-fibrotic cytokine and growth factor signaling. Differential transcription factor motif accessibility in MkPs revealed an increased activity of JUN and FOS family proteins (Fig. 4f), which have recently been implicated in inflammation and as important mediators of fibrosis in various conditions, including myelofibrosis. In contrast, differential transcription factor motif accessibility signals were reverted by ruxolitinib treatment (Fig. 11e), consistent with mutated versus wildtype motif footprinting analysis for JUN (Fig. 4g). To validate the dependency of JUN/FOS activation on JAK2^{V617F}, we again interrogated the reversible Jak2RL mouse model (Fig. 4h). Consistent with our findings in human myelofibrosis, differential bulk RNA-seq analysis of sorted megakaryocytic-erythroid progenitors (MEPs) showed depletion of an erythroid gene set as well as JUN targets upon reversal of the Jak2^{V617F} allele (Fig. 4i), demonstrating that Jak2^{V617F} is required for increased JUN activity in MEPs. Taken together, these data demonstrate cell type-specific and cell-intrinsic alterations in the epigenetic landscape leading to aberrant erythropoiesis and increased pro-fibrotic JUN/FOS activation in megakaryocyte progenitors in MF.

### Example 7: GoT-ChA with select antigen profiling allows mitochondrial-based genotyping imputation and protein measurement integration.

To expand the reach of GoT-ChA for multimodality single-cell sequencing, we integrated it with ASAP-seq, a method that assays genome-wide chromatin accessibility simultaneously with targeted protein expression utilizing fixed whole cells (rather than nuclei in standard scATAC-seq). We applied the combined method to two MF patient samples, Pt-02 (untreated) and Pt-06 (ruxolitinib treated). Genotyping of the JAK2V617 locus was available for 2,663 out of 11,457 (23.2%) and for 489 out of 2,928 (16.7%) cells for Pt-02 and Pt-06, respectively (Fig. 12a), while retaining chromatin accessibility quality (Fig. 12b-c). We note a decrease in genotyping rate with GoT-ChA-ASAP (23.2% and 16.7% for Pt-02 and Pt-06, respectively) compared to when the samples were profiled with GoT-ChA alone (51.8% and 27.8% for Pt-02 and Pt-06, respectively), likely due to the fixation conditions required for ASAP-seq. However, by assaying entire cells rather than isolating nuclei during sample preparation, ASAP-seq results in a massively increased coverage of the mitochondrial genome (Fig. 5a). We hypothesized that JAK2^{V617F} mutant cells might harbor mitochondrial variants, as previously observed31 (Fig. 12d), that could be utilized for imputation of genotypes in cells where the JAK2 locus was not captured directly. Of the two GoT-ChA-ASAP samples, only Pt-02 showed two mitochondrial variants (12,786 A>G and 3,834 G>A) at high heteroplasmy in JAK2^{V617F} cells (average heteroplasmy of 77.7% and 18.1%, respectively), while exhibiting low heteroplasmy in wildtype cells (average heteroplasmy of 7.85% and 1.47%, respectively; Fig. 5b). While both mitochondrial variants were in phase with the JAK2^{V617F} mutation, they were mutually exclusive, suggesting that they were acquired early after the JAK2^{V617F} mutation.

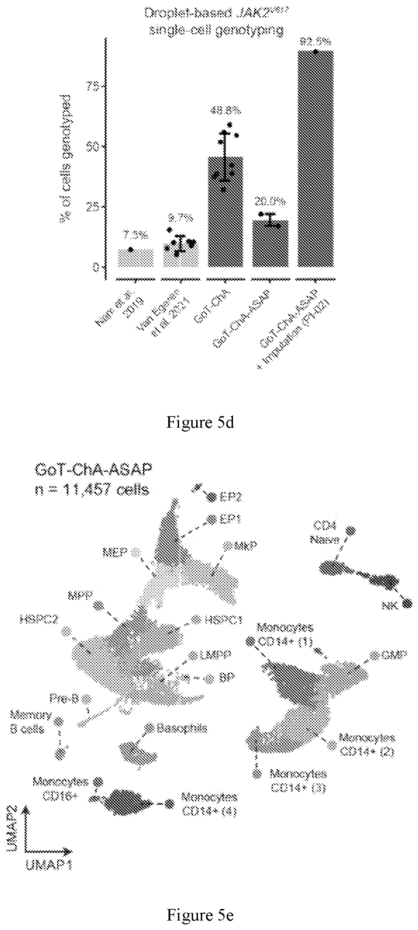

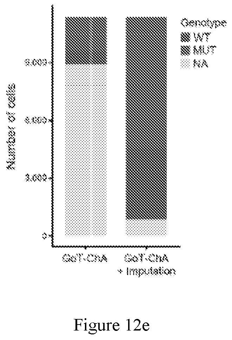

To leverage the clonal phasing of mitochondrial variants and JAK2^{V617F}, we developed a random forest classifier to impute missing JAK2 genotypes based on heteroplasmy levels. We trained the classifier on a random sampling of 90% of Pt-02 genotyped cells (training set) and assessed performance with the remaining 10% of genotyped cells (test set), resulting in a genotyping accuracy of 94.7% (Fig. 5c). Implementation of the classifier on non-genotyped cells resulted in a striking increase in genotyped cells from 23.2% to 92.53% of cells (Fig. 5d; Fig. 12e). Thus, while standalone GoT-ChA already demonstrated a significant improvement for genotyping loci that were previously challenging due to low expression and mutation location, the additional implementation of an imputation classifier built on phased mitochondrial variants yielded the ability to accurately genotype nearly all cells within the sample.

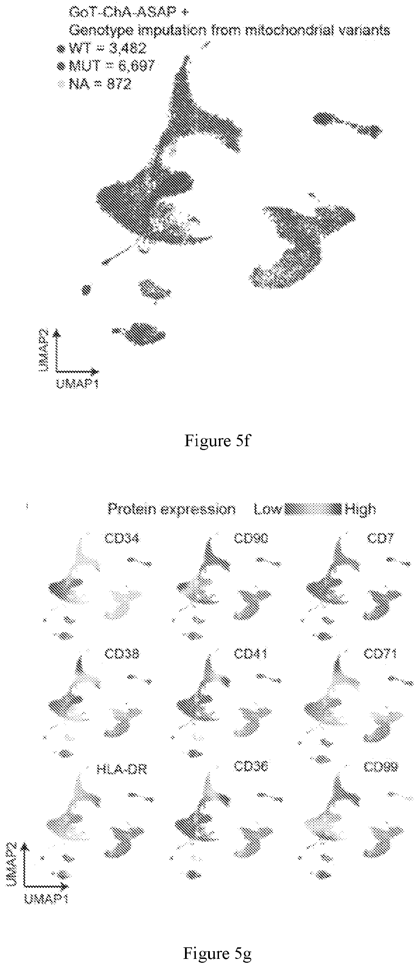

Cell clustering of the Pt-02 sample based on chromatin accessibility alone, and therefore agnostic to genotyping, protein, or mitochondrial data, resulted in the expected cell clusters (Fig. 5e) identified via accessibility of canonical marker genes (Fig. 12f-g). Importantly, while mutant and wildtype cells intermingled in most progenitor subtypes, HSPC clusters 1 and 2 were predominantly enriched by wildtype and mutant cells, respectively, suggesting a discrete enrichment of later HSPCs (HSPC2) with JAK2^{V617F}-mutated cells in this sample (Fig. 5f).

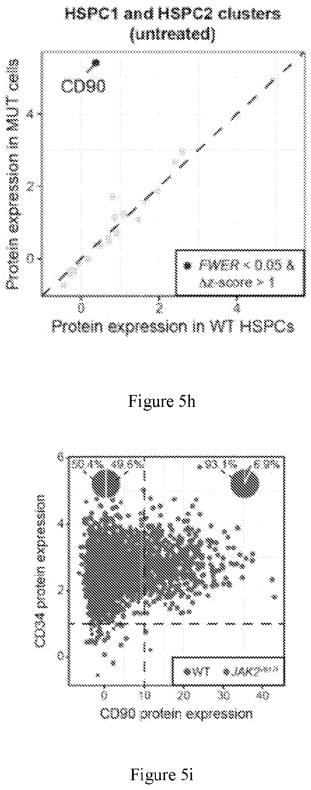

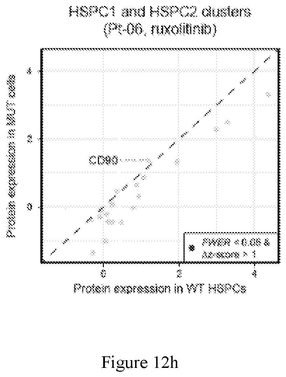

We further leveraged GoT-ChA-ASAP for simultaneous measurement of protein expression, applying a panel of 21 cell surface protein markers to orthogonally validate cluster assignments (see materials and methods). Progenitor clusters HSPC1, HSPC2, and MPP showed increased CD34 and decreased CD38 staining, while MkPs showed increased CD41 and CD36, EPs showed high CD71, lymphoid clusters showed high CD99 staining, and T-cells showed high CD7 levels(Fig. 5g). Differential protein expression comparisons showed that mutant HSPCs exhibited higher expression of CD90 than their wildtype counterparts (Fig. 5h; Dzscore > 1 and FWER < 0.05; Wilcoxon rank sum test followed by Bonferroni correction), while Pt-06, undergoing ruxolitinib treatment at the time of sample collection, showed no significant changes in CD90 protein expression levels between wildtype and JAK2^{V617F} cells within HSPCs (Fig. 12h). Indeed, Pt-02 CD34high, CD90high HSPC cells were comprised of 93.1% mutant cells compared to 50.4% of CD34high, CD90low cells (Fig. 5i). Concordantly, Pt-02 mutant HSPCs exhibited an increase in gene accessibility of the CD90 gene (THY1; FDR = 1.1 x 10⁻³), as well as in accessibility of a proximal upstream regulatory element (FDR = 4.7 x 10⁻³) relative to wildtype cells (Fig. 5j). Importantly, we observed increased accessibility of the CD90 gene in HSPCs in the other untreated MF samples in this cohort (Fig. 5k, upper panel; P = 5.3 x 10⁻⁵; LMM followed by likelihood ratio test, HSPC1 and HSPC2 combined; see Fig. 12i for per patient analysis) while ruxolitinib treatment reverted this effect (Fig. 5k, bottom panel; P = 0.98; LMM followed by likelihood ratio test, HSPC1 and HSPC2 combined). CD90 is a cell surface marker expressed in a subset of primitive HSPCs131. Indeed, expansion of the CD90high HSPC cell population has been reported in PV23. Furthermore, Rodriguez-Meira and colleagues also noted aberrant expression of CD90 in homozygous JAK2^{V617F} MPN HSPCs using TARGET-seq. However, due to the lower throughput of plate-based methods, this observation was supported by only five cells, demonstrating the advantage of high-throughput single-cell multi-omics integration.

Overall, these results demonstrate the capability of GoT-ChA to deliver a highly multi-modal single-cell platform to link mutated genotypes with mitochondrial variants and cell surface proteins, together with chromatin accessibility, enabling discovery of clonal changes across multiple layers of information in a single, high-throughput, unified assay.

### Example 8: Genotyping of Transcriptomes links somatic genotyping and single-cell RNA-seq in high throughput.

To overcome the limitations of existing technology, we adapted the droplet-based scRNA-seq platform (10x Genomics) to enable capture of somatic mutations in single cells. After generation of the barcoded cDNA library, we amplify the locus of interest, and integrate genotyping with scRNA-seq data via shared cell barcodes, demonstrating precision genotyping via species mixing studies (Fig. 15a).

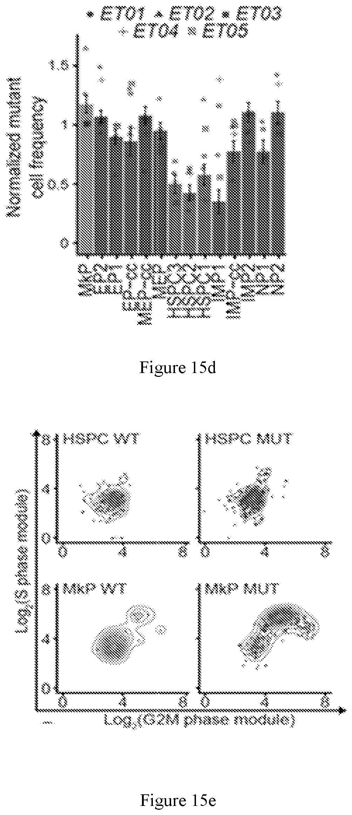

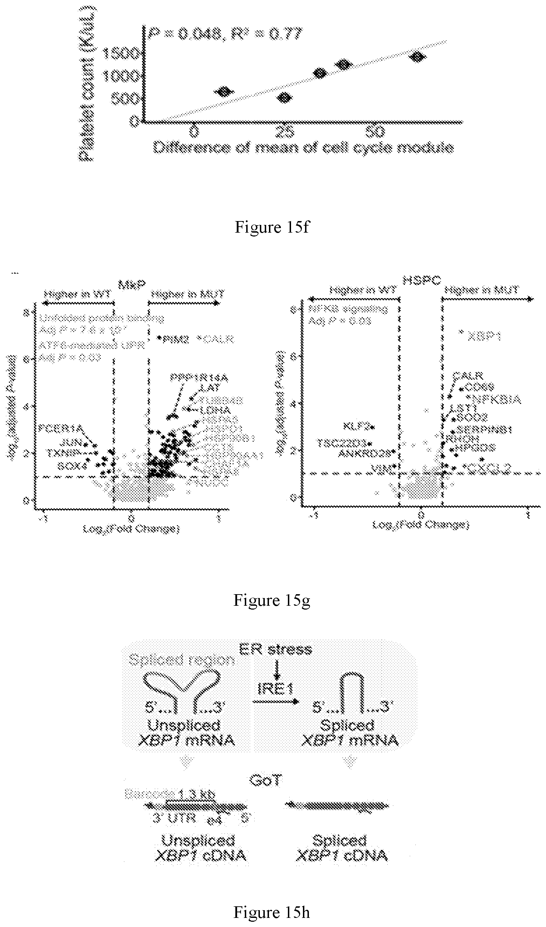

We studied CD34⁺ cells from patients with *CALR*-mutated essential thrombocythemia. Mapping of mutant and wildtype cells across the differentiation topology demonstrated that they co-mingle throughout development, consistent with mutations arising in stem cells to populate the entire differentiation tree (**Fig. 15b-15c**). However, mutant cell frequency increased with differentiation toward committed myeloid progenitors vs. early hematopoietic stem progenitor cells (HSPCs, **Fig. 15d**). We further observed cell cycle-related genes upregulation (**Fig. 15e**), correlated with the patients' platelet count (**Fig. 15f**). Importantly, these data enable *de novo* differential expression analysis by comparing the gene expression of mutant and wildtype cells within the same progenitor subset. We identified the upregulation of the unfolded protein response (UPR), as well as upregulation of *XBP1,* an important regulator of the IRE1 arm of UPR linked to enhanced cell survival (**Fig. 15g**). We further repurposed genotyping of transcriptomes to capture the *XBP1* splice site at single-cell resolution (**Fig. 15h**) and showed robust *XBP1* splicing in mutant cell, as a functional read out of IRE 1 activity (**Fig. 15i**).

As ongoing clonal evolution results in multi-clonal populations, we require genotyping of multiple mutations in parallel. To test this ability, we targeted 3 mutations in CD34⁺ cells from a patient with myelofibrosis (**Fig. 16a**). In addition to clonal *SF3B1* mutations, we obtained genotyping for *CALR* and *NFE2* in 74% and 60% of cells, respectively, validated with single-cell cloning. This allowed us to compare the transcriptional outputs of the different mutations alone or in combination (**Fig. 16b**). Thus, multiplexed genotyping demonstrated the ability to interrogate complex clonal structures, as well as the need to assess the combinatorial transcriptional output of multiple mutations in the context of cell identity mapping.

### Example 9: Clonal mosaicism DNMT3A^{R882} mutations linked with aberrant cellular phenotypes in primary normal human samples

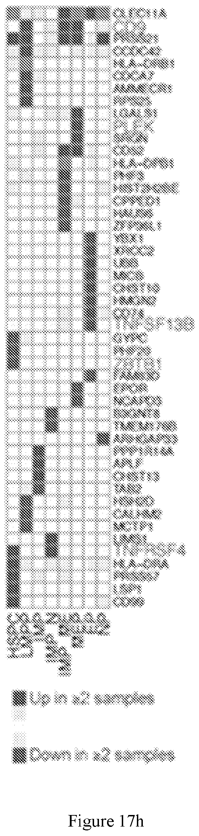

To demonstrate the unique discovery potential of single-cell multi-omics in the context of clonal mosaicism in normal tissues, we applied it to CM driven by DNMT3A^{R882} mutations (**Fig. 17a-17c**). Specific differentiation skews were apparent corresponding to differences in *DNMT3A* expression along differentiation (**Fig. 17d-****17f**). *DNMT3A^{mut}* progenitors showed a myeloid bias and disfavored lymphoid development (**Fig. 17g**). We further identified dysregulated expression of lineage markers, and dysregulated expression of genes involved in TNF-alpha signaling (e.g. *TNFRSF4, TNFSF13B),* suggesting a pro-inflammatory state (**Fig. 17h**). Gene set enrichment analysis revealed activation of MYC, IL-6 signaling and NFKB/REL.

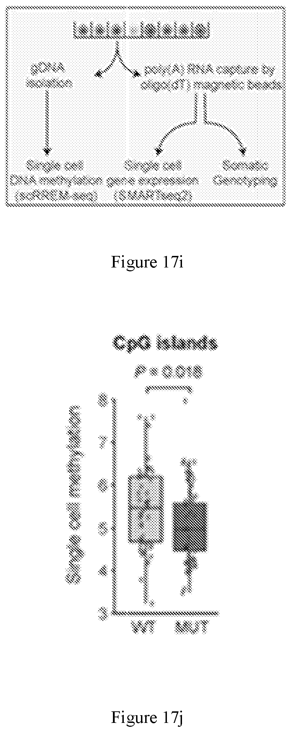

To determine how aberrant DNA methylation may serve as a link between mutations in the *de novo* methylase *DNMT3A* and observed transcriptional dysregulation, we performed single-cell multi-omics that simultaneously profiles the cells' methylome and transcriptome, linked with mutation status (**Fig. 17i**). By comparing the methylation rates in mutated vs. wildtype CD34⁺ cells within the same sample, we found that DNMT3A^{R882} results in selective hypomethylation that preferentially impacts CpG islands and PRC2 targets (**Fig. 17j-17k**). Notably, *DNMT3A*^{R882}-induced hypomethylation was enriched in a specific sequence context in which the CpG is followed by a T nucleotide (**Fig. 17l**). This *DNMT3A*^{R882} motif was enriched in the DNA binding motifs of key hematopoietic TFs (**Fig. 17m**), providing a mechanism of enhanced MYC activity (**Fig. 4N**). Collectively, these results highlight single-cell multi-omics as a powerful tool for interrogating somatic mutations directly in primary normal human tissues.

### Example 10: Genotyping of Targeted loci: a modular approach for genotyping single cells compatible with multiplexed single cell NTT-seq.

Nanobody-tethered transposition may be performed as detailed at world wide web at biorxiv.org/content/10.1101/2022.03.08.483436v1

Profiling chromatin status at single-cell resolution has an enormous impact on functional epigenomic characterization in a variety of biological contexts. However, available protocols typically measure only a single histone modification profile in a single cell. Recently, the development of Nanobody-tethered transposition followed by sequencing (NTT-seq) allowed for multiplexed measurements of histone post-translational modifications and DNA binding proteins. Thus, up to three epigenomic features can be jointly measured in the same cell.

Since NTT-seq is based on the 10X Genomics single cell ATAC-seq platform, it would be compatible with the genotyping capture approach used in GoT-ChA. In this manner, multiple epigenomic features can be measured simultaneously with genotyping, in the same single cell.

How does it relate to the main protocol? Adapting the genotyping step from the GoTChA protocol into the NTT-seq protocol is straightforward (**Figure 13a**).

The proposed modification adds an additional and unique feature to our genotyping method, as now epigenomic measurements can be extended to features beyond the chromatin accessibility profiles, towards specific post-translational histone modifications and DNA binding proteins. While not essential to practicing the invention, it uniquely allows to uncover how somatic mutations disrupt the specific epigenetic features targeted in the context of clonal mosaicism and cancer.

### Example 11: Preliminary results - Multiplexed single cell GoT-NTT-seq for phosphorylated RNA polymerase 2 and H3K27me3 cell mixing study.

To capture multiple epigenetic modifications simultaneously with genotypes, we developed GoT-NTT-seq. After nuclei isolation, staining with the primary antibodies against the targets of interest is performed. Critically, primary antibodies must be from different species or immunoglobulin isotypes to allow multiplexing. Next, stained nuclei are incubated with NTT-bodies directing transposition events towards regions in which the primary targets are bound. Importantly, each NTT-body contains a specific barcoded adaptor allowing for downstream de-multiplexing of the targeted sequences. After transposition, nuclei are flowed into the 10X Chromium solution machine, together with locus-specific genotyping primers (GoT primers). Importantly, as in GoT-ChA, the GoT primers contain a partial overlap with read 1 sequence, allowing capturing of the genotyping fragments by the gel beads containing the unique cellular barcodes. Once the fragments are generated, downstream library preparation and sequencing are performed (**Figure 13a**).

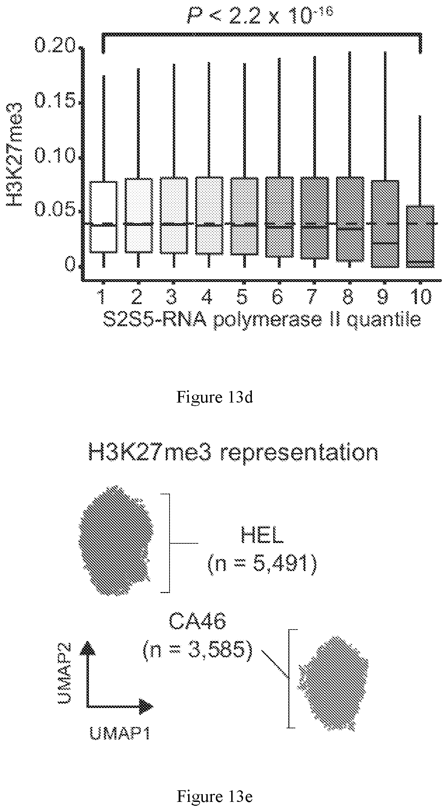

To test the feasibility GoT-NTT-seq, we performed a cell mixing study targeting the *TP53* locus. We first generated a 1:1 mix of both HEL (erythroblastic cell line homozygous wild type for TP53-R248) and CA46 (lymphoblastic cell line homozygous mutant for TP53-R248). To verify our multiplexing capabilities, we targeted both phosphorylated RNA polymerase II (S2S5-PolII) and the repressive histone modification H3K27me3. Quality control of the GoT-NTT-seq data showed that inclusion of the genotyping approach was not detrimental (**Figure 13b-13c**). As expected, genomic regions with high S2S5-PolII showed depletion of H3K27me3 (**Figure 13d**). We next performed clustering based on H3K27me3 profiles, in a manner agnostic to genotype (**Figure 13e**). Cell type identification was verified by H3K27me3 signal in genes associated with erythroblastic or lymphoblastic characteristics (**Figure 13f-13g**). Most importantly, GoT-NTT-seq resulted in 6.3% of genotyped cells with an accuracy of 94.2% (**Figure 13h**). Thus, GoT-NTT-seq allows for multiplexing of histone modifications and DNA binding proteins in phase with genotyping at single cell resolution.

### Example 12: Develop, optimize, and multiplex GoT-ChA (Chromatin Accessibility) to define the chromatin landscape of somatic driver mutations in clonal mosaicism (CM).

Linking cell genotypes with cell phenotypes (epigenetic, transcriptional and protein profiling) dramatically empowers our ability to study somatic evolution in normal tissues. Specifically, we develop:
GoT-ChA, to jointly capture mutations and snATAC-seq profiles to study the chromatin impact of clonal mutations in normal human samples;
GoT-EpiM, to jointly capture mutations and histone or DNA modifications profiles to study the epigenomic impact of clonal mutations in normal human samples;
GoT-ChA-RNA, to jointly capture mutations, transcriptomes and snATAC-seq profiles to comprehensively study the phenotypic impact of clonal mutations in normal human samples;
GoT-ChA-Pro, to jointly capture mutations, intranuclear proteins and snATAC-seq profiles to broaden the study of the phenotypic impact of clonal mutations in normal human samples; and
Advanced computational frameworks for precision integration of multi-omics single-cell data, building and extending on our established pipelines (e.g., IronThrone17 and GoT-ChA21).

Current state-of-the-art technology is limited in its ability to connect genotypes with other '-omics' profiles at the single-cell level. Methods that integrate DNA genotyping with RNA expression in single cells have been developed. For example, G&T-seq can capture single-cell genomic and whole transcriptomic data by extracting and separating gDNA and mRNA for amplification and sequencing. scRNA-seq data has also been used to identify single-nucleotide variant (SNV) drivers via the Smart-seq2 protocol, which captures full-length transcripts. TARGET-seq uses sensitive genotyping through targeted amplification of both genomic DNA (gDNA) and complementary DNA (cDNA) to enable integration of clonal structure with transcriptomic states. Finally, fluidics methods for single-cell genotyping have recently been coupled with oligo-barcoded antibodies, although these methods are limited to a small number of cell surface markers. However, collectively, these technologies share similar limitations such as low-throughput or low efficiency of somatic genotyping due to sparse coverage, and may therefore be insufficient to fully map clonal expansions in tumorigenesis and therapeutic escape. This highlights the critical need for high-throughput methods to integrate single-cell genotyping with regulatory, transcriptional and protein data.

**Rationale.** Epigenetic rewiring determines lineage commitment and stem cell identity. In particular, chromatin accessibility states have been shown to precede differentiation states via 'priming' of cells toward specific lineages. Therefore, epigenetic encoding likely plays a significant role in the interplay between the impact of somatic mutations and the cell identity leading to clonal outgrowth. To directly link clonal genotypes and chromatin accessibility, we develop GoT-ChA, adapting 10X snATAC to include *gDNA-based* genotyping.

**Approach.** To determine the regulatory networks that govern the impact of somatic mutations, we have developed a *prototype* single-cell chromatin accessibility assay that integrates somatic mutation genotyping (**GoT-ChA**, **Fig. 18a**). Briefly, we modified the broadly utilized 10x Genomics platform by adding two custom primers (GoT-ChA primers) to the cell barcoding PCR reaction mixture prior to loading the microfluidics chip for droplet generation (**Fig. 18a**). These primers are designed to flank a specific region of interest, with one primer containing a Read 1 Nextera (R1N) handle. During in-droplet PCR, the GoT-ChA primers bind to and amplify the genomic region of interest, generating an amplicon that is complementary to the 10x snATAC-seq Gel Bead oligonucleotides, allowing for cell barcoding and further amplification. In this manner, both tagmented gDNA fragments and GoT-ChA amplicons simultaneously obtain the unique cell barcode sequence and the P5 Illumina sequencing handle prior to breakage of the single-cell emulsion. Of note, tagmentation of the targeted site is not required for genotype capture, as target amplification with GoT-ChA primers results in amplicons already containing the R1N capture sequence. Moreover, while in-droplet amplification of tagmented genomic fragments is linear, GoT-ChA amplicons are exponentially amplified by design, resulting in an increased relative abundance of genotyping amplicons. During genotyping library construction, GoT-ChA fragments are specifically amplified using a hemi-nested PCR strategy and a biotin-streptavidin pulldown prior to sample indexing. Both the snATAC-seq and GoT-ChA libraries can then be sequenced together, with chromatin accessibility and genotype information linked via shared cell barcodes. Our data shows that fragment size distribution, total number of fragments per cell, and enrichment of fragments mapping to transcriptional start sites (TSS) all reflect high quality scATAC-seq data, unaffected by GoT-ChA genotyping.

Our preliminary application of GoT-ChA to *JAK2* mutated myelofibrosis CD34⁺ cells showed effective genotyping together with cell identity identification using chromatin accessibility profiles (**Fig. 15c-15d**). Building on our initial prototype, we further test and optimize GoT-ChA to profile a wide variety of genomic loci, first individually, and next, in parallel. We focus on targets commonly observed in CM across tissues (*SF3B1, DNMT3A, NOTCH1, NOTCH2, ARID1A, FAT1, TP53, PIK3CA, FBXW7).* Optimization is less complex than in the case of cDNA-based genotyping as the primer capture is not based on the poly-A sequencing, and therefore not impacted by the mutation distance from transcript end.

To develop and test the ability of GoT-ChA to target mutations across genes of interest, we perform GoT-ChA on admixtures of cell lines with known variants across the genes, cluster and identify cell lines via gene accessibility patterns (see **Fig. 18b**), and apply our GoT-ChA genotyping pipeline. Briefly, we use kernel density estimation (KDE) to define the boundaries between background noise and genotyping signal, followed by clustering and genotype assignment based on the z-scores of genotyping read counts. A proof-of-principle mixing study using two human cell lines of distinct JAK2 V617 genotypes, showed genotyping coverage of 56.5% of single cells with a genotyping accuracy of 98.4% (**Fig. 18b**). Importantly, target capture is done directly at the gDNA level, overcoming the limitation of cDNA-based genotyping in capturing lowly expressed genes (for example, capture of only 7% of CD34+ for the JAK2^{V617}). Of note, this dependency on expression is compounded in the application to nuclei with an even lower number of transcripts (e.g., <2% genotyping efficiency when applying genotyping of transcriptomes to single nuclei compared with 35% in single cells), highlighting the unique advantage of gDNA-based genotyping in GoT-ChA.

Our preliminary experience with the GoT-ChA application demonstrated robust genotyping across TP53 mutations as well as a JAK2 mutation (>50% genotyping efficiency, >98% precision), as well as the ability to capture multiple alleles in a heterozygously mutated locus. Notably, performance was maintained between cell line admixtures and clinical samples in terms of genotyping efficiency. The clinical samples analysis also confirmed efficient genotyping across cell type without a strong dependence of chromatin accessibility as anticipated from the methods' design. These data importantly validate the ability of cell line admixtures proposed in the UG3 phase to serve as a robust tool for technology development.

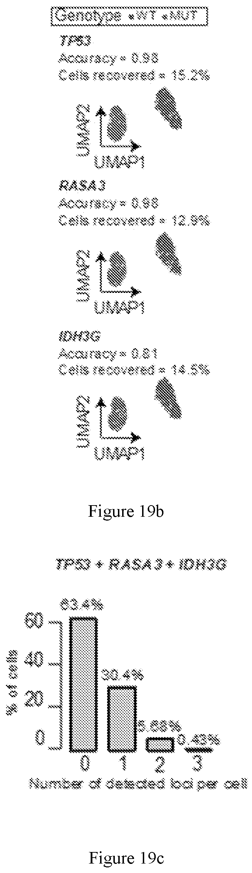

Lastly, GoT-ChA is expanded to enable substantial multiplexed genotyping (20-30 loci total across the 10 recurrently mutated genes), particularly as many tissues exhibit complex clonal structures involving multiple concurrent mutations. Primer design is optimized by testing multiple primers for each locus to assess the impact of sequence specificity, distance from the targeted locus and the impact of local chromatin structure. These data aid in generating a primer design interface to ensure that GoT-ChA will be compatible with production level scale. Our preliminary data supports multi-plexing for up to 3 targets (**Fig. 19**).

### Example 13: Develop GoT-EpiM (Epigenetic Modifications) to define the epigenomic landscape of somatic driver mutations in CM.

**Rationale.** Histone modifications act in a concerted fashion to regulate chromatin states and transcription. While accessibility measurements from ATAC-seq are informative, the diversity and richness of chromatin states are best captured through the combinatorial binding patterns of multiple histone modifications. These assays can reveal the presence of poised and active enhancers, promoters, repressed, and heterochromatic regions. Profiling multiple chromatin marks is crucial for thorough chromatin state characterization in heterogeneous tissues. Up to now, several high throughput chromatin factor-profiling assays have been developed. Cleavage Under Targets and Tagmentation (CUT&Tag) has most successfully been adapted to single-cell resolution assays. Briefly, CUT&Tag generates a library from DNA fragments colocalizing with the chromatin factor of interest by fragmenting out DNA surrounding antibody-bound chromatin factor. This process is facilitated by pA-Tn5 fusion protein, where protein A (pA) has an affinity to antibodies and Tn5 is a transposase that fragments DNA and tags fragments with sequencing-ready adapters. Given our ability to add genotyping to snATAC-seq, integration with additional epigenetic modification profiling expands our ability to understand the impact of somatic mutations on the epigenome.

**Approach.** Available protocols typically measure only a single histone modification profile in a single cell. To fill this technological gap in the field, our group has adapted the CUT&Tag method into a multiplexable assay, where the protein A-Tn5 construct is replaced by selective Nanobody-tethered Tn5 (NTT-body) fusion proteins (**Fig. 13a**). When primary antibodies from different species or immunoglobulin isotypes are used to stain different chromatin marks, NTT-bodies with high specificity and affinity to antibodies from different species can selectively profile these chromatin features as compared to indiscriminate recognition of all antibody-bound epitopes by pA-Tn5. Importantly, each NTT-body contains a specific barcoded adaptor allowing for downstream de-multiplexing of the targeted sequences. We have previously demonstrated how scNTT-seq can simultaneously up to four chromatin targets simultaneously in primary human bone marrow samples.To test the feasibility of integrating genotyping with NTT-seq (**GoT-EpiM**), we performed a cell mixing study targeting the *TP53* locus (HEL homozygous wild type and CA46 homozygous mutant for TP53-R248). To verify our multiplexing capabilities, we targeted both phosphorylated RNA polymerase II (S2S5-PolII) and the repressive histone modification H3K27me3. Quality control of the data showed that inclusion of the genotyping approach was not detrimental (**Fig. 13b-13c**). As expected, genomic regions with high S2S5-PolII showed depletion of H3K27me3 (**Fig. 13d**). We next performed clustering based on H3K27me3 profiles, in a manner agnostic to genotype (**Fig. 13e-13g**). These first experiences with GoT-EpiM resulted in 6.3% of genotyped cells with an accuracy of 94.2% (**Fig. 13h**), demonstrating its promise for multiplexing of histone modifications and S2S5-PolII occupation with genotyping at single cell resolution. Additional preliminary experiments demonstrated the feasibility of simultaneously targeting H3K27Ac, and H3K27me3, together with genotyping at 7% resolution.

The genotyping efficiency of GoT-EpiM is optimized. The genotyping efficiency is increased through: i) producing high quality barcoded recombinant NTT-bodies, minimizing the amount of free sequencing adaptors that can interfere with the genotyping reaction; ii) titering the amount of genotyping primers to improve the efficiency of the reaction; and iii) we aim to prolong the denaturation and hybridization steps in PCR to increase PCR fidelity in a microdroplet as has been done previously. This approach results in a higher percentage of cells for which we can generate high-confidence genotyping data.

### Example 14: Develop GoT-ChA-RNA to jointly define the chromatin and transcriptional phenotypes of somatic driver mutations in CM.

**Rationale.** Our work using genotyping of transcriptomes (see **Fig. 15-17**) has shown that cells carrying somatic mutation are transcriptionally distinct from wildtype cells in normal tissue. Transcriptional profiling allows deep cellular phenotyping to identify how somatic mutations alter cells to allow clonal outgrowth. However, as described above, genotyping of transcriptomes leads to limiting dependencies on gene expression of the mutated locus. The 10X genomics multi-ome kit is optimized to be more compatible with GoT-ChA due to lack of in-droplet PCR critical for target locus exponential amplification. A substantial extension enabling simultaneous measurement of genotypes, chromatin accessibility and transcriptomes simultaneously in single cells (GoT-ChA-RNA) is designed to identify the transcriptional, phenotypic alterations downstream of somatic driver mutations leading to clonal outgrowth.

**Approach.** To accomplish this goal, we leverage recent advances that report the ability to perform tagmentation on RNA/DNA hybrid molecules, including RNA/cDNA hybrid molecules that arise from reverse transcription. Importantly, recent work (ISSAAC-seq) demonstrates the ability to perform this tagmentation *in-situ* and in single-cells without performing cellular lysis, allowing tagmentation to take place without physically separating a cell's RNA and chromatin molecules. Reverse transcription is performed *in situ,* which creates an RNA/cDNA hybrid. When performing this workflow, the 10X ATAC-seq bead can capture tagged chromatin fragments and cDNA molecules using the capture sites that are introduced during tagmentation. The 10X protocol with integrated GoT-ChA adds a droplet barcode, which can uniquely identify the cell-of-origin for each modality after computational demultiplexing, allowing simultaneous genotype, chromatin accessibility and transcriptome profiling.

To test the feasibility of this approach, we performed a pilot species-mixing experiment where we simultaneously profiled chromatin accessibility and mRNA expression using the ISSAAC-seq protocol, followed by shallow (MiSeq) sequencing. Using a single lane of the 10x snATAC-seq kit, we successfully recovered paired modality profiles for 8,257 cells. Importantly, both modalities exhibited a high degree of species-specificity, and species classifications were fully concordant across paired RNA and ATAC profiles (**Fig. 20**).

### Example 15: Develop GoT-ChA-Pro to jointly define the chromatin landscape and protein abundance landscape of somatic driver mutations in CM.

**Rationale.** Integrating protein measurements into multimodal single-cell assays is widely adopted in the biological community (e.g., CITE-seq). Protein-measurements are robust, particularly given their large copy number per cell, and we have shown that these data are highly complementary to scRNA-seq and informative for discovering cell types, states, and developmental trajectories. We introduced ASAP-seq, an assay that marks the location of DNA-protein interactions while simultaneously quantifying cell surface protein levels based on a panel of barcoded antibodies (antibody-derived tags; ADT). While we have shown that this approach is compatible with GoT-ChA and allows to connect somatic genotypes with protein expression, this has been limited to cell surface markers. Given the use of extracted nuclei from frozen solid tissues, we require a technology that can connect genotypes with intra-nuclear protein capture (such as transcription factors) to highlight regulators whose abundance varies across somatic genotypes.

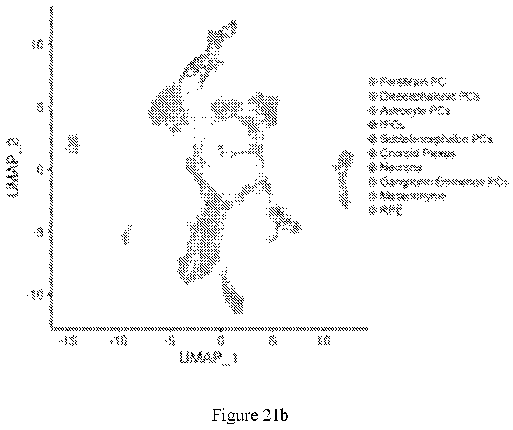

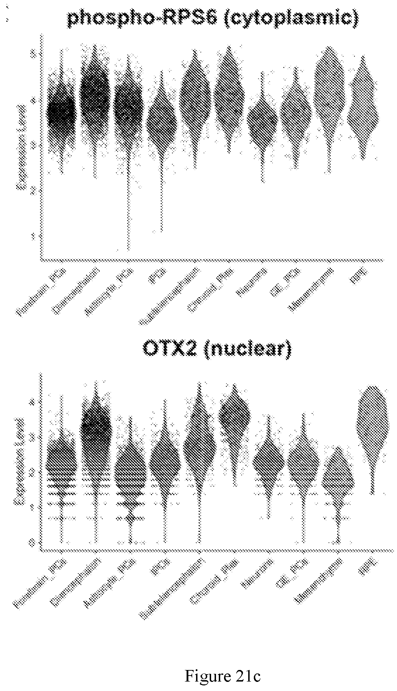

**Approach.** We have developed a single-cell profiling assay that simultaneously measures chromatin accessibility with intranuclear proteins (i.e. transcription factors), and intracellular proteins (i.e. phosphorylation-dependent signaling regulators). We combine the use of single-stranded binding protein to mask the negative charge of ADTs of intracellular proteins, substantially reducing background signal. In preliminary experiments, we profiled in-vitro human brain organoids, generating high-quality chromatin accessibility measurements (7,095 fragments/cell), paired with measurements of 64 intracellular/nuclear proteins (**Fig. 21**). Unsupervised analysis of scATAC-seq data revealed the presence of distinct neuronal linages, which exhibited striking heterogeneity in protein abundance for transcriptional regulators (i.e. OTX2, SOX2), as well as signaling regulators (e.g., pRPS6, marker of mTORC signaling). Importantly, each of our intranuclear antibodies was conjugated using an optimized iEDDA click chemistry protocol. We combine this approach with GoT-ChA technology, yielding a scalable assay for simultaneous profiling of somatic genotypes, chromatin accessibility with large panels of intranuclear Proteins (GoT-ChA-Pro). The first optimization is to identify optimized fixation and permeabilization conditions that enable robust genotyping, while also preserving the nuclear membrane, and allowing nuclear antibody entry for measuring protein abundances. We accomplish this by testing a series of experimental conditions laid out in our previous work, where we found that optimizing the balance of digitonin and Tween-20 concentrations enables compatibility with multiple molecular assays. Second, we construct, optimize, and validate a large panel of intranuclear antibodies for solid epithelial tissue profiling. We conjugate at least two antibodies for each target, and perform an experiment where annotated cell line mixtures are used to cluster cells, allowing us to identify antibodies that exhibit dynamic abundances. We use the Gini index to quantify heterogeneity, and select the best-performing panel of 100 antibodies. We then perform a separate optimization experiment to optimize antibody concentrations. As we have demonstrated, antibody-based multiplexing approaches can be used to efficiently titrate each individual antibody in a large panel over a wide dynamic range, allowing us to systematically identify the amount of each antibody that optimizes signal/noise.

## Claims

1. A method of genotyping a locus, comprising:
generating a plurality of biological particles, each biological particle comprising a polynucleotide;
generating a plurality of partitions, each partition comprising one of the plurality of biological particles, a primer pair, and a barcoded bead; and
wherein the primer pair comprises:
a 5' primer-adapter nucleic acid molecule comprising a nucleotide sequence that is complementary to a sequence located at the 3' end of the locus within the polynucleotide,
a 3' primer-adapter nucleic acid molecule comprising a nucleotide sequence that is complementary to a sequence located at the 5' end of the locus,
wherein the biological particle comprises genomic DNA and chromatin, and wherein the genomic DNA is fragmented by a transposase to generate the plurality of genomic DNA fragments.

2. The method of claim 1, further comprising amplifying the locus using the primer pair to generate a locus nucleic acid fragment, optionally wherein the locus nucleic acid fragment is generated inside the partition.

3. The method of any one of claims 1-2, wherein the 5' end of the 5' primer-adapter nucleic acid molecule further comprises an adapter sequence, optionally wherein the adapter sequence is Read 1 Nextera sequence (R1N), or wherein the 5' end of the 3' primer-adapter nucleic acid molecule further comprises a second adapter sequence, optionally wherein the second adapter sequence is a Read 1 Nextera sequence (R1N), further optionally wherein the locus nucleic acid fragment further comprises a sequence complementary to the Read 1 Nextera sequence.

4. The method of any one of claims 1-3, wherein the barcoded bead is attached to a nucleic acid, optionally wherein the nucleic acid attached to the barcoded bead comprises a common barcode sequence, optionally wherein the nucleic acid attached to the barcoded bead further comprises a sequence complementary to at least a portion of the adapter sequence, and/or wherein the nucleic acid attached to the barcoded bead further comprises a functional sequence configured to attach to a flow cell of a sequencer, optionally wherein the functional sequence for attachment to a sequencing flow cell is a P5 sequence or a P7 sequence.

5. The method of claim 4, further comprising amplifying the locus nucleic acid fragment, or a derivative thereof, using a second primer pair comprising:
a 3' primer-adapter nucleic acid molecule comprising a nucleotide sequence that is complementary to a sequence located at the 5' end of the locus, and
a 5' primer-adapter nucleic acid molecule comprising a nucleotide sequence that is complementary to functional sequence,
thereby generating a plurality of barcoded locus nucleic acid fragments, each fragment of the plurality of barcoded locus nucleic acid fragments comprising the locus, or further comprising amplifying the locus nucleic acid fragment, or a derivative thereof, using a third primer pair comprising:
a 5' primer-adapter nucleic acid molecule comprising a nucleotide sequence that is complementary to a sequence located at the 3' end of the locus, and
a 3' primer-adapter nucleic acid molecule comprising a nucleotide sequence that is complementary to functional sequence,
thereby generating a plurality of barcoded locus nucleic acid fragments, each fragment of the plurality of barcoded locus nucleic acid fragments comprising the locus, optionally
wherein the plurality of barcoded locus nucleic acid fragments comprising the locus are generated inside the partition.

6. The method of any one of claims 1-5, wherein the transposase is fused to a set of secondary nanobodies (nb), optionally wherein a primary antibody is bound to a target of interest on the chromatin, further optionally wherein the transposase-nb fusion protein specifically binds to primary antibody, preferably wherein the transposase is a Tn5 transposase.

7. The method of claim 6, wherein the genomic DNA is fragmented by a transposase-nucleic acid complex to generate a plurality of tagged genomic DNA fragments, optionally wherein the 5' end of each of the tagged genomic DNA fragments comprises an adapter sequence, or the 3' end of each of the tagged genomic DNA fragments comprises a second adapter sequence, further optionally wherein the adapter sequence is a Read 1 sequence (R1N), or the second adapter sequence is Read 2 sequence (R2N).

8. The method of claim 6 or 7, further comprising amplifying the plurality of genomic DNA fragments, or derivatives thereof, by nucleic acid amplification generating a plurality of barcoded genomic DNA fragments, optionally wherein the plurality of barcoded genomic DNA fragments are generated inside the partition, further optionally wherein each fragment of the plurality of barcoded genomic DNA fragments comprises the tagged genomic DNA, preferably wherein each fragment of the plurality of barcoded genomic DNA fragments further comprises the common barcode sequence and/or the functional sequence configured to attach to a flow cell of a sequencer.

9. The method of any one of claims 5-8, further comprising amplifying the barcoded locus nucleic acid fragments, or derivatives thereof, by nucleic acid amplification, optionally further comprising sequencing said barcoded locus nucleic acid fragments or derivatives thereof.

10. The method of claim 8 or 9, further comprising amplifying the plurality of barcoded genomic DNA fragments, or derivatives thereof, by nucleic acid amplification, optionally further comprising sequencing said barcoded genomic DNA fragments or derivatives thereof, further optionally further comprising sequencing said barcoded locus nucleic acid fragments and said barcoded genomic DNA fragments simultaneously.

11. A partition comprising: a biological particle comprising a polynucleotide, a primer pair, and a barcoded bead; and
wherein the primer pair comprises:
a 5' primer-adapter nucleic acid molecule comprising a nucleotide sequence that is complementary to a sequence located at the 3' end of a the locus within the polynucleotide, and
a 3' primer-adapter nucleic acid molecule comprising a nucleotide sequence that is complementary to a sequence located at the 5' end of the locus,
wherein the biological particle comprises genomic DNA and chromatin, and wherein the genomic DNA has been fragmented by a transposase into a plurality of genomic DNA fragments.

12. The partition of claim 11, wherein the 5' end of the 5' primer-adapter nucleic acid molecule further comprises an adapter sequence, optionally wherein the adapter sequence is Read 1 Nextera sequence (R1N), wherein the 5' end of the 3' primer-adapter nucleic acid molecule further comprises a second adapter sequence, optionally wherein the second adapter sequence is a Read 1 Nextera sequence (R1N).

13. The partition of claim 11 or 12, wherein the barcoded bead is attached to a nucleic acid, optionally wherein the nucleic acid attached to the barcoded bead comprises a common barcode sequence, further optionally wherein the nucleic acid attached to the barcoded bead further comprises a sequence complementary to at least a portion of the adapter sequence, and/or wherein the nucleic acid attached to the barcoded bead further comprises a functional sequence configured to attach to a flow cell of a sequencer, optionally wherein the functional sequence for attachment to a sequencing flow cell is a P5 sequence or a P7 sequence.

14. The partition of any one of claims 11-13, wherein the transposase is fused to a set of secondary nanobodies (nb), optionally wherein a primary antibody is bound to a target of interest on the chromatin, further optionally wherein the transposase-nb fusion protein specifically binds to primary antibody, preferably wherein the transposase is a Tn5 transposase.

15. The partition of claim 14, wherein the genomic DNA is fragmented by a transposase-nucleic acid complex to generate a plurality of tagged genomic DNA fragments, optionally wherein the 5' end of each of the tagged genomic DNA fragments comprises an adapter sequence, or the 3' end of each of the tagged genomic DNA fragments comprises a second adapter sequence, further optionally wherein the adapter sequence is a Read 1 sequence (R1N), or the second adapter sequence is Read 2 sequence (R2N).

## Patentansprüche

1. Verfahren zur Genotypisierung eines Locus, umfassend:
Erzeugen einer Vielzahl von biologischen Partikeln, wobei jedes biologische Partikel ein Polynukleotid umfasst;
Erzeugen einer Vielzahl von Partitionen, wobei jede Partition eines von der Vielzahl von biologischen Partikeln, einem Primerpaar und einem barcodiertes Kügelchen umfasst; und
wobei das Primerpaar Folgendes umfasst:
ein 5'-Primer-Adapter-Nukleinsäuremolekül, das eine Nukleotidsequenz umfasst, die komplementär zu einer Sequenz ist, die sich an dem 3'-Ende des Locus innerhalb des Polynukleotids befindet,
ein 3'-Primer-Adapter-Nukleinsäuremolekül, das eine Nukleotidsequenz umfasst, die komplementär zu einer Sequenz ist, die sich an dem 5'-Ende des Locus befindet,
wobei das biologische Partikel genomische DNA und Chromatin umfasst und wobei die genomische DNA durch eine Transposase fragmentiert ist, um die Vielzahl von genomischen DNA-Fragmenten zu erzeugen.

2. Verfahren nach Anspruch 1, ferner umfassend Amplifizieren des Locus unter Verwendung des Primerpaars, um ein Locus-Nukleinsäurefragment zu erzeugen, optional wobei das Locus-Nukleinsäurefragment innerhalb der Partition erzeugt wird.

3. Verfahren nach einem der Ansprüche 1-2, wobei das 5'-Ende des 5'-Primer-Adapter-Nukleinsäuremoleküls ferner eine Adaptersequenz umfasst, wobei optional die Adaptersequenz die Nextera-Sequenz Read 1 (R1N) ist oder wobei das 5'-Ende des 3'-Primer-Adapter-Nukleinsäuremoleküls ferner eine zweite Adaptersequenz umfasst, wobei optional die zweite Adaptersequenz eine Nextera-Sequenz Read 1 (R1N) ist, wobei ferner optional das Locus-Nukleinsäurefragment ferner eine Sequenz umfasst, die komplementär zu der Nextera-Sequenz Read 1 ist.

4. Verfahren nach einem der Ansprüche 1-3, wobei das barcodierte Kügelchen an eine Nukleinsäure angelagert ist, wobei optional die an das barcodierte Kügelchen angelagerte Nukleinsäure eine gemeinsame Barcodesequenz umfasst, wobei optional die an das barcodierte Kügelchen angelagerte Nukleinsäure ferner eine Sequenz umfasst, die zu mindestens einem Abschnitt der Adaptersequenz komplementär ist, und/oder wobei die an das barcodierte Kügelchen angelagerte Nukleinsäure ferner eine funktionelle Sequenz umfasst, die dazu konfiguriert ist, an eine Durchflusszelle eines Sequenzers angelagert zu werden, wobei optional die funktionelle Sequenz zum Anlagern an eine Sequenzierungsdurchflusszelle eine P5-Sequenz oder eine P7-Sequenz ist.

5. Verfahren nach Anspruch 4, ferner umfassend Amplifizieren des Locus-Nukleinsäurefragments oder eines Derivats davon unter Verwendung eines zweiten Primerpaars, umfassend:
ein 3'-Primer-Adapter-Nukleinsäuremolekül, das eine Nukleotidsequenz umfasst, die komplementär zu einer Sequenz ist, die sich an dem 5'-Ende des Locus befindet, und
ein 5'-Primer-Adapter-Nukleinsäuremolekül, das eine Nukleotidsequenz umfasst, die komplementär zu der funktionellen Sequenz ist,
wodurch eine Vielzahl von barcodierten Locus-Nukleinsäurefragmenten erzeugt wird, wobei jedes Fragment der Vielzahl von barcodierten Locus-Nukleinsäurefragmenten den Locus umfasst, oder ferner umfassend Amplifizieren des Locus-Nukleinsäurefragments oder eines Derivats davon unter Verwendung eines dritten Primerpaars, das Folgendes umfasst:
ein 5'-Primer-Adapter-Nukleinsäuremolekül, das eine Nukleotidsequenz umfasst, die komplementär zu einer Sequenz ist, die sich an dem 3'-Ende des Locus befindet, und
ein 3'-Primer-Adapter-Nukleinsäuremolekül, das eine Nukleotidsequenz umfasst, die komplementär zu der funktionellen Sequenz ist,
wodurch eine Vielzahl von barcodierten Locus-Nukleinsäurefragmenten erzeugt wird, wobei jedes Fragment der Vielzahl von barcodierten Locus-Nukleinsäurefragmenten den Locus umfasst, optional
wobei die Vielzahl von barcodierten Locus-Nukleinsäurefragmenten, die den Locus umfassen, innerhalb der Partition erzeugt wird.

6. Verfahren nach einem der Ansprüche 1-5, wobei die Transposase an einen Satz sekundärer Nanobodies (nb) fusioniert ist, wobei optional ein primärer Antikörper an ein Ziel von Interesse auf dem Chromatin gebunden ist, wobei ferner optional das Transposase-nb-Fusionsprotein spezifisch an den primären Antikörper bindet, wobei vorzugsweise die Transposase eine Tn5-Transposase ist.

7. Verfahren nach Anspruch 6, wobei die genomische DNA durch einen Transposase-Nukleinsäure-Komplex fragmentiert wird, um eine Vielzahl von markierten genomischen DNA-Fragmenten zu erzeugen, wobei optional das 5'-Ende jedes der markierten genomischen DNA-Fragmente eine Adaptersequenz umfasst, oder das 3'-Ende jedes der markierten genomischen DNA-Fragmente eine zweite Adaptersequenz umfasst, wobei ferner optional die Adaptersequenz eine Sequenz Read 1 (R1N) ist, oder die zweite Adaptersequenz eine Sequenz Read 2 (R2N) ist.

8. Verfahren nach Anspruch 6 oder 7, ferner umfassend Amplifizieren der Vielzahl von genomischen DNA-Fragmenten oder Derivaten davon durch Nukleinsäureamplifikation, wodurch eine Vielzahl von barcodierten genomischen DNA-Fragmenten erzeugt wird, wobei optional die Vielzahl von barcodierten genomischen DNA-Fragmenten innerhalb der Partition erzeugt wird, wobei ferner optional jedes Fragment der Vielzahl von barcodierten genomischen DNA-Fragmenten die markierte genomische DNA umfasst, wobei vorzugsweise jedes Fragment der Vielzahl von barcodierten genomischen DNA-Fragmenten ferner die gemeinsame Barcodesequenz und/oder die funktionelle Sequenz umfasst, die dazu konfiguriert ist, an eine Durchflusszelle eines Sequenzers angelagert zu werden.

9. Verfahren nach einem der Ansprüche 5-8, ferner umfassend Amplifizieren des barcodierten Locus-Nukleinsäurefragments oder von Derivaten davon durch Nukleinsäureamplifikation, optional ferner umfassend Sequenzieren des barcodierten Locus-Nukleinsäurefragments oder von Derivaten davon.

10. Verfahren nach Anspruch 8 oder 9, ferner umfassend Amplifizieren der Vielzahl von barcodierten genomischen DNA-Fragmenten oder Derivaten davon durch Nukleinsäureamplifikation, optional ferner umfassend Sequenzieren der barcodierten genomischen DNA-Fragmente oder von Derivaten davon, ferner optional ferner umfassend Sequenzieren der barcodierten Locus-Nukleinsäurefragmente und der barcodierten genomischen DNA-Fragmente gleichzeitig.

11. Partition, umfassend: ein biologisches Partikel, umfassend ein Polynukleotid, ein Primerpaar und ein barcodiertes Kügelchen; und
wobei das Primerpaar Folgendes umfasst:
ein 5'-Primer-Adapter-Nukleinsäuremolekül, das eine Nukleotidsequenz umfasst, die komplementär zu einer Sequenz ist, die sich an dem 3'-Ende des Locus innerhalb des Polynukleotids befindet, und
ein 3'-Primer-Adapter-Nukleinsäuremolekül, das eine Nukleotidsequenz umfasst, die komplementär zu einer Sequenz ist, die sich an dem 5'-Ende des Locus befindet,
wobei das biologische Partikel genomische DNA und Chromatin umfasst und wobei die genomische DNA durch eine Transposase in eine Vielzahl von genomischen DNA-Fragmenten fragmentiert ist.

12. Partition nach Anspruch 11, wobei das 5'-Ende des 5'-Primer-Adapter-Nukleinsäuremoleküls ferner eine Adaptersequenz umfasst, wobei optional die Adaptersequenz die Nextera-Sequenz Read 1 (R1N) ist, wobei das 5'-Ende des 3'-Primer-Adapter-Nukleinsäuremoleküls ferner eine zweite Adaptersequenz umfasst, wobei optional die zweite Adaptersequenz eine Nextera-Sequenz Read 1 (R1N) ist.

13. Partition nach Anspruch 11 oder 12, wobei das barcodierte Kügelchen an eine Nukleinsäure angelagert ist, wobei optional die an das barcodierte Kügelchen angelagerte Nukleinsäure eine gemeinsame Barcodesequenz umfasst, wobei ferner optional die an das barcodierte Kügelchen angelagerte Nukleinsäure ferner eine Sequenz umfasst, die zu mindestens einem Abschnitt der Adaptersequenz komplementär ist, und/oder wobei die an das barcodierte Kügelchen angelagerte Nukleinsäure ferner eine funktionelle Sequenz umfasst, die dazu konfiguriert ist, an eine Durchflusszelle eines Sequenzers angelagert zu werden, wobei optional die funktionelle Sequenz zum Anlagern an eine Sequenzierungsdurchflusszelle eine P5-Sequenz oder eine P7-Sequenz ist.

14. Partition nach einem der Ansprüche 11-13, wobei die Transposase an einen Satz sekundärer Nanobodies (nb) fusioniert ist, wobei optional ein primärer Antikörper an ein Ziel von Interesse auf dem Chromatin gebunden ist, wobei ferner optional das Transposase-nb-Fusionsprotein spezifisch an den primären Antikörper bindet, wobei vorzugsweise die Transposase eine Tn5-Transposase ist.

15. Partition nach Anspruch 14, wobei die genomische DNA durch einen Transposase-Nukleinsäure-Komplex fragmentiert wird, um eine Vielzahl von markierten genomischen DNA-Fragmenten zu erzeugen, wobei optional das 5'-Ende jedes der markierten genomischen DNA-Fragmente eine Adaptersequenz umfasst, oder das 3'-Ende jedes der markierten genomischen DNA-Fragmente eine zweite Adaptersequenz umfasst, wobei ferner optional die Adaptersequenz eine Sequenz Read 1 (R1N) ist oder die zweite Adaptersequenz eine Sequenz Read 2 (R2N) ist.

## Revendications

1. Procédé de génotypage d'un locus, comprenant :
la génération d'une pluralité de particules biologiques, chaque particule biologique comprenant un polynucléotide ;
la génération d'une pluralité de partitions, chaque partition comprenant l'une de la pluralité de particules biologiques, une paire d'amorces et une bille à code-barres ; et
dans lequel la paire d'amorces comprend :
une molécule d'acide nucléique amorce-adaptateur 5' comprenant une séquence nucléotidique qui est complémentaire d'une séquence située à l'extrémité 3' du locus au sein du polynucléotide,
une molécule d'acide nucléique amorce-adaptateur 3' comprenant une séquence nucléotidique qui est complémentaire d'une séquence située à l'extrémité 5' du locus,
dans lequel la particule biologique comprend de l'ADN génomique et de la chromatine, et dans lequel l'ADN génomique est fragmenté par une transposase pour générer la pluralité de fragments d'ADN génomique.

2. Procédé de la revendication 1, comprenant en outre l'amplification du locus à l'aide de la paire d'amorces pour générer un fragment d'acide nucléique de locus, éventuellement dans lequel le fragment d'acide nucléique de locus est généré à l'intérieur de la partition.

3. Procédé de l'une quelconque des revendications 1 et 2, dans lequel l'extrémité 5' de la molécule d'acide nucléique amorce-adaptateur 5' comprend en outre une séquence adaptatrice, éventuellement dans lequel la séquence adaptatrice est une séquence de lecture 1 Nextera (R1N), ou dans lequel l'extrémité 5' de la molécule d'acide nucléique amorce-adaptateur 3' comprend en outre une seconde séquence adaptatrice, éventuellement dans lequel la seconde séquence adaptatrice est une séquence de lecture 1 Nextera (R1N), en outre éventuellement dans lequel le fragment d'acide nucléique de locus comprend en outre une séquence complémentaire de la séquence de lecture 1 Nextera.

4. Procédé de l'une quelconque des revendications 1 à 3, dans lequel la bille à code-barres est fixée à un acide nucléique, éventuellement dans lequel l'acide nucléique fixé à la bille à code-barres comprend une séquence de code-barres commune, éventuellement dans lequel l'acide nucléique fixé à la bille à code-barres comprend en outre une séquence complémentaire d'au moins une partie de la séquence adaptatrice, et/ou dans lequel l'acide nucléique fixé à la bille à code-barres comprend en outre une séquence fonctionnelle conçue pour se fixer à une cellule de flux d'un séquenceur, éventuellement dans lequel la séquence fonctionnelle pour la fixation à une cellule de flux de séquençage est une séquence P5 ou une séquence P7.

5. Procédé de la revendication 4, comprenant en outre l'amplification du fragment d'acide nucléique de locus, ou d'un dérivé de celui-ci, à l'aide d'une seconde paire d'amorces comprenant :
une molécule d'acide nucléique amorce-adaptateur 3' comprenant une séquence nucléotidique qui est complémentaire d'une séquence située à l'extrémité 5' du locus, et
une molécule d'acide nucléique amorce-adaptateur 5' comprenant une séquence nucléotidique qui est complémentaire de la séquence fonctionnelle,
générant ainsi une pluralité de fragments d'acide nucléique de locus à code-barres, chaque fragment de la pluralité de fragments d'acide nucléique de locus à code-barres comprenant le locus, ou
comprenant en outre l'amplification du fragment d'acide nucléique de locus, ou d'un dérivé de celui-ci, à l'aide d'une troisième paire d'amorces comprenant :
une molécule d'acide nucléique amorce-adaptateur 5' comprenant une séquence nucléotidique qui est complémentaire d'une séquence située à l'extrémité 3' du locus, et
une molécule d'acide nucléique amorce-adaptateur 3' comprenant une séquence nucléotidique qui est complémentaire de la séquence fonctionnelle,
générant ainsi une pluralité de fragments d'acide nucléique de locus à code-barres, chaque fragment de la pluralité de fragments d'acide nucléique de locus à code-barres comprenant le locus, éventuellement
dans lequel la pluralité de fragments d'acide nucléique de locus à code-barres comprenant le locus est générée à l'intérieur de la partition.

6. Procédé de l'une quelconque des revendications 1 à 5, dans lequel la transposase est fusionnée à un ensemble de nanocorps (nb) secondaires, éventuellement dans lequel un anticorps primaire est lié à une cible d'intérêt sur la chromatine, en outre éventuellement dans lequel la protéine de fusion transposase-nb se lie spécifiquement à l'anticorps primaire, de préférence dans lequel la transposase est une transposase Tn5.

7. Procédé de la revendication 6, dans lequel l'ADN génomique est fragmenté par un complexe transposase-acide nucléique pour générer une pluralité de fragments d'ADN génomique marqués, éventuellement dans lequel l'extrémité 5' de chacun des fragments d'ADN génomique marqués comprend une séquence adaptatrice, ou l'extrémité 3' de chacun des fragments d'ADN génomique marqués comprend une seconde séquence adaptatrice, en outre éventuellement dans lequel la séquence adaptatrice est une séquence de lecture 1 (R1N), ou la seconde séquence adaptatrice est une séquence de lecture 2 (R2N).

8. Procédé de l'une des revendications 6 ou 7, comprenant en outre l'amplification de la pluralité de fragments d'ADN génomique, ou de dérivés de ceux-ci, par amplification d'acide nucléique générant une pluralité de fragments d'ADN génomique à code-barres, éventuellement dans lequel la pluralité de fragments d'ADN génomique à code-barres est générée à l'intérieur de la partition, en outre éventuellement dans lequel chaque fragment de la pluralité de fragments d'ADN génomique à code-barres comprend l'ADN génomique marqué, de préférence dans lequel chaque fragment de la pluralité de fragments d'ADN génomique à code-barres comprend en outre la séquence de code-barres commune et/ou la séquence fonctionnelle conçue pour se fixer à une cellule de flux d'un séquenceur.

9. Procédé de l'une quelconque des revendications 5 à 8, comprenant en outre l'amplification des fragments d'acide nucléique de locus à code-barres, ou de dérivés de ceux-ci, par amplification d'acide nucléique, comprenant en outre éventuellement le séquençage desdits fragments d'acide nucléique de locus à code-barres ou de dérivés de ceux-ci.

10. Procédé de l'une quelconque des revendications 8 ou 9, comprenant en outre l'amplification de la pluralité de fragments d'ADN génomique à code-barres, ou de dérivés de ceux-ci, par amplification d'acide nucléique, comprenant en outre éventuellement le séquençage desdits fragments d'ADN génomique à code-barres ou de dérivés de ceux-ci, comprenant en outre éventuellement le séquençage desdits fragments d'acide nucléique de locus à code-barres et desdits fragments d'ADN génomique à code-barres simultanément.

11. Partition comprenant : une particule biologique comprenant un polynucléotide, une paire d'amorces et une bille à code-barres ; et
dans laquelle la paire d'amorces comprend :
une molécule d'acide nucléique amorce-adaptateur 5' comprenant une séquence nucléotidique qui est complémentaire d'une séquence située à l'extrémité 3' du locus au sein du polynucléotide, et
une molécule d'acide nucléique amorce-adaptateur 3' comprenant une séquence nucléotidique qui est complémentaire d'une séquence située à l'extrémité 5' du locus,
dans laquelle la particule biologique comprend de l'ADN génomique et de la chromatine, et dans laquelle l'ADN génomique a été fragmenté par une transposase en une pluralité de fragments d'ADN génomique.

12. Partition de la revendication 11, dans laquelle l'extrémité 5' de la molécule d'acide nucléique amorce-adaptateur 5' comprend en outre une séquence adaptatrice, éventuellement dans laquelle la séquence adaptatrice est une séquence de lecture 1 Nextera (R1N), dans laquelle l'extrémité 5' de la molécule d'acide nucléique amorce-adaptateur 3' comprend en outre une seconde séquence adaptatrice, éventuellement dans laquelle la seconde séquence adaptatrice est une séquence de lecture 1 Nextera (R1N).

13. Partition de l'une des revendications 11 ou 12, dans laquelle la bille à code-barres est fixée à un acide nucléique, éventuellement dans laquelle l'acide nucléique fixé à la bille à code-barres comprend une séquence de code-barres commune, en outre éventuellement dans laquelle l'acide nucléique fixé à la bille à code-barres comprend en outre une séquence complémentaire d'au moins une partie de la séquence adaptatrice, et/ou dans laquelle l'acide nucléique fixé à la bille à code-barres comprend en outre une séquence fonctionnelle conçue pour se fixer à une cellule de flux d'un séquenceur, éventuellement dans laquelle la séquence fonctionnelle pour la fixation à une cellule de flux de séquençage est une séquence P5 ou une séquence P7.

14. Partition de l'une quelconque des revendications 11 à 13, dans laquelle la transposase est fusionnée à un ensemble de nanocorps (nb) secondaires, éventuellement dans laquelle un anticorps primaire est lié à une cible d'intérêt sur la chromatine, en outre éventuellement dans laquelle la protéine de fusion transposase-nb se lie spécifiquement à l'anticorps primaire, de préférence dans laquelle la transposase est une transposase Tn5.

15. Partition de la revendication 14, dans laquelle l'ADN génomique est fragmenté par un complexe transposase-acide nucléique pour générer une pluralité de fragments d'ADN génomique marqués, éventuellement dans laquelle l'extrémité 5' de chacun des fragments d'ADN génomique marqués comprend une séquence adaptatrice, ou l'extrémité 3' de chacun des fragments d'ADN génomique marqués comprend une seconde séquence adaptatrice, en outre éventuellement dans laquelle la séquence adaptatrice est une séquence de lecture 1 (R1N), ou la seconde séquence adaptatrice est une séquence de lecture 2 (R2N).
